# EUROPEAN PATENT SPECIFICATION

(11) **EP 4 073 073 B1**
(45) Date of publication and mention of the grant of the patent: **06.12.2023**
(21) Application number: 20820181.4
(22) Date of filing: 10.12.2020
(51) Int. Cl.: C07D 495/04, A61P 11/00, A61P 1/16, A61P 9/00, A61P 27/02, A61K 31/519

(54) **THIENOPYRIMIDINE DERIVATIVES AS LPA RECEPTOR 2 INHIBITORS**
THIENOPYRIMIDIN-DERIVATE ALS LPA-REZEPTOR-2-INHIBITOREN
DÉRIVÉS DE THIÉNOPYRIMIDINE UTILISÉS EN TANT QU'INHIBITEURS DU RÉCEPTEUR 2 DE LPA

(30) Priority: 12.12.2019 EP 19215741
(43) Date of publication of application: 19.10.2022
(73) Proprietor: Chiesi Farmaceutici S.p.A., 43122 Parma (IT)
(72) Inventor: AMARI, Gabriele, 43122 Parma (IT); ARMANI, Elisabetta, 43122 Parma (IT); PAGANO, Mafalda, 43122 Parma (IT); RAVEGLIA, Luca, 43122 Parma (IT)
(74) Representative: Chiesi Farmaceutici S.p.A.
(86) International application number: PCT/EP2020/085452
(87) International publication number: WO 2021/116257

(56) References cited:
- BECK ET AL: "Discovery of potent LPA"2 (EDG4) antagonists as potential anticancer agents", BIORGANIC & MEDICINAL CHEMISTRY LETTERS, ELSEVIER, AMSTERDAM , NL, vol. 18, no. 3, 4 January 2008 (2008-01-04), pages 1037-1041, XP022475681, ISSN: 0960-894X, DOI: 10.1016/0022-1139(96)03401-X cited in the application

## Description

### FIELD OF THE INVENTION

The present invention generally relates to compounds inhibiting lysophosphatidic acid receptors (hereinafter LPA inhibitors); the invention relates to compounds that are thienopyrimidine derivatives, methods of preparing such compounds, pharmaceutical compositions containing them and therapeutic use thereof.

The compounds of the invention may be useful for instance in the treatment of many disorders associated with LPA receptors mechanisms.

### BACKGROUND OF THE INVENTION

Lysophosphatidic acid (LPA) is a phospholipid mediator concentrated in serum that act as a potent extracellular signalling molecule through at least six cognate G protein-coupled receptors (GPCRs) in numerous developmental and adult processes including cell survival, proliferation, migration, differentiation, vascular regulation, and cytokine release.

These LPA-mediated processes involve nervous system function, vascular development, immune system function, cancer, reproduction, fibrosis, and obesity (see e.g. Yung et al., J Lipid Res. 2014 Jul;55(7):1192-214*).* The formation of an LPA species depends on its precursor phospholipid, which can vary typically by acyl chain length and degree of saturation. The term LPA generally refers to 18:1 oleoyl-LPA (1-acyl-2-hydroxy-sn-glycero3-phosphate), that is the most quantitatively abundant forms of LPA in human plasma with 16:0-, 18:2-, and 18:1-LPA (see e.g. Sano et al., J Biol Chem. 2002 Dec 13; 277(50) :21197-206)*.* All LPA species are produced from membrane phospholipids via two major metabolic routes. Depending upon the site of synthesis, membrane phospholipids get converted to the corresponding lysophospholipids by the action of phospholipase A1 (PLA1), phospholipase A2 (PLA2), or PLA1 and lecithin-cholesterol acyltransferase (LCAT). Autotaxin (ATX) then acts on the lysophospholipids and converts them into LPA species. The second pathway first converts the phospholipids into phosphatidic acid by the action of phospholipase D. Then PLA1 or PLA2 metabolize phosphatidic acid to the lysophosphatidic acids (see e.g. Riaz et al., Int J Mol Sci. 2016 Feb; 17(2): 215*).*

ATX activity is the major source of plasma extracellular LPA but the source of tissue LPA that contributes to signalling pools likely involves not only ATX but other enzymes as well. The biological functions of LPA are mediated by at least six recognized cell-surface receptors.

All LPA receptors are rhodopsin-like 7-TM proteins that signal through at least two of the four Gα subunit families (Gα12/13, Gαq/11, Gαi/o and GαS). LPA receptors usually trigger response from multiple heterotrimeric G-proteins, resulting in diverse outcomes in a context and cell type dependent manner. Gα12/13-mediated LPA signalling regulates cell migration, invasion and cytoskeletal re-adjustments through activation of RHO pathway proteins. RAC activation downstream of Gαi/o-PI3K also regulates similar processes, but the most notable function of LPA-induced Gαi/o is mitogenic signalling through the RAF-MEK-MAPK cascade and survival signalling through the PI3K-AKT pathway. The LPA-coupled Gαq/11 protein primarily regulates Ca2+ homeostasis through PLC and the second messengers IP3 and DAG. Lastly, GαS can activate adenylyl cyclase and increase cAMP concentration upon LPA stimulation (see e.g. Riaz et al., Int J Mol Sci. 2016 Feb; 17(2): 215*).*

LPA, especially LPA1, LPA2 and LPA3, have been implicated in migration, invasion, metastasis, proliferation and survival and differ in their tissue distribution and downstream signalling pathways.

LPA1 is a 41-kD protein that is widely expressed, albeit at different levels, in all human adult tissues examined and the importance of LPA1 signalling during development and adult life has been demonstrated through numerous approaches (see e.g. Ye at al., 2002, Neuroreport. Dec 3;13(17):2169-75*).* Wide expression of LPA1 is observed in adult mice, with clear presence in at least brain, uterus, testis, lung, small intestine, heart, stomach, kidney, spleen, thymus, placenta, and skeletal muscle. LPA1 is also widely expressed in humans where the expression is more spatially restricted during embryonic development. LPA1 couples with and activates three types of G proteins: Gαi/o, Gαq/11, and Gα12/13. LPA1 activation induces a range of cellular responses: cell proliferation and survival, cell migration, cytoskeletal changes, Ca2+ mobilization, adenylyl cyclase inhibition and activation of mitogen-activated protein kinase, phospholipase C, Akt, and Rho pathways (see e.g. Choi et al., Annu Rev Pharmacol Toxicol. 2010; 50:157-86*).*

LPA2 in humans is a 39-kD protein and shares -55% amino acid sequence homology with LPA1 (see e.g. Yung et al., J Lipid Res. 2014 Jul; 55(7):1192-214)*.* In mouse, LPA2 is highly expressed in kidney, uterus, and testis and moderately expressed in lung; in human tissues, high expression of LPA2 is detected in testis and leukocytes, with moderate expression found in prostate, spleen, thymus, and pancreas.

In terms of signalling activity, LPA2 mostly activates the same pathways as triggered by LPA1 with some exceptions that regards its unique cross-talk behaviour. For example, LPA2 promotes cell migration through interactions with focal adhesion molecule TRIP6 (see e.g. Lai YJ, 2005, Mol.Cell.Biol. 25:5859-68), and several PDZ proteins and zinc finger proteins are also reported to interact directly with the carboxyl-terminal tail of LPA2 (see e.g. Lin FT, 2008, Biochim.Biophys.Acta 1781:558-62).

Human LPA3 is a 40-kD protein and shares sequence homology with LPA1 (-54%) and LPA2 (-49%). In adult humans LPA3 is highly expressed in heart, pancreas, prostate and testis. Moderate levels of expression are also found in brain, lungs and ovary. Like LPA1 and LPA2 the signaling activity of LPA3 results from its coupling to Gαi/o and Gαq/11 (see e.g Ishii et al., Mol Pharmacol 58:895-902, 2000). Each LPA has multiple important regulatory functions throughout the body.

As LPA signalling has been strongly implicated in many disease states, great interest has been expressed in developing specific LPA inhibitors (see e.g. Stoddard et el., Biomol Ther (Seoul) 2015 Jan;23(1):1-11). Different studies have demonstrated a positive role for LPA in the pathogenesis of pulmonary fibrosis (PF), a devastating disease characterized by alveolar epithelial cell injury, accumulation of myofibroblasts and deposition of extracellular matrix proteins leading to a loss of lung function and death (see e.g. Wilson MS, Wynn TA (2009), Mucosal Immunol 2: 103-121).

Evidences showed that lysophosphatidic acid levels dramatically increase in bronchoalveolar lavage fluid of PF patients where it mediates fibroblast migration in the injured lung acting through LPA1 (see e.g. Tager et al., Nat Med. 2008 Jan; 14(1):45-54). In addition, mice lacking LPA1 or LPA2 are markedly protected from fibrosis and mortality in a mouse model of the bleomycin induced pulmonary fibrosis (see e.g. Huang et al., Am J Respir Cell Mol Biol. 2013 Dec; 49(6): 912-922 and Tager et al., Nat Med. 2008 Jan;14(1):45-54).

In vitro, LPA1 is known to induce the proliferation and differentiation of lung fibroblasts (see e.g. Shiomi et al., Wound Repair Regen. 2011 Mar Apr; 19(2): 229 240), and to augment the fibroblast-mediated contraction of released collagen gels (see e.g. Mio et al., Journal of Laboratory and Clinical Medicine, Volume 139, Issue 1, January 2002, Pages 20-27*).* In human lung fibroblasts, the knockdown of LPA2 attenuated the LPA-induced expression of TGF-β1 and the differentiation of lung fibroblasts to myofibroblasts, resulting in the decreased expression of different profibrotic markers such as FN, α-SMA, and collagen, as well as decreased activation of extracellular regulated kinase 1/2, Akt, Smad3, and p38 mitogen-activated protein kinase (see e.g. Huang et al., Am J Respir Cell Mol Biol. 2013 Dec; 49(6): 912-922*).* Moreover Xu et al., confirmed that the expression of LPA2 was also up-regulated in lungs from bleomycin-challenged mice where it is able to induce the activation of TGF-β pathway, a key cytokine that play an essential role during the development of the disease, via a RhoA and Rho kinase pathway (see e.g. Xu et al., Am J Pathol. 2009 Apr;174(4):1264-79*).* In in vivo preclinical model, the oral administration of an LPA1 antagonist significantly reduced bleomycin-induced pulmonary fibrosis in mice (Tager etal., Nat Med. 2008 Jan;14(1):45-54*;* Swaney et al., Br J Pharmacol. 2010 Aug; 160(7): 1699-1713*),* and the intraperitoneal injection of an LPA1/3 antagonist ameliorated irradiation-induced lung fibrosis (see e.g. Gan et al., 2011, Biochem Biophys Res Commun 409: 7-13). In a renal fibrosis model, LPA1 administration of an LPA1 antagonist suppressed renal interstitial fibrosis (see e.g Pradere et al., JAm Soc Nephrol 2007;18:3110 3118*).*

Various compounds have been described in the literature as LPA1 or LPA2 antagonist.

WO2019126086 and WO2019126087 (Bristol-Myers Squibb) disclose cyclohexyl acid isoxazole azines as LPA1 antagonist, useful for the treatment of disorder or condition associated with dysregulation of lysophosphatidic acid receptor 1.

WO2019126099 (Bristol-Myers Squibb) discloses isoxazole N-linked carbamoyl cyclohexyl acid as LPA1 antagonist for the treatment of disorder or condition associated with dysregulation of lysophosphatidic acid receptor 1.

WO2019126090 (Bristol-Myers Squibb) discloses triazole N-linked carbamoyl cyclohexyl acids as LPA1 antagonists. The compounds are selective LPA1 receptor inhibitors and are useful for the treatment of disorder or condition associated with dysregulation of lysophosphatidic acid receptor 1.

WO2017223016 (Bristol-Myers Squibb) discloses carbamoyloxymethyl triazole cyclohexyl acids as LPA1 antagonist for the treatment of fibrosis including idiopathic pulmonary fibrosis.

WO2012028243 (Merck) discloses pyrazolopyridinone derivatives according to formula (I) and a process of manufacturing thereof as LPA2 receptor antagonists for the treatment of various diseases.

Amgen Inc. discloses in "Discovery of potent LPA2 (EDG4) antagonists as potential anticancer agents" Bioorg Med Chem Lett. 2008 Feb 1;18(3):1037-41, LPA2 antagonists. Key compounds were evaluated in vitro for inhibition of LPA2 mediated Erk activation and proliferation of HCT-116 cells. These compounds could be used as tool compounds to evaluate the anticancer effects of blocking LPA2 signalling.

Of note, antagonizing the LPA receptors may be useful for the treatment of fibrosis and disease, disorder and conditions that result from fibrosis, and even more antagonizing receptor LPA2 may be particularly efficacious in the treatment of the above-mentioned disease, disorder and conditions.

Several efforts have been done in the past years to develop novel LPA1 receptor antagonist useful for the treatment of several disease and some of those compounds have shown efficacy also in humans.

Thus, there remains a potential for developing inhibitors of receptors LPA2 useful for the treatment of diseases or conditions associated with a dysregulation of LPA receptors, in particular fibrosis.

In this respect, the state of the art does not describe or suggest thienopyrimidine derivatives of general formula (I) of the present invention having an antagonist activity on receptor LPA2 which represent a solution to the aforementioned need.

### SUMMARY OF THE INVENTION

In a first aspect the invention refers to a compound of formula (I) 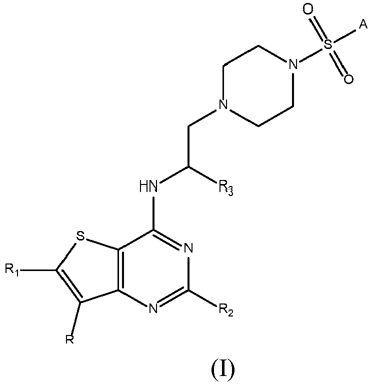 wherein
**R** is H or selected from the group consisting of (C₁-C₄)alkyl, (C₁-C₄)haloalkyl and 5-6 membered heteroaryl wherein each of said heteroaryl may be optionally substituted by one or more group selected from (C₁-C₄)alkyl, halo and (C₁-C₄)haloalkyl;
**R₁** is H or (C₁-C₄)alkyl;
**R₂** is H or selected from the group consisting of (C₁-C₄)alkyl, halo, (C₁-C₄)haloalkyl and (C₃-C₈)cycloalkyl;
**R₃** is H or (C₁-C₄)alkyl;
**A** is selected from the group consisting of 5-6 membered heteroaryl wherein each of said heteroaryl may be optionally substituted by one or more group selected from (C₁-C₄)alkyl, -C(O)R₁, -C(O)OR₁, -C(O)R₁, (C₁-C₄)haloalkyl, halo, -NR_{A}C(O)R₁, -NR_{A}C(O)OR₁, -NRaC(O)-(C₁-C₄)alkylene-OR₁, -NR_{A}C(O)R_{C}, -NR_{A}C(O)NR_{A}R_{B}, -N(C₁₋C₄)alkylene-NR_{A}R_{B}, aryl and heteroaryl optionally substituted by one or more (C₁-C₄)alkyl and (C₁-C₄)haloalkyl, or
**R_{C}** is selected from the group consisting of heteroaryl, aryl, (C₃-C₈) cycloalkyl and (C₄-C₈) heterocycloalkyl wherein said heteroaryl, aryl, heterocycloalkyl and cycloalkyl may be optionally substituted by one or more (C₁-C₄)alkyl and -C(O)ORi;
**R_{A}** and **R_{B}** are at each occurrence independently H or selected from the group consisting of (C₁-C₄)alkyl, (C₃-C₈)cycloalkyl, (C₁-C₆)haloalkyl and halo, or
R_{A} and R_{B} may form together with the nitrogen atom to which they are attached a 4-6 membered saturated heterocyclic ring system optionally containing a further heteroatom selected from N, S or O, said heterocyclic ring system may be optionally substituted by one or more groups selected from (C₁-C₄)alkyl, (C₁-C₄) haloalkyl and halo.

In a second aspect, the invention refers to pharmaceutical composition comprising a compound of formula (I) in admixture with one or more pharmaceutically acceptable carrier or excipient.

In a third aspect, the invention refers to a compound of formula (I) for use as a medicament.

In a further aspect, the invention refers to a compound of formula (I) for use in treating disease, disorder, or condition associated with dysregulation of lysophosphatidic acid receptor 2 (LPA2).

In a further aspect, the invention refers to a compound of formula (I) for use in the prevention and/or treatment of fibrosis and/or diseases, disorders, or conditions that involve fibrosis.

In a further aspect, the invention refers to a compound of formula (I) for use in the prevention and/or treatment idiopathic pulmonary fibrosis (IPF).

### DETAILED DESCRIPTION OF THE INVENTION

Unless otherwise specified, the compound of formula (I) of the present invention is intended to include also stereoisomer, tautomer or pharmaceutically acceptable salt or solvate thereof.

The term "pharmaceutically acceptable salts", as used herein, refers to derivatives of compounds of formula (I) wherein the parent compound is suitably modified by converting any of the free acid or basic group, if present, into the corresponding addition salt with any base or acid conventionally intended as being pharmaceutically acceptable.

Suitable examples of said salts may thus include mineral or organic acid addition salts of basic residues such as amino groups, as well as mineral or organic basic addition salts of acid residues such as carboxylic groups.

Cations of inorganic bases which can be suitably used to prepare salts comprise ions of alkali or alkaline earth metals such as potassium, sodium, calcium or magnesium.

Those obtained by reacting the main compound, functioning as a base, with an inorganic or organic acid to form a salt comprise, for example, salts of hydrochloric acid, hydrobromic acid, sulfuric acid, phosphoric acid, methane sulfonic acid, camphor sulfonic acid, acetic acid, oxalic acid, maleic acid, fumaric acid, succinic acid and citric acid.

The term "solvate" means a physical association of a compound of this invention with one or more solvent molecules, whether organic or inorganic. This physical association includes hydrogen bonding. In certain instances, the solvate will be capable of
isolation, for example, when one or more solvent molecules are incorporated in the crystal lattice of the crystalline solid. The solvate may comprise either a stoichiometric or nonstoichiometric amount of the solvent molecules.

The term "stereoisomer" refers to isomers of identical constitution that differ in the arrangement of their atoms in space. Enantiomers and diastereomers are examples of stereoisomers.

The term "enantiomer" refers to one of a pair of molecular species that are mirror images of each other and are not superimposable.

The term "diastereomer" refers to stereoisomers that are not mirror images.

The term "racemate" or "racemic mixture" refers to a composition composed of equimolar quantities of two enantiomeric species, wherein the composition is devoid of optical activity.

The symbols "R" and " S" represent the configuration of substituents around a chiral carbon atom(s). The isomeric descriptors "R" and "S" are used as described herein for indicating atom configuration(s) relative to a core molecule and are intended to be used as defined in the literature (IUP AC Recommendations 1996, Pure and Applied Chemistry, 68:2193-2222 (1996)).

The term "tautomer" refers to each of two or more isomers of a compound that exist together in equilibrium and are readily interchanged by migration of an atom or group within the molecule.

The term "halogen" or "halogen atoms" or "halo" as used herein includes fluorine, chlorine, bromine, and iodine atom.

The term "5-membered heterocyclyl" refers to a mono satured or unsatured group containing one or more heteroatoms selected from N and O.

The term "(Cₓ-C_{y}) alkyl" wherein x and y are integers, refers to a straight or branched chain alkyl group having from x to y carbon atoms. Thus, when x is 1 and y is 6, for example, the term includes methyl, ethyl, n-propyl, isopropyl, n-butyl, isobutyl, sec-butyl, t-butyl, n-pentyl and n-hexyl.

The term "(Cₓ-C_{y})alkylene" wherein x and y are integers, refers to a Cₓ-C_{y}alkyl radical having in total two unsatisfied valencies, such as a divalent methylene radical.

The expressions "(Cₓ-C_{y}) haloalkyl" wherein x and y are integers, refer to the above defined "Cₓ-C_{y}alkyl" groups wherein one or more hydrogen atoms are replaced by one or more halogen atoms, which can be the same or different.

Examples of said "(Cₓ-C_{y}) haloalkyl" groups may thus include halogenated, poly-halogenated and fully halogenated alkyl groups wherein all hydrogen atoms are replaced by halogen atoms, e.g. trifluoromethyl.

The term "(Cₓ-C_{y}) cycloalkyl" wherein x and y are integers, refers to saturated cyclic hydrocarbon groups containing the indicated number of ring carbon atoms. Examples include cyclopropyl, cyclobutyl, cyclopentyl, cyclohexyl, cycloheptyl.

The term "aryl" refers to mono cyclic carbon ring systems which have 6 ring atoms wherein the ring is aromatic. Examples of suitable aryl monocyclic ring systems include, for instance, phenyl.

The term "heteroaryl" refers to a mono- or bi-cyclic aromatic group containing one or more heteroatoms selected from S, N and O, and includes groups having two such monocyclic rings, or one such monocyclic ring and one monocyclic aryl ring, which are fused through a common bond.

The term "(Cₓ-C_{y}) heterocycloalkyl" wherein x and y are integers, refers to saturated or partially unsaturated monocyclic (Cₓ-C_{y}) cycloalkyl groups in which at least one ring carbon atom is replaced by at least one heteroatom (e.g. N, S or O) or may bear an -oxo (=O) substituent group. Said heterocycloalkyl may be further optionally substituted on the available positions in the ring, namely on a carbon atom, or on a heteroatom available for substitution. Substitution on a carbon atom includes spiro disubstitution as well as substitution on two adjacent carbon atoms, in both cases thus form additional condensed 5 to 6 membered heterocyclic ring.

The term "(Cₓ-C_{y}) aminoalkyl" wherein x and y are integers, refers to the above defined "(C₁-C₆) alkyl" groups wherein one or more hydrogen atoms are replaced by one or more amino group.

The term "(Cₓ-C_{y}) hydroxyalkyl" wherein x and y are integers, refers to the above defined "(C₁-C₆) alkyl" groups wherein one or more hydrogen atoms are replaced by one or more hydroxy (OH) group.

The term "(Cₓ-C_{y}) alkoxy" or "(Cₓ-C_{y}) alkoxyl" wherein x and y are integers, refer to a straight or branched hydrocarbon of the indicated number of carbons, attached to the rest of the molecule through an oxygen bridge.

A dash ("-") that is not between two letters or symbols is meant to represent the point of attachment for a substituent.

The carbonyl group is herein preferably represented as -C(O)- as an alternative to the other common representations such as -CO-, -(CO)- or -C(=O)-.

In general, the bracketed group is a lateral group, not included into the chain, and brackets are used, when deemed useful, to help disambiguating linear chemical formulas; e.g. the sulfonyl group -SO₂- might be also represented as -S(O)₂- to disambiguate e.g. with respect to the sulfinic group -S(O)O-.

Whenever basic amino or quaternary ammonium groups are present in the compounds of formula I, physiologically acceptable anions may be present, selected among chloride, bromide, iodide, trifluoroacetate, formate, sulfate, phosphate, methanesulfonate, nitrate, maleate, acetate, citrate, fumarate, tartrate, oxalate, succinate, benzoate,
p-toluenesulfonate, pamoate and naphthalene disulfonate. Likewise, in the presence of acidic groups such as COOH groups, corresponding physiological cation salts may be present as well, for instance including alkaline or alkaline earth metal ions.

As above indicated, the present invention refers to a series of compounds represented by the general formula (I) as herein below described in details, which are endowed with an inhhinitory activity on receptor LPA2.

Advantageously, the antagonist action receptor LPA2 can be effective in the treatment of those diseases where the LPA receptors play a relevant role in the pathogenesis such as fibrosis and disease, disorder and condition from fibrosis.

Differently from similar compounds of the prior art, such as compounds disclosed for example in Merck WO2012028243 and Amgen compounds, the compounds of formula (I) of the present invention are much more acitve on the LPA2.

The Merck and Amgen compounds show a maximum potency expressed as half maximal inhibitory concentration (IC₅₀) on LPA2 around 500 nm.

As indicated in the experimental part, in particular in Table 2, the compounds of formual (I) of the present invention show a notable potency with respect to their inhibitory activity on receptor LPA2 below about 500 nm confirming that they are able to antagonize the isoform of LPA2 receptor involved in fibrosis and diseases that result from fibrosis with a greater potency respect to the compound of the prior art.

Advantageously, the compounds of the present invention characterized by a very high potency, could be administered in human at a lower dosage respect to the compounds of the prior art, thus reducing the adverse events that typically occur administering higher dosages of drug.

Therefore, the compounds of the present invention are particularly appreciated by the skilled person when looking at a suitable and efficacious compounds useful for the treatment of fibrosis, in particular idiopatic pulmonary fibrosis.

Thus, in one aspect the present invention relates to a compound of general formula (I) as LPA2 antagonist 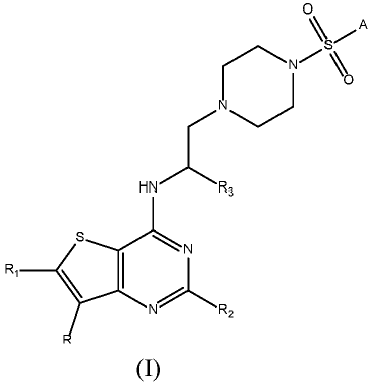 wherein
**R** is H or selected from the group consisting of (C₁-C₄)alkyl, (C₁-C₄)haloalkyl and 5-6 membered heteroaryl wherein each of said heteroaryl may be optionally substituted by one or more group selected from (C₁-C₄)alkyl, halo, (C₁-C₄)haloalkyl;
**R₁** is H or (C₁-C₄)alkyl;
**R₂** is H or selected from the group consisting of (C₁-C₄)alkyl, halo, (C₁-C₄)haloalkyl, (C₃-C₈)cycloalkyl;
**R₃** is H or (C₁-C₄)alkyl;
**A** is selected from the group consisting of 5-6 membered heteroaryl wherein each of said heteroaryl may be optionally substituted by one or more group selected from (C₁-C₄)alkyl, -C(O)R₁, -C(O)ORi, -C(O)R₁, (C₁-C₄)haloalkyl, halo, -NR_{A}C(O)R₁, -NR_{A}C(O)OR₁, -NRaC(O)-(C₁-C₄)alkylene-OR₁, -NR_{A}C(O)R_{C}, -NR_{A}C(O)NR_{A}R_{B}, -N(C₁-C₄)alkylene-NR_{A}R_{B}, aryl and heteroaryl optionally substituted by one or more (C₁-C₄)alkyl and (C₁-C₄)haloalkyl, or
**R_{C}** is selected from the group consisting of heteroaryl, aryl, (C₃-C₈) cycloalkyl and (C₄-C₈) heterocycloalkyl wherein said heteroaryl, aryl, heterocycloalkyl and cycloalkyl may be optionally substituted by one or more (C₁-C₄)alkyl and -C(O)ORi;
**R_{A}** and **R_{B}** are at each occurrence independently H or selected from the group consisting of (C₁-C₄)alkyl, (C₃-C₈)cycloalkyl, (C₁-C₆)haloalkyl, halo, or
R_{A} and R_{B} may form together with the nitrogen atom to which they are attached a 4-6 membered saturated heterocyclic ring system optionally containing a further heteroatom selected from N, S or O, said heterocyclic ring system may be optionally substituted by one or more groups selected from (C₁-C₄)alkyl, (C₁-C₄) haloalkyl, halo.

In one preferred embodiment A is selected from the group consisting of 5-6 membered heteroaryl and aryl wherein each of said heteroaryl and aryl may be optionally substituted by one or more group selected from (C₁-C₄)alkyl, -C(O)R₁, -C(O)ORi, -C(O)R₁, (C₁-C₄)haloalkyl, halo, -NR_{A}C(O)R₁, -NR_{A}C(O)OR₁, -NRaC(O)-(C₁-C₄)alkylene-OR₁, -NR_{A}C(O)R_{C}, -NR_{A}C(O)NR_{A}R_{B}, -N(C₁-C₄)alkylene-NR_{A}R_{B}, aryl and heteroaryl optionally substituted by one or more (C₁-C₄)alkyl and (C₁-C₄)haloalkyl selected from the group consisting of isoxazole, pyridine, thiazole, oxazole, 1,2,4-oxadiazole, 1,3,4-oxadiazole and pyrazole.

In one preferred embodiment when A is 5-6 membered heteroaryl said 5-6 membered heteroaryl is selected from the group consisting of thiazole, thiophene and furan.

In one preferred embodiment when **R_{C}** is heteroaryl said heteroaryl is isoxazole optionally substituted by one or more (C₁-C₄)alkyl and -C(O)OR₁.
**R** is H or selected from the group consisting of (C₁-C₄)alkyl, (C₁-C₄)haloalkyl and 5-6 membered heteroaryl wherein each of said heteroaryl may be optionally substituted by one or more group selected from (C₁-C₄)alkyl, halo, (C₁-C₄)haloalkyl;
**R₁** is H or (C₁-C₄)alkyl;
**R₂** is H or selected from the group consisting of (C₁-C₄)alkyl, halo, (C₁-C₄)haloalkyl, (C₃-C₈)cycloalkyl;
**R₃** is (C₁-C₄)alkyl;
**A** is selected from the group consisting of 5-6 membered heteroaryl wherein each of said heteroaryl may be optionally substituted by one or more group selected from (C₁-C₄)alkyl, -C(O)R₁, -C(O)OR₁, (C₁-C₄)haloalkyl, halo, -NR_{A}C(O)R₁, -NR_{A}C(O)OR₁, -NRaC(O)-(C₁-C₄)alkylene-OR₁, -NR_{A}C(O)R_{C}, aryl and heteroaryl optionally substituted by one or more (C₁-C₄)alkyl and (C₁-C₄)haloalkyl;
**R_{C}** is selected from the group consisting of heteroaryl and (C₄-C₈) heterocycloalkyl wherein said heteroaryl and heterocycloalkyl may be optionally substituted by one or more (C₁-C₄)alkyl and -C(O)ORi,
**R_{A}** is H or (C1-C4)alkyl.

According to the preferred embodiment, the invention refers to at least one of the compounds listed in the Table 1 below and pharmaceutical acceptable salts thereof; those compounds are active on LPA2, as shown in Table 2. Examples 5, 6, 7, 9, 11, 12, 16, 17, 18, 23, 24 and 54 are not according to the invention.

**Table 1 List of preferred compounds of Formula (I)**

| **Example No.** | **Structure** | **IUPAC name** |
|---|---|---|
| 1 | 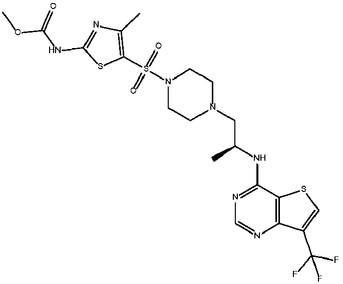 | methyl N-[4-methyl-5-({4-[(2S)-2-{[7-(trifluoromethyl)thieno[3,2-d]pyrimidin-4-yl]amino}propyl]piperazin-1-yl}sulfonyl)-1,3-thiazol-2-yl]carbamate |
| 2 | 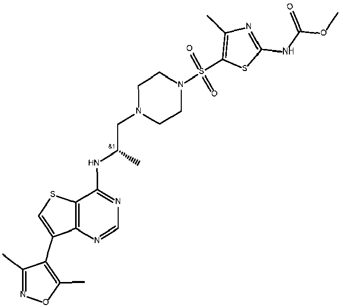 | methyl N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]carbamate |
| 3 | 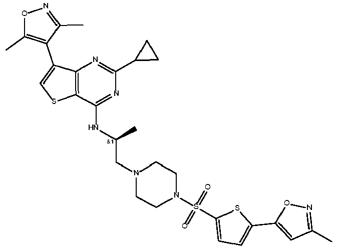 | 2-cyclopropyl-7-(3,5-dimethyl-1,2-oxazol-4-yl)-N-[(2S)-1-(4-{[5-(3-methyl-1,2-oxazol-5-yl)thiophen-2-yl]sulfonyl}piperazin-1- yl)propan-2-yl]thieno[3,2-d]pyrimidin-4-amine |
| 4 | 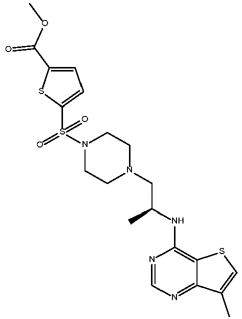 | methyl 5-({4-[(2S)-2-({7-methylthieno[3,2-d]pyrimidin-4-yl}amino)propyl]piperazin-1- yl}sulfonyl)thiophene-2-carboxylate |
| 5 | 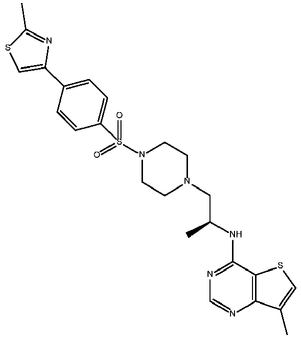 | 7-methyl-N-[(2S)-1-{4-[4-(2-methyl-1,3-thiazol-4-yl)benzenesulfonyl]piperazin -1-yl}propan-2-yl]thieno[3,2-d]pyrimidin-4-amine |
| 6 | 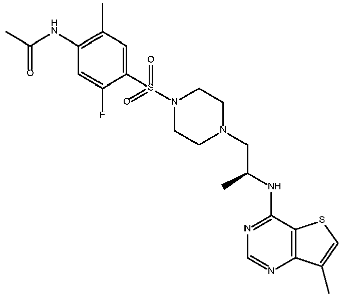 | N-[5-fluoro-2-methyl-4-({4-[(2S)-2-({7-methylthieno[3,2-d]pyrimidin-4-yl}amino)propyl]piperazin-1- yl}sulfonyl)phenyl]acetamid e |
| 7 | 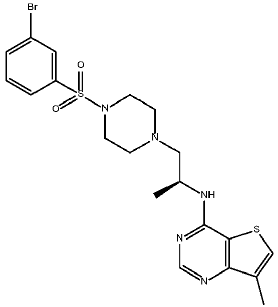 | N-[(2S)-1-[4-(3-bromobenzenesulfonyl)piper azin-1-yl]propan-2-yl]-7-methylthieno[3,2-d]pyrimidin-4-amine |
| 8 | 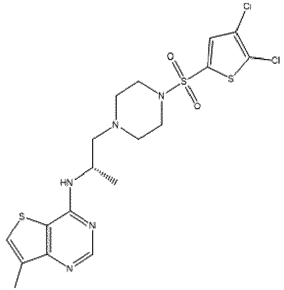 | N-[(2S)-1-{4-[(4,5-dichlorothiophen-2-yl)sulfonyl]piperazin-1- yl}propan-2-yl]-7-methylthieno[3,2-d]pyrimidin-4-amine |
| 9 | 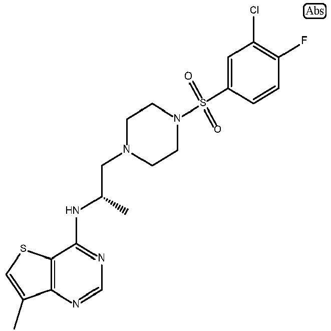 | N-[(2S)-1-[4-(3-chloro-4-fluorobenzenesulfonyl)pipera zin-1-yl]propan-2-yl]-7-methylthieno[3,2-d]pyrimidin-4-amine |
| 10 | 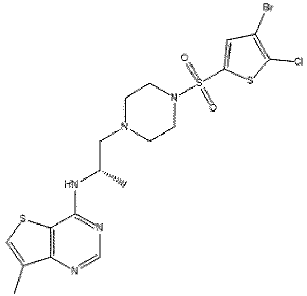 | N-[(2S)-1-{4-[(4-bromo-5-chlorothiophen-2-yl)sulfonyl]piperazin-1 - yl}propan-2-yl]-7-methylthieno[3,2-d]pyrimidin-4-amine |
| 11 | 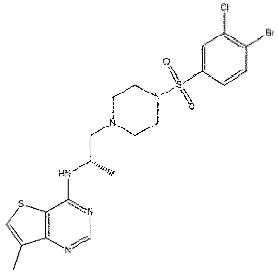 | N-[(2S)-1-[4-(4-bromo-3-chlorobenzenesulfonyl)pipera zin-1-yl]propan-2-yl]-7-methylthieno[3,2-d]pyrimidin-4-amine |
| 12 | 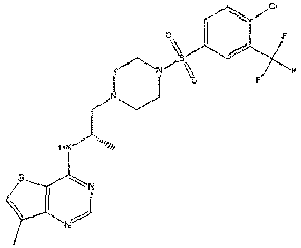 | N-[(2S)-1-{4-[4-chloro-3-(trifluoromethyl)benzenesulf onyl]piperazin-1-yl}propan-2-yl]-7-methylthieno[3,2-d]pyrimidin-4-amine |
| 13 | 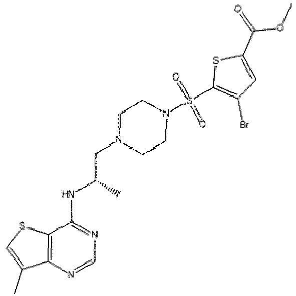 | methyl 4-bromo-5-({4-[(2S)-2-({7-methylthieno[3,2-d]pyrimidin-4-yl}amino)propyl]piperazin-1- yl}sulfonyl)thiophene-2-carboxylate |
| 14 | 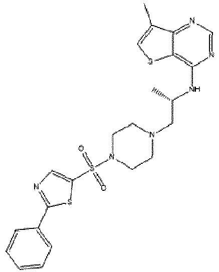 | 7-methyl-N-[(2S)-1-{4-[(2-phenyl-1,3-thiazol-5-yl)sulfonyl]piperazin-1- yl}propan-2-yl]thieno[3,2-d]pyrimidin-4-amine |
| 15 | 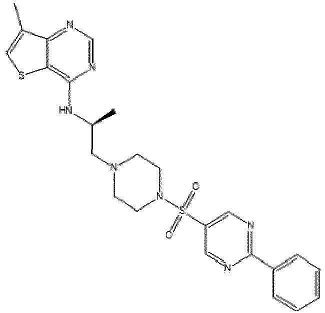 | 7-methyl-N-[(2S)-1-{4-[(2-phenylpyrimidin-5-yl)sulfonyl]piperazin-1- yl}propan-2-yl]thieno[3,2-d]pyrimidin-4-amine |
| 16 | 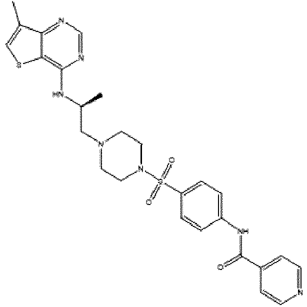 | N-[4-({4-[(2S)-2-({7-methylthieno[3,2-d]pyrimidin-4-yl}amino)propyl]piperazin-1- yl}sulfonyl)phenyl]pyridine-4-carboxamide |
| 17 | 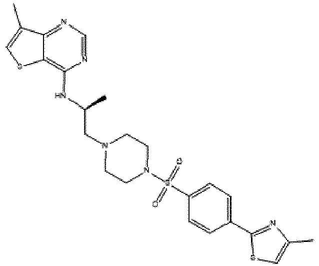 | 7-methyl-N-[(2S)-1-{-4-[4-(4-methyl-1,3-thiazol-2-yl)benzenesulfonyl]piperazin -1-yl}propan-2-yl]thieno[3,2-d]pyrimidin-4-amine |
| 18 | 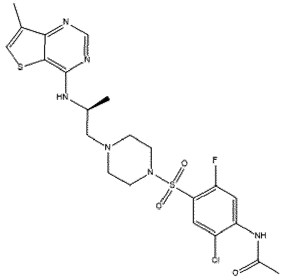 | N-[2-chloro-5-fluoro-4-({4-[(2S)-2-({7-methylthieno[3,2-d]pyrimidin-4-yl}amino)propyl]piperazin-1- yl}sulfonyl)phenyl]acetamid e |
| 19 | 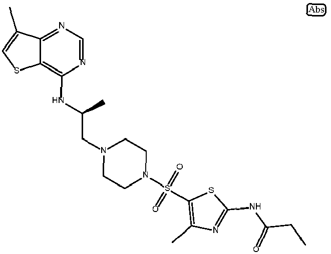 | N-[4-methyl-5-({4-[(2S)-2-({7-methylthieno[3,2-d]pyrimidin-4-yl}amino)propyl]piperazin-1- yl}sulfonyl)-1,3-thiazol-2-yl]propanamide |
| 20 | 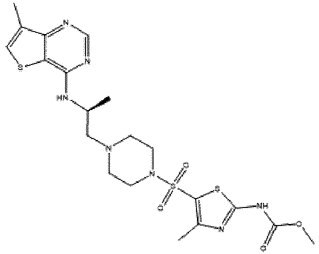 | methyl N-[4-methyl-5-({4-[(2S)-2-({7-methylthieno[3,2-d]pyrimidin-4-yl}amino)propyl]piperazin-1- yl}sulfonyl)-1,3-thiazol-2-yl]carbamate |
| 21 | 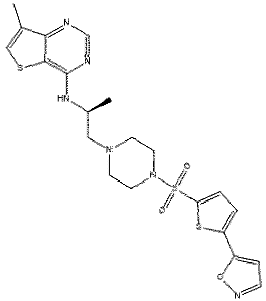 | 7-methyl-N-[(2S)-1-(4-{[5-(1,2-oxazol-5-yl)thiophen-2-yl]sulfonyl}piperazin-1-yl)propan-2-yl]thieno[3,2-d]pyrimidin-4-amine |
| 22 | 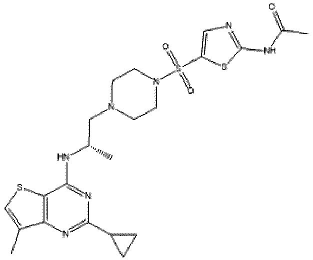 | N-[5-({4-[(2S)-2-({2-cyclopropyl-7-methylthieno[3,2-d]pyrimidin-4-yl}amino)propyl]piperazin-1- yl}sulfonyl)-1,3-thiazol-2-yl]acetamide |
| 23 | 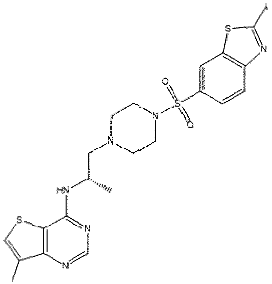 | 7-methyl-N-[(2S)-1-{4-[(2-methyl-1,3-benzothiazol-6-yl)sulfonyl]piperazin-1- yl}propan-2-yl]thieno[3,2-d]pyrimidin-4-amine |
| 24 | 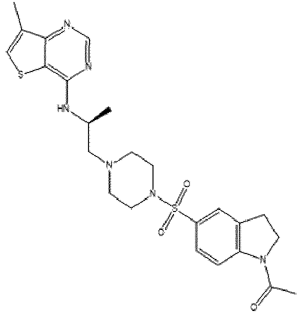 | 1-[5-(14-[(2S)-2-(17-methylthieno[3,2-d]pyrimidin-4-yl}amino)propyl]piperazin-1- yl}sulfonyl)-2,3-dihydro-1H-indol-1-yl]ethan-1-one |
| 25 | 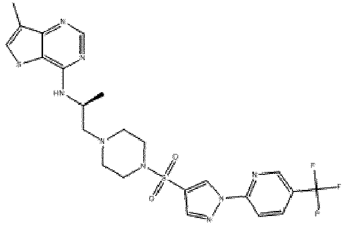 | 7-methyl-N-[(2S)-1-[4-({1-[5-(trifluoromethyl)pyridin-2-yl]-1H-pyrazol-4-yl}sulfonyl)piperazin-1-yl]propan-2-yl]thieno[3,2-d]pyrimidin-4-amine |
| 26 | 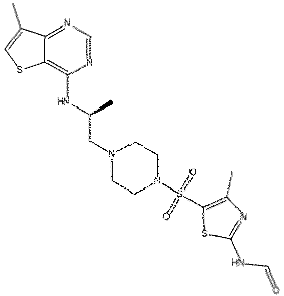 | N-[4-methyl-5-({4-[(2S)-2-({7-methylthieno[3,2-d]pyrimidin-4-yl}amino)propyl]piperazin-1-yl} sulfonyl)-1,3-thiazol-2-yl]formamide |
| 27 | 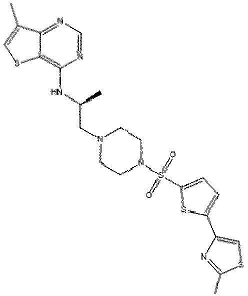 | 7-methyl-N-[(2S)-1-(4-{[5-(2-methyl-1,3-thiazol-4-yl)thiophen-2-yl]sulfonyl}piperazin-1-yl)propan-2-yl]thieno[3,2-d]pyrimidin-4-amine |
| 28 | 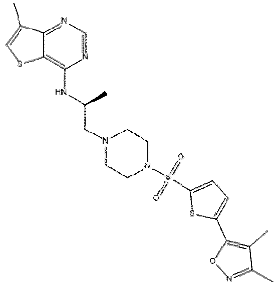 | N-[(2S)-1-(4-{ [5-(3,4-dimethyl-1,2-oxazol-5-yl)thiophen-2-yl]sulfonyl}piperazin-1-yl)propan-2-yl]-7-methylthieno[3,2-d]pyrimidin-4-amine |
| 29 | 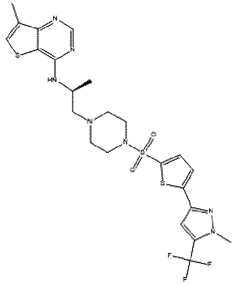 | 7-methyl-N-[(2S)-1-[4-({5-[1-methyl-5-(trifluoromethyl)-1H-pyrazol-3-yl]thiophen-2-yl}sulfonyl)piperazin-1-yl]propan-2-yl]thieno[3,2-d]pyrimidin-4-amine |
| 30 | 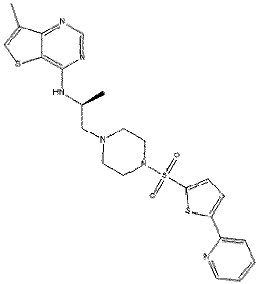 | 7-methyl-N-[(2S)-1-(4-{[5-(pyridin-2-yl)thiophen-2-yl]sulfonyl}piperazin-1- yl)propan-2-yl]thieno[3,2-d]pyrimidin-4-amine |
| 31 | 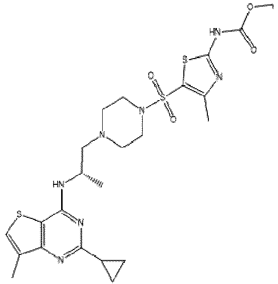 | methyl N-[5-({4-[(2S)-2-({2-cyclopropyl-7-methylthieno[3,2-d]pyrimidin-4-yl}amino)propyl]piperazin-1- yl}sulfonyl)-4-methyl-1,3- thiazol-2-yl]carbamate |
| 32 | 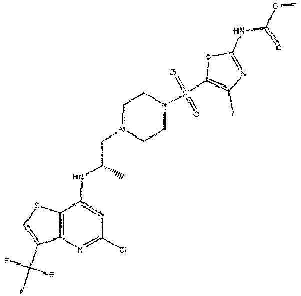 | methyl N-[5-({4-[(2S)-2-{[2-chloro-7-(trifluoromethyl)thieno[3,2-d]pyrimidin-4-yl]amino }propyl]piperazin-1-yl}sulfonyl)-4-methyl-1,3-thiazol-2-yl]carbamate |
| 33 | 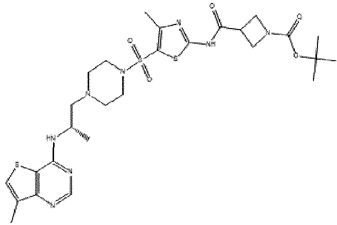 | tert-butyl 3-{[4-methyl-5-({4-[(2S)-2-({7-methylthieno[3,2-d]pyrimidin-4-yl}amino)propyl]piperazin-1- yl}sulfonyl)-1,3-thiazol-2-yl]carbamoyl}azetidine-1-carboxylate |
| 34 | 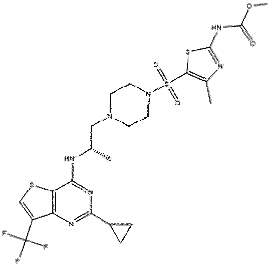 | methyl N-[5-({4-[(2S)-2-{[2-cyclopropyl-7-(trifluoromethyl)thieno[3,2-d]pyrimidin-4-yl]amino}propyl]piperazin-1-yl}sulfonyl)-4-methyl-1,3-thiazol-2-yl]carbamate |
| 35 | 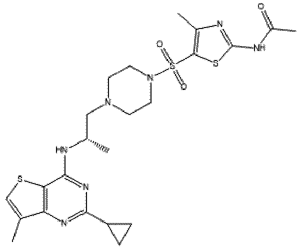 | N-[5-({4-[(2S)-2-({2-cyclopropyl-7-methylthieno[3,2-d]pyrimidin-4-yl}amino)propyl]piperazin-1-yl}sulfonyl)-4-methyl-1,3-thiazol-2-yl]acetamide |
| 36 | 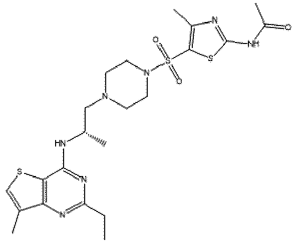 | N-[5-({4-[(2S)-2-({2-ethyl-7-methylthieno[3,2-d]pyrimidin-4-yl}amino)propyl]piperazin-1- yl}sulfonyl)-4-methyl-1,3-thiazol-2-yl]acetamide |
| 37 | 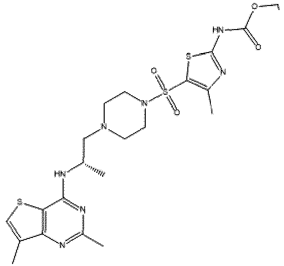 | methyl N-[5-({4-[(2S)-2-({2,7-dimethylthieno[3,2-d]pyrimidin-4-yl} amino)propyl]piperazin-1- yl}sulfonyl)-4-methyl-1,3- thiazol-2-yl]carbamate |
| 38 | 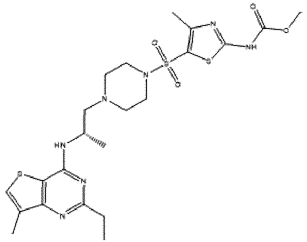 | methyl N-[5-({4-[(2S)-2-({2-ethyl-7-methylthieno[3,2-d]pyrimidin-4-yl}amino)propyl]piperazin-1- yl}sulfonyl)-4-methyl-1,3-thiazol-2-yl]carbamate |
| 39 | 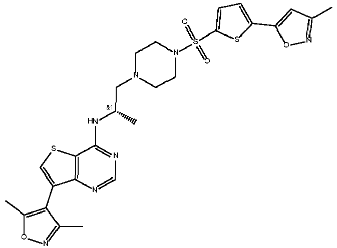 | 7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine |
| 40 | 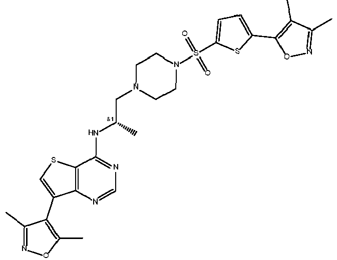 | 7-(3,5-dimethyl-1,2-oxazol-4-yl)-N-[(2S)-1-(4-{[5-(3,4-dimethyl-1,2-oxazol-5-yl)thiophen-2-yl]sulfonyl}piperazin-1-yl)propan-2-yl]thieno[3,2-d]pyrimidin-4-amine |
| 41 | 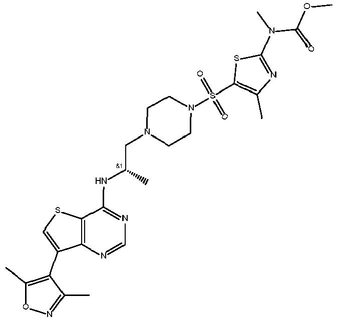 | methyl N-[5-({4-[(2S)-2-{[7-(3,5-dimethyl-1,2-oxazol-4-yl)thieno[3,2-d]pyrimidin-4-yl]amino}propyl]piperazin-1- yl}sulfonyl)-4-methyl-1, 3-thiazol-2-yl]-N-methylcarbamate |
| 42 | 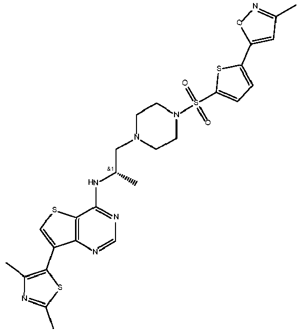 | 7-(2,4-dimethyl-1,3-thiazol-5-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine |
| 43 | 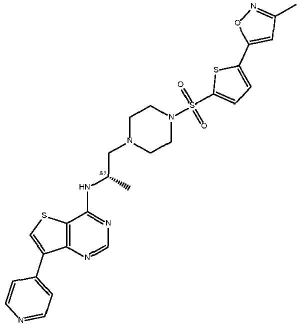 | N-[(2S)-1-(4-{[5-(3-methyl-1,2-oxazol-5-yl)thiophen-2-yl]sulfonyl}piperazin-1-yl)propan-2-yl]-7-(pyridin-4-yl)thieno[3,2-d]pyrimidin-4-amine |
| 44 | 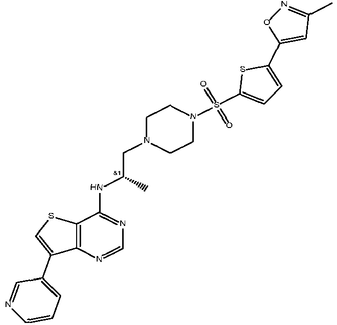 | N-[(2S)-1-(4-{[5-(3-methyl- 1,2-oxazol-5-yl)thiophen-2-yl]sulfonyl}piperazin-1- yl)propan-2-yl]-7-(pyridin-3-yl)thieno[3,2-d]pyrimidin-4-amine |
| 45 | 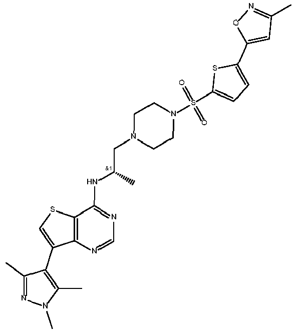 | N-[(2S)-1-(4-{[5-(3-methyl-1,2-oxazol-5-yl)thiophen-2-yl]sulfonyl}piperazin-1-yl)propan-2-yl]-7-(1,3,5-trimethyl-1H-pyrazol-4-yl)thieno[3,2-d]pyrimidin-4-amine |
| 46 | 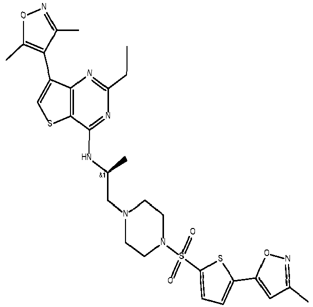 | 7-(3,5-dimethyl-1,2-oxazol-4-yl)-2-ethyl-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine |
| 47 | 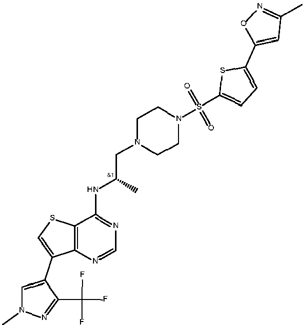 | N-[(2S)-1-(4-{[5-(3-methyl-1,2-oxazol-5-yl)thiophen-2-yl]sulfonyl}piperazin-1-yl)propan-2-yl]-7-[1-methyl-3-(trifluoromethyl)-1H-pyrazol-4-yl]thieno[3,2-d]pyrimidin-4-amine |
| 48 | 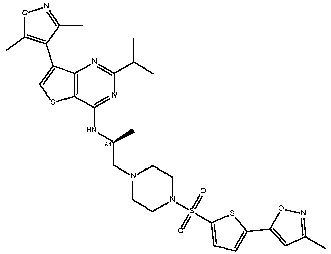 | 7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]-2-(propan-2-yl)thieno[3,2-d]pyrimidin-4-amine |
| 49 | 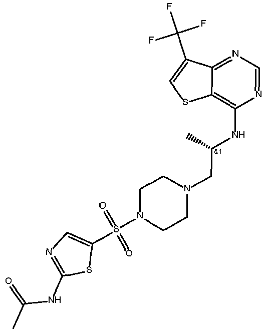 | N-[5-({4-[(2S)-2-{[7-(trifluoromethyl)thieno[3,2-d]pyrimidin-4-yl]amino}propyl]piperazin-1-yl}sulfonyl)-1,3-thiazol-2-yl]acetamide |
| 50 | 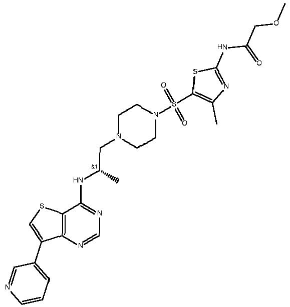 | 2-methoxy-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]acetamide |
| 51 | 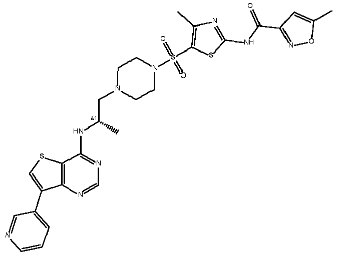 | 5-methyl-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]-1,2-oxazole-3-carboxamide |
| 52 | 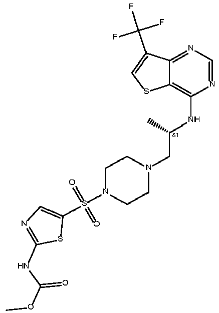 | methyl N-[5-({4-[(2S)-2-{[7-(trifluoromethyl)thieno[3,2-d]pyrimidin-4-yl]amino}propyl]piperazin-1-yl}sulfonyl)-1,3-thiazol-2-yl]carbamate |
| 53 | 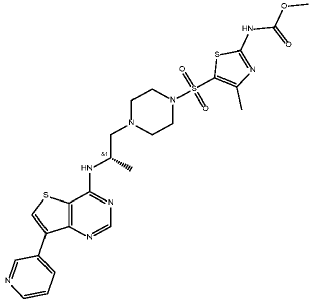 | methyl N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]carbamate |
| 54 | 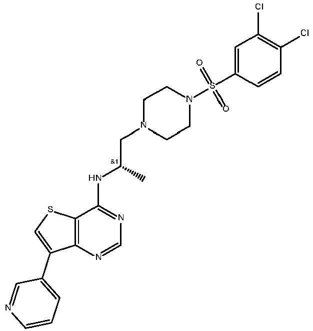 | N-[(2S)-1-[4-(3,4-dichlorobenzenesulfonyl)pipe razin-1-yl]propan-2-yl]-7-(pyridin-3-yl)thieno[3,2-d]pyrimidin-4-amine |
| 55 | 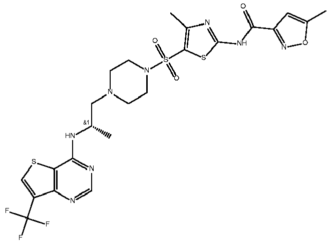 | 5-methyl-N-[4-methyl-5-({4-[(2S)-2-{[7-(trifluoromethyl)thieno[3,2-d]pyrimidin-4-yl]amino}propyl]piperazin-1-yl}sulfonyl)-1,3-thiazol-2-yl]-1,2-oxazole-3-carboxamide |

The compounds of the present invention can be prepared in a number of ways known to one skilled in the art of organic synthesis. It will be understood by those skilled in the art of organic synthesis that the functionality present on the molecule should be consistent with the transformation proposed. This will sometimes require a modification of the order of synthetic steps in order to obtain a desired compound of the invention. The compounds of formula (I), including all the compounds here above listed, can be generally prepared according to the procedure outlined in Schemes shown below using generally known methods.

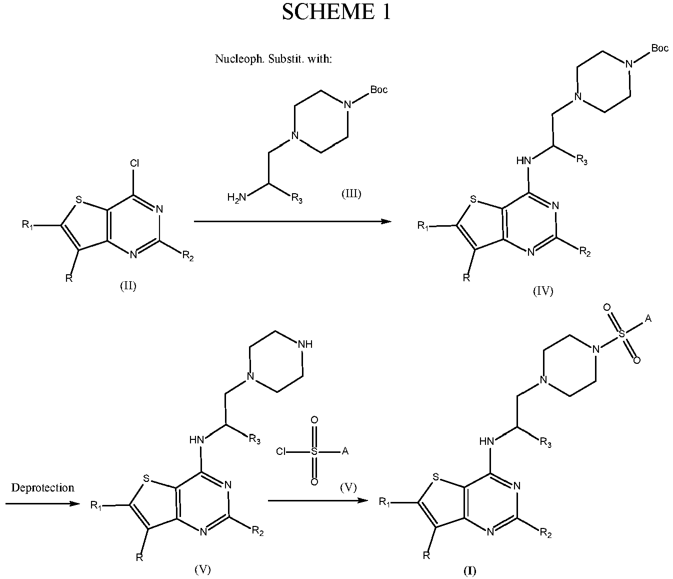

Compound of formula (II) can be reacted with a nitrogen based nucleophile of formula (III), in the presence of a suitable base e.g. N,N-diisopropylethylamine in a suitable solvent such as Acetonitrile, to provide compound (IV), containing a Boc-protected amino group. Deprotection under well-known procedures give compound (V) and final reaction with a suitable sulphonyl chloride (VI) led to compound of formula (I).

The examples 4-21, 23-30 of the present invention can be prepared following the synthetic route outlined in Scheme 1.

In another embodiment of the present invention, wherein R= CF₃ and R₂=H, compound (IXa) may be prepared according to Scheme 2 from commercially available dihalo thienopyrimidine (VI) by conversion in the corresponding iodide, through Grignard formation, followed by trifluoromethylation reaction using a suitable reagent like for example methyl 2,2-difluoro-2-(fluorosulfonyl)acetate. Compound (VIII) is then converted to the final compound (IXa) by following the synthetic sequence previously described in Scheme 1. The examples 1 and 49 of the present invention can be prepared following the synthetic route outlined in Scheme 2.

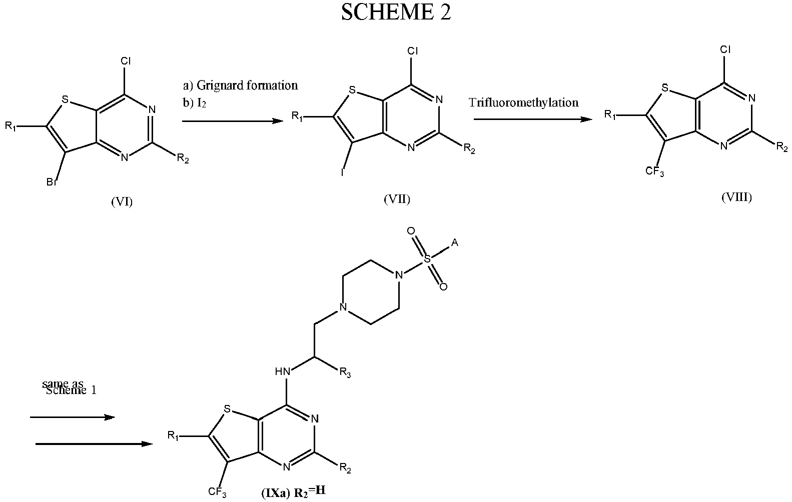

For compounds where R= CF₃ and R2^H, compound (IXb) can be synthesized as outlined in Scheme 3. Compound of formula (X), corresponding to Intermediate 7-5 in WO 2013078765, can be reacted with a nitrogen based nucleophile of formula (III) to provide compound (XI), containing a Boc-protected amino group. Wherein R2=Cl, compound (XI) is directly converted to the final compound (IX) by following the synthetic sequence previously described in Scheme 1. For compounds where R2≠H, Cl intermediate (XI) undergoes Suzuki coupling in the presence of a suitable Palladium catalyst e.g. [1,1'-Bis(diphenylphosphino)ferrocene]dichloropalladium(II), to introduce suitable R2 substituent and then is submitted to the same synthetic sequence outlined in Scheme 1 to obtain final compound (IXb). The examples 32 and 34 of the present invention can be prepared following the synthetic route outlined in Scheme 3.

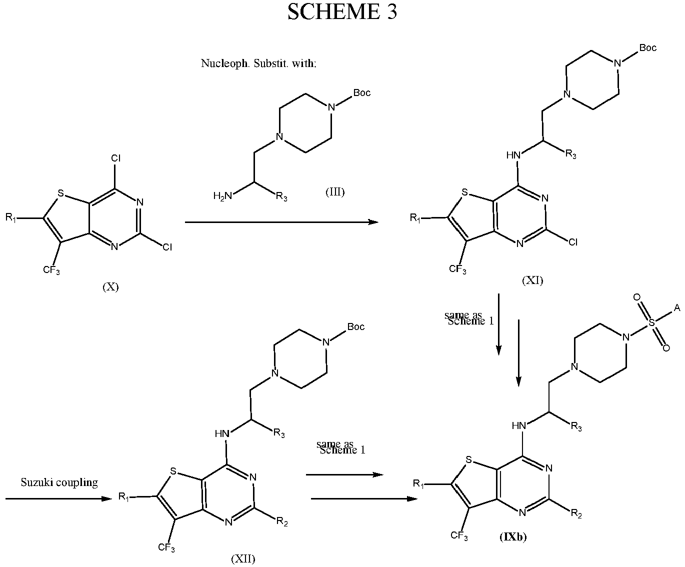

In another embodiment of the present invention, wherein R= Aryl, Heteroaryl, intermediate (II) is obtained following the synthetic route outlined in Scheme 4. Dihalo thienopyrimidine (VI) can be converted in the methylsulfanyl-derivative (XIII), which undergoes Suzuki coupling with commercially available boronic acid to provide intermediate (XIV). Subsequent reaction of compound (XI) with sulfuryl dichloride provide intermediate (II) that is converted to the final compound (I) by following the synthetic sequence previously described in Scheme 1. The examples 2, 39, 40, 42-45, 47, 53-54 of the present invention can be prepared following the synthetic route outlined in Scheme 4.

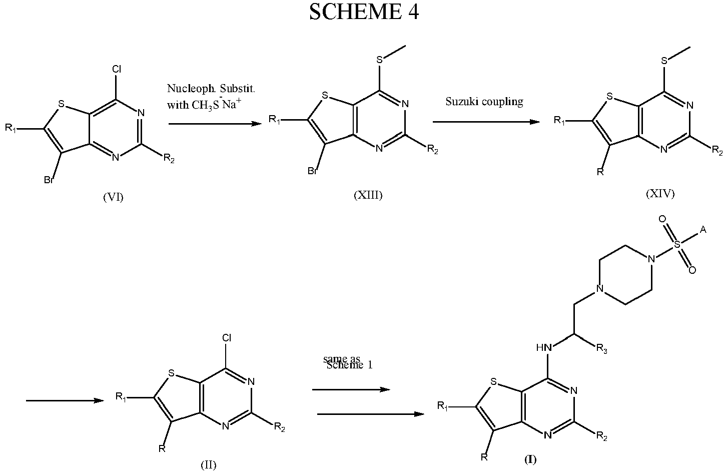

Alternatively, according to Scheme 5, for compounds wherein R= Aryl, Heteroaryl, intermediate (IV) can be obtained from nucleophilic substitution with a nitrogen based nucleophile of formula (III) on compound (VI) and subsequent Suzuki coupling with commercially available boronic acid. Compound (IV) is then converted to the final compound (I) by following the synthetic sequence previously described in Scheme 1.

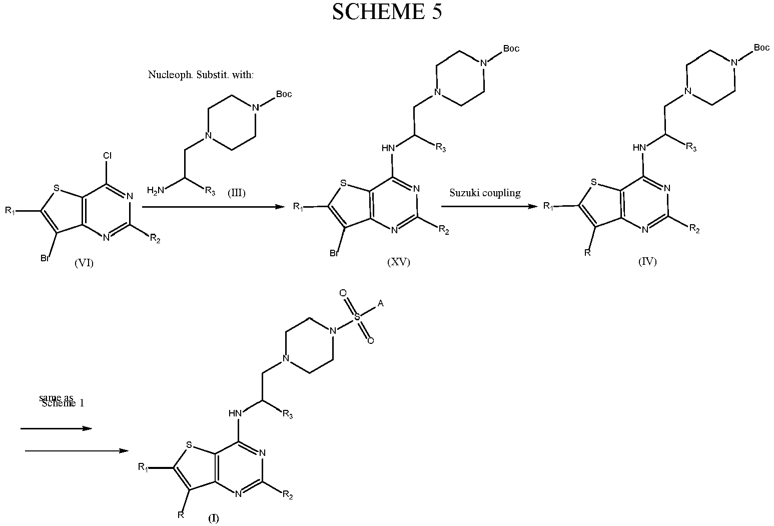

In another embodiment of the present invention, compound (II), where R2#I, can be synthesized as reported in Scheme 6.

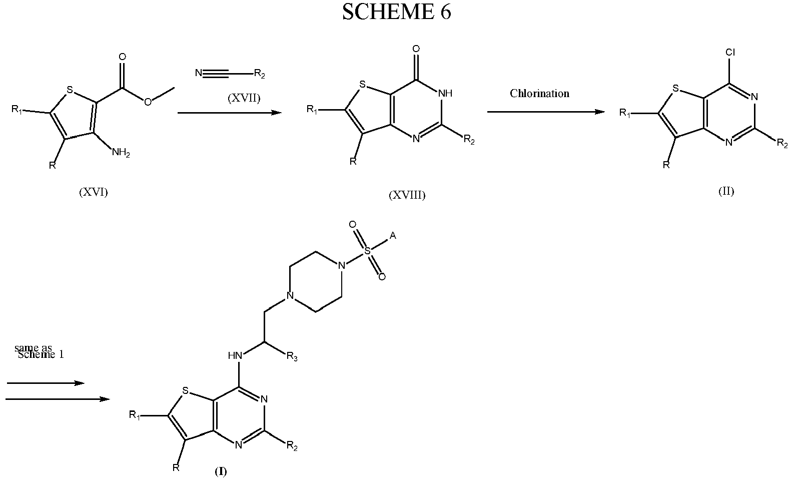

Cyclization of commercially available compound (XVI) with the appropriate nitrile (XVII) and subsequent chlorination with a suitable chlorinating agent such as POCl₃ provide intermediate (II). Compound (II) is then converted to the final compound (I) by following the synthetic sequence previously outlined in Scheme 1. The examples 22, 31, 35-38 of the present invention can be prepared following the synthetic route outlined in Scheme 6.

For compounds wherein R2^H and R= Aryl, Heteroaryl, intermediate (IV) is obtained following the synthetic route outlined in Scheme 7.

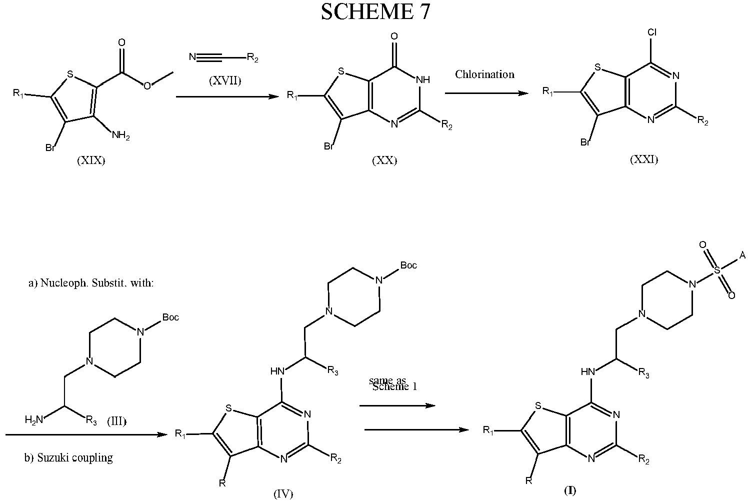

Cyclization of commercially available compound (XIX) with the appropriate nitrile (XIV) followed by chlorination with a suitable chlorinating agent such as POCl₃ provide intermediate (XXI). Nucleophilic substitution with a nitrogen based nucleophile of formula (III) and subsequent Suzuki coupling with commercially available boronic acid give intermediate (IV). Compound (IV) is then converted to the final compound (I) by following the synthetic sequence previously described in Scheme 1. The examples 3, 46 and 48 of the present invention can be prepared following the synthetic route outlined in Scheme 7.

In another embodiment of the present invention, wherein A is an N-acylated aminothiazole with R4 and R5= H, CH₃, compound (XXVI) may be obtained according to Scheme 8. Reaction of intermediate (V) with N-acetyl thiazole sulfonyl chloride (XXII) followed by deacetylation under acid condition and final acetylation with a suitable acyl chloride in presence of a base such as N,N-dimethyl-4-pyridinamine led to final compound (XXVI). The examples 33, 41, 50-52, 55 of the present invention can be prepared following the synthetic route outlined in Scheme 8.

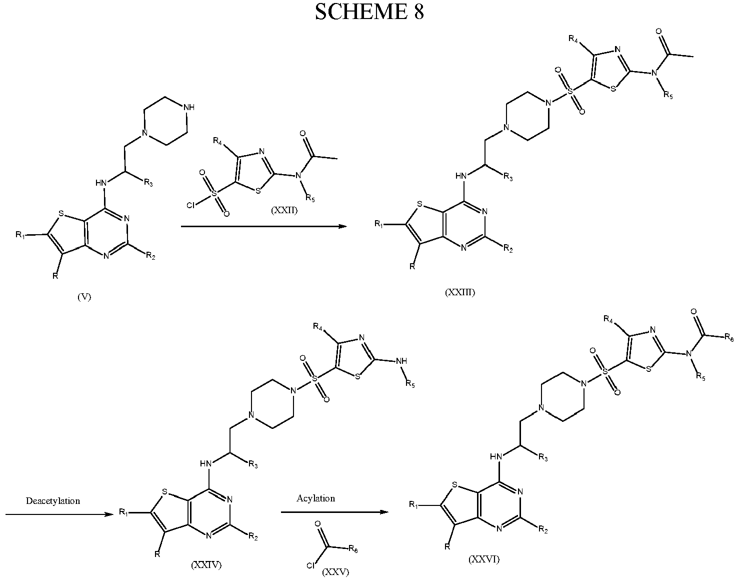

The compounds of formula (I) of the present invention have surprisingly been found to effectively inhibit receptor LPA2. Advantageously, the inhibition of LPA2 may result in an efficacious treatment of the diseases or condition wherein the LPA receptors are involved.

In particular in this respect, it has now been found that the compounds of formula (I) of the present invention have an antagonist drug potency expressed as half maximal inhibitory concentration (IC₅₀) on LPA2 lesser or equal than 1000 nM as shown in the present experimental part.

Preferably, the compounds of the present invention have an IC₅₀ on LPA2 lesser or equal than 100 nM.

More preferably, the compounds of the present invention have an IC₅₀ on LPA2 lesser or equal than 10 nM.

In one aspect, the present invention refers to a compound of formula (I) for use as a medicament.

In a preferred embodiment, the invention refers to a compound of formula (I) for use in the treatment of disorders associated with LPA receptors mechanism.

In a further embodiment, the present invention refers to a compound of formula (I) for use in the treatment of a disease, disorder or condition associated with dysregulation of lysophosphatidic acid receptor 2 (LPA2).

In one embodiment, the present invention refers to a compound of formula (I) useful for the prevention and/or treatment of fibrosis and/or diseases, disorders, or conditions that involve fibrosis.

The terms "fibrosis" or "fibro sing disorder," as used herein, refers to conditions that are associated with the abnormal accumulation of cells and/or fibronectin and/or collagen and/or increased fibroblast recruitment and include but are not limited to fibrosis of individual organs or tissues such as the heart, kidney, liver, joints, lung, pleural tissue, peritoneal tissue, skin, cornea, retina, musculoskeletal and digestive tract.

Preferably, the compounds of formula (I) of the present invention are useful for the treatment and/or prevention of fibrosis such as pulmonary fibrosis, idiopathic pulmonary fibrosis (IPF), hepatic fibrosis, renal fibrosis, ocular fibrosis, cardiac fibrosis, arterial fibrosis and systemic sclerosis.

More preferably, the compounds of formula (I) of the present invention are useful for the treatment of idiopathic pulmonary fibrosis (IPF).

In one aspect, the disclosure also refers to a method for the prevention and/or treatment of disorders associated with LPA receptors mechanisms, said method comprises administering to a patient in need of such treatment a therapeutically effective amount of a compound of formula (I).

In one aspect, the disclosure refers to the use of a compound of formula (I) in the preparation of a medicament for the treatment of disorders associated with LPA receptors mechanism.

In a further aspect, the disclosure refers to a method for the prevention and/or treatment of disorder or condition associated with dysregulation of lysophosphatidic acid receptor 2 (LPA2) administering a patient in need of such treatment a therapeutically effective amount of a compound of formula (I).

In a further aspect, the invention refers to the use of a compound of formula (I) according to the invention, or a pharmaceutically acceptable salt thereof, for the treatment of disorders associated with LPA receptors mechanism.

In a further aspect, the present disclosure refers to the use of a compound of formula (I) for the treatment of a disease, disorder or condition associated with dysregulation of receptor 2 (LPA2).

As used herein, "safe and effective amount" in reference to a compound of formula (I) or a pharmaceutically acceptable salt thereof or other pharmaceutically-active agent means an amount of the compound sufficient to treat the patient's condition but low enough to avoid serious side effects and it can nevertheless be routinely determined by the skilled artisan.

The compounds of formula (I) or pharmaceutically acceptable salts thereof may be administered once or according to a dosing regimen wherein a number of doses are administered at varying intervals of time for a given period of time. Typical daily dosages may vary depending upon the route of administration chosen.

The present invention also refers to a pharmaceutical composition comprising a compound of formula (I) in admixture with at least one or more pharmaceutically acceptable carrier or excipient.

In one embodiment, the invention refers to a pharmaceutical composition of compounds of formula (I) in admixture with one or more pharmaceutically acceptable carrier or excipient, for example those described in Remington's Pharmaceutical Sciences Handbook, XVII Ed., Mack Pub., N.Y., U.S.A.

Administration of the compounds of the invention and their pharmaceutical compositions may be accomplished according to patient needs, for example, orally, nasally, parenterally (subcutaneously, intravenously, intramuscularly, intrasternally and by infusion) and by inhalation.

Preferably, the compounds of the present invention are administered orally or by inhalation.

More preferably, the compounds of the present invention are administered orally.

In one preferred embodiment, the pharmaceutical composition comprising the compound of formula (I) is a solid oral dosage form such as tablets, gelcaps, capsules, caplets, granules, lozenges and bulk powders.

In one embodiment, the pharmaceutical composition comprising the compound of formula (I) is a tablet.

The compounds of the invention can be administered alone or combined with various pharmaceutically acceptable carriers, diluents (such as sucrose, mannitol, lactose, starches) and known excipients, including suspending agents, solubilizers, buffering agents, binders, disintegrants, preservatives, colorants, flavorants, lubricants and the like.

In a further embodiment, the pharmaceutical composition comprising a compound of formula (I) is a liquid oral dosage forms such as aqueous and non-aqueous solutions, emulsions, suspensions, syrups, and elixirs. Such liquid dosage forms can also contain suitable known inert diluents such as water and suitable known excipients such as preservatives, wetting agents, sweeteners, flavorants, as well as agents for emulsifying and/or suspending the compounds of the invention.

In a further embodiment, the pharmaceutical composition comprising the compound of formula (I) is an inhalable preparation such as inhalable powders, propellant-containing metering aerosols or propellant-free inhalable formulations.

For administration as a dry powder, single- or multi-dose inhalers known from the prior art may be utilized. In that case the powder may be filled in gelatine, plastic or other capsules, cartridges or blister packs or in a reservoir.

A diluent or carrier chemically inert to the compounds of the invention, e.g. lactose or any other additive suitable for improving the respirable fraction may be added to the powdered compounds of the invention.

Inhalation aerosols containing propellant gas such as hydrofluoroalkanes may contain the compounds of the invention either in solution or in dispersed form. The propellant-driven formulations may also contain other ingredients such as co-solvents, stabilizers and optionally other excipients.

The propellant-free inhalable formulations comprising the compounds of the invention may be in form of solutions or suspensions in an aqueous, alcoholic or hydroalcoholic medium and they may be delivered by jet or ultrasonic nebulizers known from the prior art or by soft-mist nebulizers.

The compounds of the invention can be administered as the sole active agent or in combination with other pharmaceutical active ingredients.

The dosages of the compounds of the invention depend upon a variety of factors including among others the particular disease to be treated, the severity of the symptoms, the route of administration and the like.

The invention is also directed to a device comprising a pharmaceutical composition comprising a compound of Formula (I) according to the invention, in form of a single- or multi-dose dry powder inhaler or a metered dose inhaler.

All preferred groups or embodiments described above for compounds of formula I may be combined among each other and apply as well *mutatis mutandis.*

The various aspects of the invention described in this application are illustrated by the following examples which are not meant to limit the invention in any way.

### PREPARATIONS OF INTERMEDIATES AND EXAMPLES

Chemical Names of the compounds were generated with Structure To Name Enterprise 10.0 Cambridge Software.

All reagents, for which the synthesis is not described in the experimental part, are either commercially available, or are known compounds or may be formed from known compounds by known methods by a person skilled in the art.

### ABBREVIATION - MEANING

AcOEt= Ethyl acetate
CsCO₃= Cesium carbonate
CyHex= Cyclohexane
DCM= Dichloromethane
DMF= Dimethylformamide
DMSO= Dimethylsulfoxide
h= hour
HCl= Hydrochloric acid
HCOOH= Formic acid
H₂O= Water
K₃PO₄= Potassium phosphate tribasic
LC-MS= liquid chromatography/mass spectrometry
MeCN= Acetonitrile
MW= microwave
NaCl= Sodium chloride
Na₂CO₃= Sodium carbonate
NaHCO₃= Sodium hydrogen carbonate
Na₂SO₃= Sodium sulfite
Na₂SO₄= Sodium sulfate
NH₄Cl= Ammonium chloride
NH₃= Ammonia
N₂= Nitrogen
Pd(dppf)Cl₂= [1,1'-Bis(diphenylphosphino)ferrocene]dichloropalladium(II)
Pd(PPh₃)₄= Tetrakis(triphenylphosphine)-palladium(0)
POCl₃= Phosphorus(V) oxychloride
r.t.= room temperature
THF= Tetrahydrofuran
UPLC=ultra-performance liquid chromatography

### General Experimental Details

### Analytical method

### Instruments, materials and methods employed for analyses

### ¹H-NMR

¹H-NMR spectra were performed on a Varian MR-400 spectrometer operating at 400 MHZ (proton frequency), equipped with: a self-shielded Z-gradient coil 5 mm 1H/nX broadband probe head for reverse detection, deuterium digital lock channel unit, quadrature digital detection unit with transmitter offset frequency shift, or on AgilentVNMRS-500 or on a Bruker Avance 400 or on a Bruker Avance 300 spectrometers. Chemical shift are reported as 6 values in ppm relative to trimethylsilane (TMS) as an internal standard. Coupling constants (*J* values) are given in hertz (Hz) and multiplicities are reported using the following abbreviation (s=singlet, d=doublet, t=triplet, q=quartet, m=multiplet, br. s=broad singlet, nd=not determined).

### LC/UV/MS

LC/MS retention times are estimated to be affected by an experimental error of +0.5 min. LCMS may be recorded under the following conditions: diode array DAD chromatographic traces, mass chromatograms and mass spectra may be taken on UPLC/PDA/MS Acquity^{™} system coupled with Micromass ZQ^{™} or Waters SQD single quadrupole mass spectrometer operated in positive and/or negative electron spray ES ionization mode and/or Fractionlynx system used in analytical mode coupled with ZQ^{™} single quadrupole operated in positive and/or negative ES ionization mode or Waters Alliance e2695 with Photodiode Detector 2998 coupled with Column Oven and Mass Spectrometer ZQ operated in positive and/or negative ES ionization mode. Quality Control methods used operated under low pH conditions or under high pH conditions:
Method 1, low pH conditions: column: Acquity CSH C18 2.1x50mm 1.7um, the column temperature was 40°C; mobile phase solvent A was milliQ water+0.1% HCOOH, mobile phase solvent B MeCN+0.1% HCOOH. The flow rate was 1 mL/min. The gradient table was t=0 min 97% A 3% B, t=1.5 min 0.1% A 99.9% B, t=1.9 min 0.1% A 99.9% B and t=2 min 97% A 3% B. The UV detection range was 210-350 nm and ES+/ES- range was 100 to 1500 AMU.
Method 2, high pH conditions: column: Acquity Kinetex 1.7 um EVO C18 100A, 2.1×50mm, the column temperature was 40°C; mobile phase solvent A was 10 mM aqueous solution of NH₄HCO₃ adjusted to pH=10 with ammonia, mobile phase solvent B MeCN. The flow rate was 1 mL/min. The gradient table was t=0 min 97% A 3% B, t=1.5 min 0.1% A 99.9% B, t=1.9 min 0.1% A 99.9% B and t=2 min 97% A 3% B. The UV detection range was 210-350 nm and ES+/ES- range was 100 to 1500 AMU.
Method 3, low pH conditions: column: Acquity CSH C18 2.1×50mm 1.7µm, the column temperature was 40°C; mobile phase solvent A was milliQ water/MeCN 95:5 +0.05% HCOOH, mobile phase solvent B MeCN/milliQ water 95:5 +0.05% HCOOH. The flow rate was 1 mL/min. The gradient table was t=0 min 99% A 1% B, t=1.5 min 0.1% A 99.9% B, t=1.9 min 0.1% A 99.9% B and t=2 min 99% A 1% B. The UV detection range was 210-400 nm and ES+/ES- range was 100 to 1200 AMU.
Method 4, low pH conditions: column: Phenomenex Gemini-NX C18, 150×2.0mm, 3µm with security guard Gemini-NX C18, 4×2.0mm, 3µm, the column temperature was 25°C; mobile phase solvent A was water + 0.1% Formic acid filtered with 0.22 µm nylon filter, mobile phase solvent B Acetonitrile + 0.1% Formic acid filtered with 0.22 µm nylon filter. The flow rate was 0.2 mL/min. The gradient table was t=0 min 95% A 5% B, t=10 min 20% A 80% B, t=30 min 20% A 80 B. The UV detection λ was 210 nm and ES+/ES-range was 50 to 900 Da.
Method 5, low pH conditions: column: Acquity CSH C18 2.1×50mm 1.7µm, the column temperature was 40°C; mobile phase solvent A was milliQ water/MeCN 95:5 +0.05% HCOOH, mobile phase solvent B MeCN/milliQ water 95:5 +0.05% HCOOH. The flow rate was 1 mL/min. The gradient table was t=0 min 99% A 1% B, t=3.5 min 0.1% A 99.9% B, t=3.9 min 0.1% A 99.9% B and t=4 min 99% A 1% B. The UV detection range was 210-400 nm and ES+/ES- range was 100 to 1200 AMU.

### Example 1

### methyl N-[4-methyl-5-(14-[(2S)-2-1[7-(trifluoromethyl)thieno[3,2-d]pyrimidin-4-yl]amino)propyl]piperazin-1-yl]sulfonyl)-1,3-thiazol-2-yl]carbamate

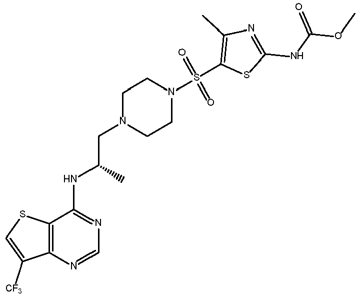

### Step 1: Preparation of 4-chloro-7-iodothieno[3,2-d]pyrimidine (Intermediate 1)

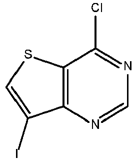

7-bromo-4-chlorothieno[3,2-d]pyrimidine (1 g, 4 mmol) was dissolved in THF (10 mL), cooled at 0°C and added dropwise with isopropylmagnesium chloride 2.0M solution in THF (3 mL, 6 mmol). After stirring for 30 minutes at 0°C, the mixture was treated dropwise with a solution of iodine (1.53 g, 6 mmol) in THF (20 mL). After 1 hour at 0 °C the reaction was quenched by addition of saturated aqueous NH₄Cl and partitioned between AcOEt and water. The organic phase was washed with a saturated solution of Na₂SO₃. The organic layer was dried over Na₂SO₄, filtered and concentrated under reduced pressure to give a crude material that was purified by flash chromatography eluting with MeCN (from 0% to 40%) in water to provide title compound (850 mg, 2.87 mmol, 71% yield) as a brown solid.
LC-MS (ESI): *m*/*z* (M+1): 297 (Method 1)
¹H NMR (400 MHz, DMSO-d6) δ ppm 9.16 (s, 1H), 8.84 (s, 1H)

### Step 2: Preparation of 4-chloro-7-(trifluoromethyl)thieno[3,2-d]pyrimidine (Intermediate 2)

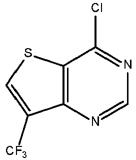

Copper (I) iodide (576.28 mg, 3 mmol) was dissolved in DMF (15 mL) and methyl 2,2-difluoro-2-(fluorosulfonyl)acetate (0.73 mL, 5.7 mmol) was added. The resulting solution was stirred for 5 minutes at room temperature. Intermediate 1 (850 mg, 2.87 mmol) was added and the reaction was stirred at 80 °C overnight and then 3 days at room temperature. The mixture was filtered and the solid was washed with ethyl acetate. The filtrate was washed with NaCl sat. sol. (3 times). The organic phase was dried over Na₂SO₄, filtered and evaporated under reduced pressure. The crude was purified by flash chromatography eluting with AcOEt in cyclohexane (form 0% to 10%) to give the title compound (459 mg, 1.9 mmol, 67% yield) as a yellowish solid.
LC-MS (ESI): *m*/*z* (M+1): 239 (Method 1)
¹H NMR (400 MHz, *DMSO-d6*) δ ppm 9.29 (s, 1H), 9.21 (s, 1H)

### Step 3: Preparation of tert-butyl 4-[(2S)-2-[[7-(trifluoromethyl)thieno[3,2-d]pyrimidin-4-yl]amino]propyl]piperazine-1-carboxylate (Intermediate 3)

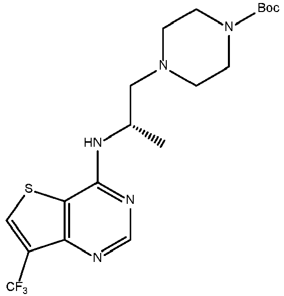

To a solution of Intermediate 2 (459 mg, 1.9 mmol) in MeCN (15 mL), tert-butyl 4-[(2S)-2-aminopropyl]piperazine-1-carboxylate (468 mg, 1.9 mmol) was added, followed by N,N-diisopropylethylamine (0.37 mL, 2.12 mmol). The solution was heated at 40 °C overnight. The mixture was diluted with water and AcOEt and the aqueous phase was extracted with AcOEt (2 times). The combined organic layers were dried over Na₂SO₄, filtered and concentrated under reduced pressure. The crude was purified by flash chromatography eluting with AcOEt in cyclohexane (from 0% to 60%) to provide title compound (450 mg, 1 mmol, 52% yield) as a white solid.
LC-MS (ESI): *m*/*z* (M+1): 446 (Method 1)
¹H NMR (400 MHz, *DMSO-d₆*) δ ppm 8.78 (s, 1H), 8.50 (s, 1H), 7.94 (d, *J* = 8.1 Hz, 1H), 4.61 (p, *J* = 6.9 Hz, 1H), 3.15 - 3.29 (m, 4H), 2.53 - 2.59 (m, 1H), 2.34 - 2.40 (m, 5H), 1.38 (s, 9H), 1.22 (d, *J* = 6.6 Hz, 3H)

### Step 4: Preparation of N-[(2S)-1-piperazin-1-ylpropan-2-yl]-7-(trifluoromethyl)thieno[3,2-d]pyrimidin-4-amine (Intermediate 4)

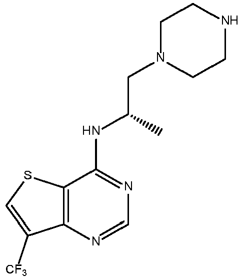

To a solution of Intermediate 3 (450 mg, 1 mmol), in DCM (12 mL), 2,2,2-trifluoroacetic acid (1.16 mL, 15 mmol) was added. The reaction was stirred at room temperature overnight. The mixture was then concentrated under reduced pressure and the residue was purified using a SCX cartridge eluting with a solution of NH₃ 2M in methanol to afford title compound (340 mg, 0.98 mmol, 97% yield) as a yellow solid.
LC-MS (ESI): *m*/*z* (M+1): 346 (Method 2)
¹H NMR (400 MHz, *DMSO-d₆*) δ ppm 8.78 (d, *J* = 1.1 Hz, 1H), 8.49 (s, 1H), 7.90 (d, *J* = 8.1 Hz, 1H), 4.60 (p, *J* = 6.7 Hz, 1H), 2.56 - 2.70 (m, 4H), 2.46 - 2.48 (m, 2H), 2.20 - 2.41 (m, 5H), 1.21 (d, *J* = 6.6 Hz, 3H)

### Step 5: Preparation of methyl N-[4-methyl-5-({4-[(2S)-2-{[7-(trifluoromethyl)thieno[3 ,2-d]pyrimidin-4-yl]amino}propyl]piperazin-1 -yl} sulfonyl)-1,3-thiazol-2-yl]carbamate (Example 1).

To a solution of Intermediate 4 (340 mg, 0.98 mmol) in pyridine/DCM 3:2 (5 mL), methyl N-[5-(chlorosulfonyl)-4-methyl-1,3-thiazol-2-yl]carbamate (94.05 mg, 0.350 mmol) was added and the reaction was stirred at r.t. for 12 hours. The solvent was removed under vacuum and the crude product was purified by flash chromatography eluting with AcOEt in cyclohexane from 0% to 25% to afford title compound (22.6 mg, 0.039 mmol, 13.47% yield) as a white solid.
LC-MS (ESI): *m*/*z* (M+1): 580 (Method 1)
¹H NMR (400 MHz, *DMSO-d₆*) δ ppm 12.37 (br s, 1 H), 8.76 (s, 1 H), 8.48 (s, 1 H), 7.91 (d, *J*=8.0 Hz, 1 H), 4.53 (dt, *J*=13.9, 7.1 Hz, 1 H), 3.73 (s, 3 H), 2.97 (br s, 4 H), 2.52 - 2.60 (m, 5 H), 2.41 (s, 3 H), 2.32 - 2.38 (m, 1 H), 1.18 (d, *J*=6.6 Hz, 3 H)

The Examples in the following table were prepared from commercially available reagents by using methods analogous to Example 1.

| **Example No.** | **Structure & Name** | **Analytical data** |
|---|---|---|
| 49 | 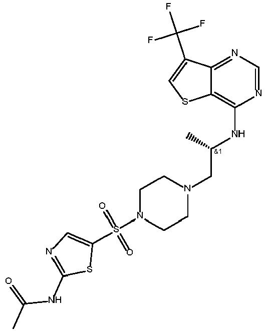 N-[5-({4-[(2S)-2-{ [7-(trifluoromethyl)thieno[3,2-d]pyrimidin-4-yl]amino}propyl]piperazin-1-yl} sulfonyl)-1,3 -thiazol-2-yl]acetamide | LC-MS (ESI): m/z (M+1): 550 (Method 1) |
| | | ¹H NMR (400 MHz, *DMSO-d₆*) δ ppm 12.68 (br s, 1 H), 8.75 (d, *J*=0.7 Hz, 1 H), 8.47 (s, 1 H), 7.80 - 8.03 (m, 2 H), 4.53 (dt, *J*=14.0, 6.9 Hz, 1 H), 2.91 (br s, 4 H), 2.52 - 2.62 (m, 5 H) 2.37 (dd, 7=12.3, 7.0 Hz, 1 H), 2.17 (s, 3 H), 1. 17 (d, *J*=6.4 Hz, 3 H) |

### Example 2

### methyl N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]carbamate

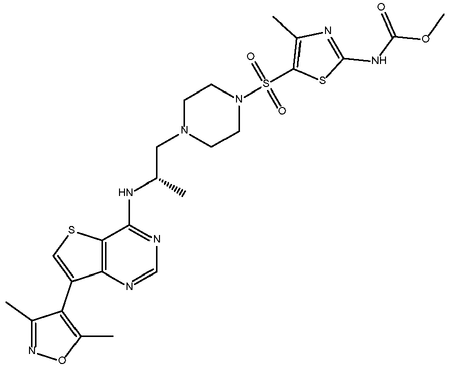

### Step 1: Preparation of 7-bromo-4-(methylsulfanyl)thieno[3,2-d]pyrimidine (Intermediate 5)

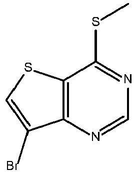

To a solution of 7-bromo-4-chlorothieno[3,2-d]pyrimidine (100 mg, 0.4 mmol) in THF (1.5ml), sodium methanethiolate (70 mg, 1 mmol) was added at 0°C. The mixture was stirred at r.t. overnight. Ice water was added and the resulting solid was filtered and dried to provide title compound (85 mg, 0.33 mmol, 83% yield) as a white solid.
LC-MS (ESI): *m*/*z* (M+1): 262.9 (Method 1)
¹H NMR (400 MHz, *DMSO-d6*) δ ppm 9.10 (s, 1H), 8.59 (s, 1H), 2.78 (s, 3H)

### Step 2: Preparation of 3,5-dimethyl-4-(4-methylsulfanylthieno[3,2-d]pyrimidin-7-yl)-1,2-oxazole (Intermediate 6)

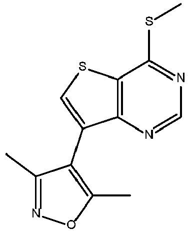

To a suspension of Intermediate 5 (85 mg, 0.33 mmol) and 3,5-dimethyl-4-(4,4,5,5-tetramethyl-1,3,2-dioxaborolan-2-yl)-1,2-oxazole (128 mg, 0.5 mmol) in water (0.7 mL), cesium carbonate (249 mg, 0.66 mmol) was added. After degassing with N₂, Pd(PPh₃)₄ (44 mg, 0.04 mmol) was added and the reaction was heated at 100 °C for 16h. After cooling, AcOEt was added and the crude mixture was washed with NaCl sat. sol. The organic layer was concentrated under reduced pressure. The crude was purified by flash chromatography eluting with AcOEt in cyclohexane from 0% to 20% affording title compound (26.5 mg, 0.096 mmol, 29% yield).
LC-MS (ESI): *m*/*z* (M+1): 278 (Method 1)

### Step 3: Preparation of 4-(4-chlorothieno[3,2-d]pyrimidin-7-yl)-3,5-dimethyl-1,2-oxazole (Intermediate 7)

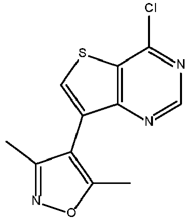

To a solution of Intermediate 6 (226.5 mg, 0.82 mmol) in DCM (2.5 mL) sulfuryl dichloride (0.26 mL, 3.27 mmol) was added and the reaction was stirred at 25 °C for 15 min. A saturated solution of NaHCO₃ followed by DCM was added, then the organic layer was separated and concentrated under reduced pressure to give title compound (323 mg, crude) that was used in the next step without further purification.
LC-MS (ESI): *m*/*z* (M+1): 266.3 (Method 1)

### Step 4: Preparation of tert-butyl 4-[(2S)-2-[[7-(3,5-dimethyl-1,2-oxazol-4-yl)thieno[3,2-d]pyrimidin-4-yl]amino]propyl]piperazine-1-carboxylate (Intermediate 8)

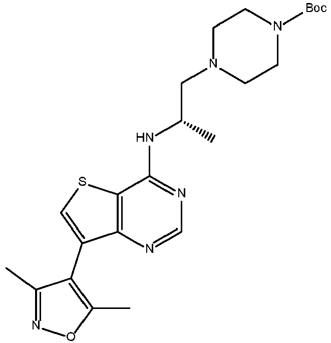

Title compound was prepared following the procedure used for the synthesis of Intermediate 4, starting from 4-(4-chlorothieno[3,2-d]pyrimidin-7-yl)-3,5-dimethyl-1,2-oxazole (Intermediate 7, 323 mg, crude) to afford title compound (247 mg, 0.52 mmol, 64% yield).
LC-MS (ESI): *m*/*z* (M+1): 472.9 (Method 2)

### Step 5: Preparation of 7-(3,5-dimethyl-1,2-oxazol-4-yl)-N-[(2S)-1-piperazin-1-ylpropan-2-yl]thieno[3,2-d]pyrimidin-4-amine hydrochloride (Intermediate 9)

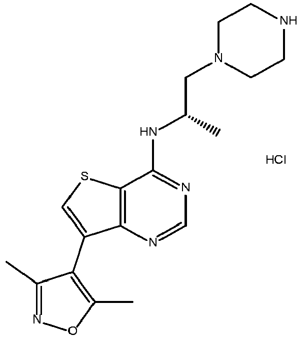

To a solution of tert-butyl 4-[(2S)-2-[[7-(3,5-dimethyl-1,2-oxazol-4-yl)thieno[3,2-d]pyrimidin-4-yl]amino]propyl]piperazine-1-carboxylate (Intermediate 8, 247 mg, 0.52 mmol) in 1,4-dioxane (3 mL), HCl 4M in 1,4-dioxane (0.65 mL, 2.61 mmol) was added. The reaction was stirred at r.t. for 3h. The mixture was then concentrated under reduced pressure to afford title compound (300 mg, crude).
LC-MS (ESI): *m*/*z* (M+1): 373.4 (Method 2)

### Step 5: Preparation of methyl N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]carbamate (Example 2)

Title compound was prepared following the procedure used for the synthesis of Example 1, starting from 7-(3,5-dimethyl-1,2-oxazol-4-yl)-N-[(2S)-1-piperazin-1-ylpropan-2-yl]thieno[3,2-d]pyrimidin-4-amine hydrochloride (Intermediate 9, 65 mg, 0.158 mmol) and methyl N-[5-(chlorosulfonyl)-4-methyl-1,3-thiazol-2-yl]carbamate (43.46 mg, 0.160 mmol) to afford the title compound (26.5 mg, 0.044 mmol, 28% yield) as a white solid.
LC-MS (ESI): *m*/*z* (M+1): 607.08 (Method 2)
¹H NMR (400 MHz, *DMSO-d₆*) δ ppm 12.33 (br s, 1 H), 8.42 (s, 1 H), 8.13 (s, 1 H), 7.67 (d, *J*=8.1 Hz, 1 H), 4.44 - 4.62 (m, 1 H), 3.75 (s, 3 H), 2.99 (br s, 4 H), 2.56 (br s, 5 H), 2.15 - 2.46 (m, 9 H), 1.18 (d, *J*=6.4 Hz, 3 H)

The Examples in the following table were prepared from commercially available reagents by using methods analogous to Example 2.

| **Example No.** | **Structure & Name** | **Analytical data** |
|---|---|---|
| 39 | 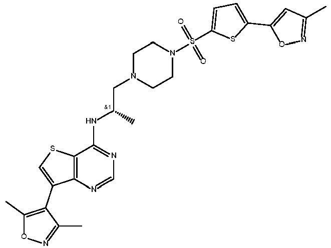 | LC-MS (ESI): m/z (M+1): 600.1 (Method 2) |
| | 7-(3,5-dimethyl-1,2-oxazol-4-yl)-N-[(2S)-1-(4-{[5-(3-methyl-1,2-oxazol-5-yl)thiophen-2-yl]sulfonyl }piperazin-1-yl)propan-2-yl]thieno[3,2-d]pyrimidin-4-amine | ¹H NMR (400 MHz, *DMSO-d₆*) δ ppm 8.41 (s, 1 H), 8.11 (s, 1 H), 7.76 (d, *J*=4.0 Hz, 1 H), 7.70 (d, *J*=4.0 Hz, 1 H), 7.65 (d, *J*=8.1 Hz, 1 H), 6.97 (s, 1 H), 4.55 (dt, *J*=14.1, 6.8 Hz, 1 H), 2.98 (br s, 4 H), 2.55 - 2.62 (m, 5 H), 2.31 - 2.42 (m, 4 H), 2.29 (s, 3 H), 2.17 (s, 3 H), 1.17 (d, *J*=6.6 Hz, 3 H) |
| 40 | 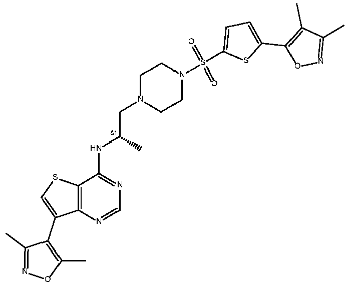 | LC-MS (ESI): m/z (M+1): 614.03 (Method 2) |
| | 7-(3,5-dimethyl-1,2-oxazol-4-yl)-N-[(2S)-1-(4-{[5-(3,4-dimethyl-1,2-oxazol-5-yl)thiophen-2-yl]sulfonyl}piperazin-1-yl)propan-2-yl]thieno[3,2-d]pyrimidin-4-amine | ¹H NMR (400 MHz, *DMSO-d₆*) δ ppm 8.41 (s, 1 H), 8.10 (s, 1 H), 7.61 - 7.74 (m, 3 H), 4.50 - 4.59 (m, 1 H), 2.98 (br s, 4 H), 2.54 - 2.63 (m, 5 H), 2.38 (dd, *J*=12.1, 6.8 Hz, 1 H), 2.14 - 2.34 (m, 12 H), 1.17 (d, *J*=6.6 Hz, 3 H) |
| 42 | 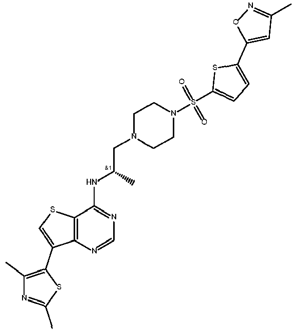 | LC-MS (ESI): m/z (M+1): 616 (Method 2) |
| | 7-(2,4-dimethyl-1,3-thiazol-5-yl)-N-[(2S)-1-(4-{[5-(3-methyl-1,2-oxazol-5-yl)thiophen-2-yl]sulfonyl} pi perazi n-1- yl)propan-2-yl]thieno[3,2-d]pyrimidin-4-amine | ¹H NMR (400 MHz, *DMSO-d₆*) δ ppm 8.46 (s, 1 H), 8.18 (s, 1 H), 7.75 (d, *J*=4.0 Hz, 1 H), 7.69 (d, *J*=4.4 Hz, 1 H), 7.65 - 7.69 (m, 1 H), 6.95 (s, 1 H), 4.61 - 4.48 (m, 1H), 2.88 - 3.05 (m, 4 H), 2.63 (s, 3 H), 2.53 - 2.61 (m, 5 H), 2.44 (s, 3 H), 2.38 (br dd, *J*=12.5, 6.8 Hz, 1 H), 2.28 (s, 3H), 1.16 (d, *J*=6.6 Hz, 3 H) |
| 43 | 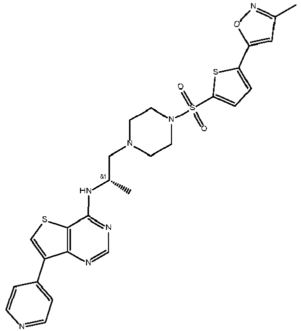 | LC-MS (ESI): m/z (M+1): 582 (Method 2) |
| | N-[(2S)-1-(4-{[5-(3-methyl-1,2-oxazol-5-yl)thiophen-2-yl]sulfonyl}piperazin-1-yl)propan-2-yl]-7-(pyridin-4-yl)thieno[3,2-d]pyrimidin-4-amine | ¹H NMR (400 MHz, *DMSO-d₆*) δ ppm 8.68 - 8.60 (m, 3 H), 8.52 (s, 1 H), 8.12 (d, *J*=6.0 Hz, 2 H), 7.75 (d, *J*=3.8 Hz, 1 H), 7.69 (br d, *J*=4.1 Hz, 2 H), 6.94 (s, 1 H), 4.61 - 4.51 (m, 1 H), 2.97 (br s, 4 H), 2.54 - 2.62 (m, 5 H), 2.39 (br dd, *J*=12.5, 6.7 Hz, 1 H), 2.28 (s, 3 H), 1.18 (d, *J*=6.3 Hz, 3 H) |
| 44 | 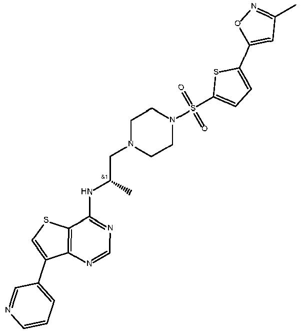 | LC-MS (ESI): m/z (M+1): 582 (Method 2) |
| | N-[(2S)-1-(4-{[5-(3-methyl-1,2-oxazol-5-yl)thiophen-2-yl]sulfonyl(piperazin-1-yl)propan-2-yl]-7-(pyridin-3-yl)thieno[3,2-d]pyrimidin-4-amine | ¹H NMR (400 MHz, *DMSO-d₆*) δ ppm 9.16 - 9.20 (m, 1 H), 8.56 (dd, *J*=4.7, 1.7 Hz, 1 H), 8.50 (s, 1 H), 8.45 (s, 1 H), 8.43 (dt, *J*=8.1, 1.9 Hz, 1 H), 7.75 (d, *J*=4.0 Hz, 1 H), 7.68 - 7.71 (m, 1 H), 7.66 (d, *J*=7.9 Hz, 1 H), 7.49 (dd, *J*=7.4, 4.7 Hz, 1 H), 6.95 (s, 1 H), 4.49 - 4.63 (m, 1 H), 2.97 (br s, 4 H), 2.53 - 2.66 (m, 5 H), 2.35 - 2.44 (m, 1 H), 2.28 (s, 3 H), 1.18 (d, *J*=6.6Hz, 3 H) |
| 45 | 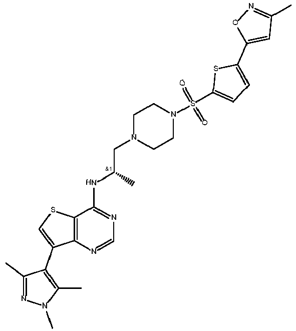 | LC-MS (ESI): m/z (M+1): 613.1 (Method 2) |
| | N-[(2S)-1-(4-{[5-(3-methyl-1,2-oxazol-5-yl)thiophen-2-yl]sulfonyl}piperazin-1- yl)propan-2-yl]-7-(1,3,5-trimethyl-1H-pyrazol-4-yl)thieno[3,2-d]pyrimidin-4-amine | ¹H NMR (400 MHz, *DMSO-d₆*) δ ppm 8.37 (s, 1 H), 7.81 (s, 1 H), 7.76 (d, J=3.9 Hz, 1 H), 7.70 (d, *J*=3.9 Hz, 1 H), 7.50 (d, *J*=8.1 Hz, 1 H), 6.97 (s, 1 H), 4.48 - 4.61 (m, 1 H), 3.70 (s, 3 H), 2.98 (br s, 4 H), 2.53 - 2.63 (m, 5 H), 2.37 (dd, *J*=12.4, 6.9 Hz, 1 H), 2.29 (s, 3 H), 2.11 (s, 3 H), 2.03 (s, 3 H), 1.16 (d, *J*=6.6 Hz, 3 H) |
| 47 | 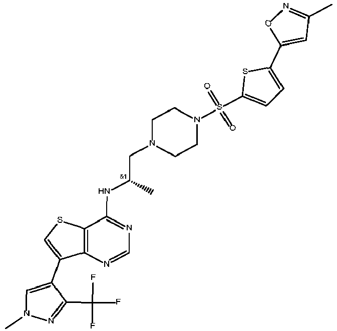 | LC-MS (ESI): m/z (M+1): 653 (Method 2) |
| | N-[(2S)-1-(4-{[5-(3-methyl-1,2-oxazol-5-yl)thiophen-2-yl]sulfonyl}piperazin-1-yl)propan-2-yl]-7-[1-methyl-3-(trifluoromethyl)-1H-pyrazol-4-yl]thieno[3,2-d]pyrimidin-4-amine | ¹H NMR (400 MHz, *DMSO*-d₆) δ ppm 8.49 (s, 1 H), 8.46 (s, 1 H), 7.97 (s, 1 H), 7.76 (d, *J*=3.9 Hz, 1 H), 7.69 (d, *J*=3.9 Hz, 1 H), 7.64 (d, *J*=7.9 Hz, 1 H), 6.95 (s, 1 H), 4.48 - 4.61 (m, 1 H), 4.00 (s, 3 H), 2.90 - 3.08 (m, 4 H), 2.52 - 2.64 (m, 5 H), 2.38 (br dd, *J*=12.3, 7.0 Hz, 1 H), 2.28 (s, 3H), 1.16 (d, *J*=6.6 Hz, 3 H) |
| 53 | 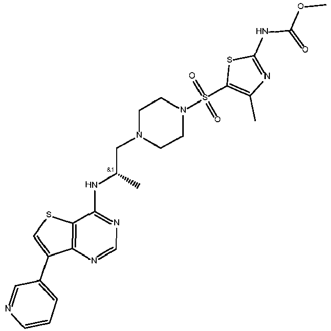 | LC-MS (ESI): m/z (M+1): 589.1 (Method 2) |
| | methyl N-[4-methyl-5-({4-[(2S)-2-{[7-(pyridin-3-yl)thieno[3,2-d]pyrimidin-4-yl]amino}propyl]piperazin-1-yl} sulfonyl)-1,3 -thiazol-2-yl]carbamate | ¹H NMR (400 MHz, *DMSO-d₆*) δ ppm 12.32 (br s, 1 H), 9.19 (dd, *J*=2.2, 0.7 Hz, 1 H), 8.56 (dd, *J*=4.7, 1.7 Hz, 1 H), 8.51 (s, 1 H), 8.47 (s, 1 H), 8.44 (dt, *J*=8.1, 1.9 Hz, 1 H), 7.67 (d, *J*=7.9 Hz, 1 H), 7.50 (ddd, *J*=8.0, 4.8, 0.8 Hz, 1 H), 4.56 (dt, *J*=13.9, 7.0 Hz, 1 H), 3.73 (s, 3H), 2.99 (br s, 4 H), 2.53 - 2.64 (m, 5 H), 2.42 (s, 3 H), 2.36 - 2.42 (m, 1 H), 1.20 (d, *J*=6.6 Hz, 3 H) |
| 54 | 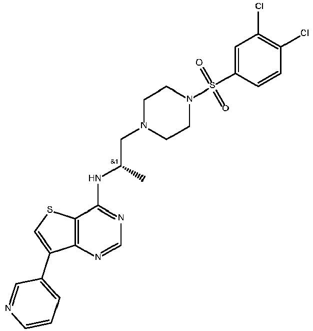 | LC-MS (ESI): m/z (M+1): 563 (Method 2) |
| | N-[(2S)-1-[4-(3,4-dichlorobenzenesulfonyl)piperazin-1-yl]propan-2-yl]-7-(pyridin-3-yl)thieno[3,2-d]pyrimidin-4-amine | ¹H NMR (400 MHz, *DMSO-d*_{*6*)} δ ppm 9.19 (dd, *J*=2.2, 0.8 Hz, 1 H), 8.56 (dd, *J*=4.7, 1.7 Hz, 1 H), 8.49 (s, 1 H), 8.47 (s, 1 H), 8.44 (dt, *J*=8.1, 1.9 Hz, 1 H), 7.91 (d, *J*=2.1 Hz, 1 H), 7.89 (d, *J*=8.5 Hz, 1 H), 7.68 (dd, *J*=8.4, 2.1 Hz, 1 H), 7.64 (d, *J*=8.0 Hz, 1 H), 7.50 (ddd, *J*=8.0, 4.8, 0.8 Hz, 1 H), 4.56 (spt, *J*=7.0 Hz, 1 H), 2.92 (br s, 4 H), 2.51 - 2.62 (m, 5 H), 2.37 (dd, *J*=12.4, 6.9 Hz, 1 H), 1.17 (d, *J*=6.6 Hz, 3 H) |

### Example 3

### 2-cyclopropyl-7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine

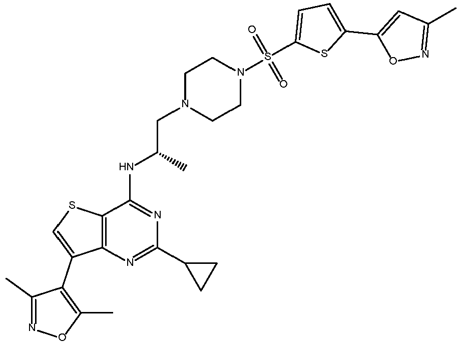

### Step 1: Preparation of 7-bromo-2-cyclopropyl-3H-thieno[3,2-d]pyrimidin-4-one (Intermediate 10)

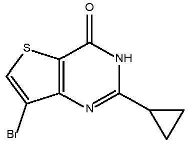

To a solution of methyl 3-amino-4-bromothiophene-2-carboxylate (1.18 g, 5 mmol) in cyclopropanecarbonitrile (11 mL, 165.39 mmol) 2N HCl (2.5 mL, 5 mmol) was added. The mixture was heated at 130 °C under MW irradiation for 2 cycles of 1 h. NaHCO₃ sat. sol. was added and the mixture was extracted 3 times with AcOEt; the combined organic layers were dried over Na₂SO₄, filtered and evaporated under vacuum. The crude was purified by flash chromatography eluting with cyclohexane/AcOEt from 9/1 to 1/1 to afford title compound (163 mg, 0.6 mmol, 12% yield) as a pale yellow solid.
LC-MS (ESI): *m*/*z* (M+1): 273 (Method 1)
¹H NMR (400 MHz, *DMSO-d₆*) δ ppm 12.83 (br. s., 1 H), 8.30 (s, 1 H), 1.97 - 2.08 (m, 1H), 1.02 - 1.14 (m, 4 H)

### Step 2: Preparation of 7-bromo-4-chloro-2-cyclopropylthieno[3,2-d]pyrimidine (Intermediate 11)

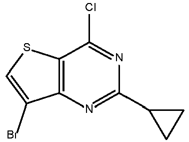

A solution of Intermediate 10 (163 mg, 0.6 mmol) in POCl₃ (10 mL) was stirred at 100 °C overnight. POCl₃ was evaporated, then H₂O (5 mL) and NaHCO₃ (5 mL) were added and the mixture was. The combined organic layers were dried over Na₂SO₄, filtered and evaporated to provide title compound (150 mg, 0.52 mmol, 73% yield) that was used in the next step without further purification.
LC-MS (ESI): *m*/*z* (M+1): 290.9 (Method 1)
¹H NMR (400 MHz, *DMSO*-d₆) δ ppm 8.71 (s, 1 H), 2.31 - 2.42 (m, 1 H), 1.06 - 1.21 (m, 4 H)

### Step 3: Preparation of tert-butyl 4-[(2S)-2-[(7-bromo-2-cyclopropylthieno[3,2-d]pyrimidin-4-yl)amino]propyl]piperazine-1-carboxylate (Intermediate 12)

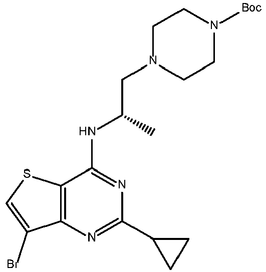

Title compound was prepared following the procedure used for the synthesis of Intermediate 3, starting from 7-bromo-4-chloro-2-cyclopropylthieno[3,2-d]pyrimidine (Intermediate 11, 150 mg, 0.52 mmol) to provide title compound (248 mg, 0.5 mmol, 96.5% yield) as a white solid.
LC-MS (ESI): *m*/*z* (M+1): 498.1 (Method 1)
¹H NMR (400 MHz, *DMSO-d6)* δ ppm 8.21 (s, 1 H), 7.62 (d, *J*=7.92 Hz, 1 H), 4.40 - 4.59 (m, 1 H), 4.04 (q, *J*=7.12 Hz, 1 H), 3.19 - 3.29 (m, 4 H), 2.35 - 2.46 (m, 4 H), 2.30 (dd, *J*=12.21, 7.37 Hz, 1 H), 2.02 - 2.10 (m, 1 H), 1.39 (s, 9 H), 1.19 - 1.27 (m, 3 H), 0.88 - 1.08 (m, 4H)

### Step 4: Preparation of tert-butyl 4-[(2S)-2-[[2-cyclopropyl-7-(3,5-dimethyl-1,2-oxazol-4-yl)thieno[3,2-d]pyrimidin-4-yl]amino]propyl]piperazine-1-carboxylate (Intermediate 13)

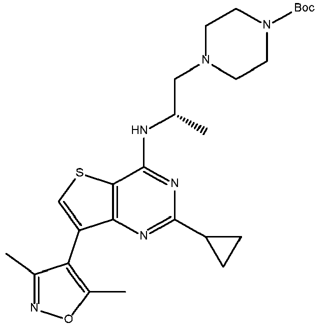

To a solution of Intermediate 12 (248 mg, 0.5 mmol) in THF (5 mL) and water (2 mL), 3,5-dimethyl-4-(4,4,5,5-tetramethyl-1,3,2-dioxaborolan-2-yl)isoxazole (446 mg, 2 mmol), Pd(dppf)Cl₂ (36.67 mg, 0.05 mmol) and K₃PO₄ (215 mg, 1 mmol) were added. The mixture was stirred at 80 °C overnight. Brine was added and the mixture was extracted 3 times with AcOEt. The combined organic layers were dried over Na₂SO₄, filtered and evaporated to dryness to afford a crude product that was purified by flash column chromatography eluting with cyclohexane/AcOEt from 6/4 to 2/8 to afford title compound (430 mg, 0.84 mmol, 84% yield) as a yellow oil.
LC-MS (ESI): *m*/*z* (M+1): 513.2 (Method 1)
¹H NMR (400 MHz, *DMSO-d₆*) δ ppm 8.06 (s, 1 H), 7.92 (s, 1 H), 7.50 (d, *J*=7.70 Hz, 1 H), 4.45 - 4.62 (m, 1 H), 3.92 (s, 6 H), 3.27 (t, *J*=4.62 Hz, 3 H), 2.36 - 2.45 (m, 6 H), 2.32 (dd, *J*=12.21, 7.37 Hz, 1 H), 2.22 (s, 3 H), 1.95 - 2.03 (m, 2 H), 1.39 (s, 8 H), 1.21 - 1.27 (m, 3 H), 1.14 - 1.21 (m, 3 H), 1.08 (s, 36 H), 0.86 - 0.97 (m, 4 H)

### Step 5: Preparation of 2-cyclopropyl-7-(3,5-dimethyl-1,2-oxazol-4-yl)-N-[(2S)-1-piperazin-1-ylpropan-2-yl]thieno[3,2-d]pyrimidin-4-amine hydrochloride (Intermediate 14)

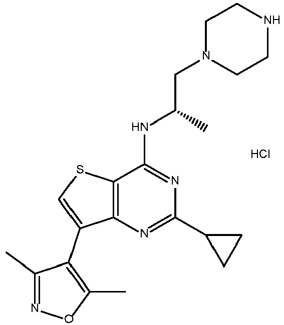

Title compound was prepared following the procedure used for the synthesis of Intermediate 9, starting from tert-butyl 4-[(2S)-2-[[2-cyclopropyl-7-(3,5-dimethyl-1,2-oxazol-4-yl)thieno[3,2-d]pyrimidin-4-yl]amino]propyl]piperazine-1-carboxylate (Intermediate 13, 430 mg, 0.84 mmol) to afford title compound (394 mg, 0.81 mmol, 97% yield) as a pale yellow solid.
LC-MS (ESI): *m*/*z* (M+1): 413.1 (Method 2)
¹H NMR (400 MHz, *DMSO-d₆*) δ ppm 9.47- 9.78 (m, 1 H), 8.20 - 8.46 (m, 1 H), 4.78 - 4.99 (m, 1 H), 3.67 - 4.03 (m, 11 H), 3.31 - 3.52 (m, 4 H), 2.35 (s, 3 H), 2.16 (s, 2 H), 1.10- 1.35 (m, 4 H), 1.08 (s, 10 H)

### Step 6: Preparation of 2-cyclopropyl-7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine (Example 3)

Title compound was prepared following the procedure used for the synthesis of Example 1, starting from 2-cyclopropyl-7-(3,5-dimethyl-1,2-oxazol-4-yl)-N-[(2S)-1-piperazin-1-ylpropan-2-yl]thieno[3,2-d]pyrimidin-4-amine hydrochloride (Intermediate 14, 394 mg, 0.81 mmol) and 5-(3-methyl-1,2-oxazol-5-yl)thiophene-2-sulfonyl chloride (235 mg, 0.89 mmol) to afford title compound (77 mg, 0.12 mmol, 15% yield) as a white solid.
LC-MS (ESI): *m*/*z* (M+1): 640 (Method 2)
¹H NMR (400 MHz, *DMSO-d₆*) δ ppm 8.02 (s, 1 H), 7.77 (d, *J*=4.0 Hz, 1 H), 7.70 (d, *J*=4.0 Hz, 1 H), 7.46 (d, *J*=7.8 Hz, 1 H), 6.97 (s, 1 H), 4.38 - 4.53 (m, 1 H), 2.98 (br s, 4 H), 2.56 - 2.63 (m, 4 H), 2.48 - 2.63 (m, 1 H), 2.35 (s, 3 H), 2.29 (s, 3 H), 2.26 - 2.36 (m, 1 H), 2.18 (s, 3 H), 1.93 - 2.02 (m, 1 H), 1.17 (d, *J*=6.6 Hz, 3 H), 0.83 - 0.92 (m, 4 H)

The Examples in the following table were prepared from commercially available reagents by using methods analogous to Example 3.

| **Example No.** | **Structure & Name** | **Analytical data** |
|---|---|---|
| 46 | 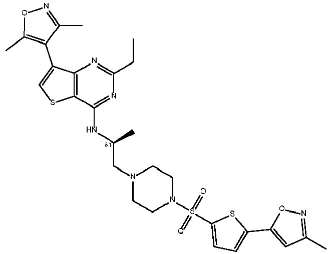 7-(3,5-dimethyl-1,2-oxazol-4-yl)-2-ethyl-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine | LC-MS (ESI): m/z (M+1): 628.1 (Method 2) |
| | | ¹H NMR (400 MHz, *DMSO-d₆*) δ ppm 8.04 (s, 1 H), 7.75 (d, *J*=4.0 Hz, 1 H), 7.69 (d, *J*=4.2 Hz, 1 H), 7.48 (d, *J*=7.9 Hz, 1 H), 6.96 (s, 1 H), 4.54 (dt, *J*=14.3, 6.9 Hz, 1 H), 2.97 (br s, 4 H), 2.67 (q, *J*=7.6 Hz, 2 H), 2.53 - 2.60 (m, 5 H), 2.36 (s, 3 H), 2.31 - 2.34 (m, 1 H), 2.29 (s, 3 H), 2.19 (s, 2 H), 1.20 (t, *J*=6.7 Hz, 4 H), 1.18 (d, *J*=4.8 Hz, 3 H) |
| 48 | 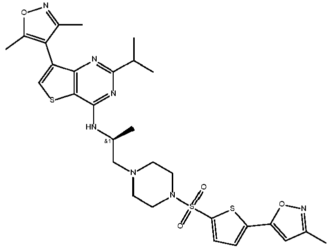 7-(3,5-dimethyl-1,2-oxazol-4-yl)-N-[(2S)-1-(4-{[5-(3-methyl-1,2-oxazol-5-yl)thiophen-2-yl]sulfonyl (piperazin-1 - yl)propan-2-yl]-2-(propan-2-yl)thieno[3,2-d]pyrimidin-4-amine | LC-MS (ESI): m/z (M+1): 642.16 (Method 2) |
| | | ¹H NMR (400 MHz, *DMSO-d₆*) δ ppm 8.05 (s, 1 H), 7.76 (d, *J*=4.0 Hz, 1 H), 7.69 (d, *J*=4.0 Hz, 1 H), 7.49 (d, *J*=7.9 Hz, 1 H), 6.96 (s, 1 H), 4.54 (spt, *J*=6.9 Hz, 1 H), 2.95 - 3.03 (m, 4 H), 2.85 - 2.94 (m, 1 H), 2.54 - 2.65 (m, 5 H), 2.37 (s, 3 H), 2.33 (dd, *J*=12.4, 7.2 Hz, 1 H), 2.29 (s, 3 H), 2.20 (s, 3 H), 1.17 - 1.25 (m, 9 H) |

### Example 4

### Methyl 5-({4-[(2S)-2-({7-methylthieno[3,2-d]pyrimidin-4-yl}amino)propyl]piperazin-1-yl}sulfonyl)thiophene-2-carboxylate

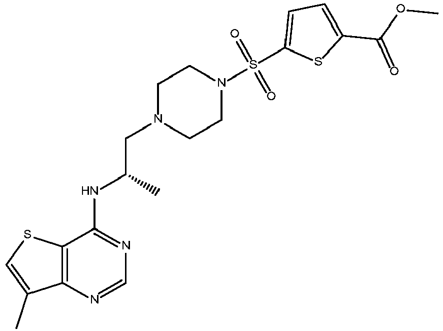

### Step 1: Preparation of tert-butyl 4-[(2S)-2-({7-methylthieno[3,2-d]pyrimidin-4-yl}amino)propyl]piperazine-1-carboxylate (Intermediate 15)

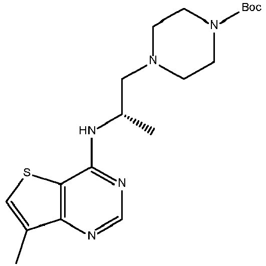

Title compound was prepared following the procedure used for the synthesis of Intermediate 3, starting from commercially available 4-chloro-7-methylthieno[3,2-d]pyrimidine (1.0 g, 5.42 mmol) to provide title compound (1.61 g, 4.11 mmol, 76% yield) as a whitenish solid.
LC-MS (ESI): *m*/*z* (M+1): 392.3 (Method 2)

### Step 2: Preparation of 7-methyl-N-[(2S)-1-piperazin-1-ylpropan-2-yl]thieno[3,2-d]pyrimidin-4-amine (Intermediate 16)

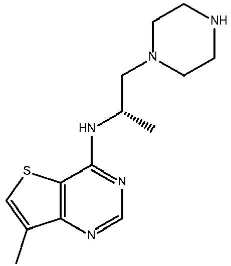

Title compound was prepared following the procedure used for the synthesis of Intermediate 4, starting from tert-butyl 4-[(2S)-2-({7-methylthieno[3,2-d]pyrimidin-4-yl}amino)propyl]piperazine-1-carboxylate (Intermediate 15, 1.61 g, 4.11 mmol) to afford title compound (1.12 g, 3.84 mmol, 98.% yield) as a yellowish solid.
LC-MS (ESI): *m*/*z* (M+1): 292.2 (Method 2)

### Step 3: Preparation of Methyl 5-({4-[(2S)-2-({7-methylthieno[3,2-d]pyrimidin-4-yl}amino)propyl]piperazin-1-yl}sulfonyl)thiophene-2-carboxylate

### (Example 4)

To a solution of (7-methyl-N-[(2S)-1-piperazin-1-ylpropan-2-yl]thieno[3,2-d]pyrimidin-4-amine trihydrochloride (Intermediate 16, 50 mg, 0.125 mmol) in dry pyridine (0.7 ml) methyl 5-(chlorosulfonyl)thiophene-2-carboxylate (30 mg, 0.125 mmol) was added and the solution was sonicated for 5 min. After evaporation of pyridine, the residue was treated with heptane (35 ml) and the solvent was evaporated under vacuum. The crude was partitioned between a saturated solution of NaHCO₃ (10 ml) and EtOAc (30 ml), the organic layer was dried over Na₂SO₄ and evaporated to dryness to obtain the title compound as a yellow solid
LC-MS (ESI): m/z (M+1): 496.18 (Method 3 )
¹H NMR (400 MHz, *Acetone-d₆*) δ ppm 8.47 (s, 1 H) 7.83 (d, *J*=3.91 Hz, 1 H) 7.57 (d, *J*=4.03 Hz, 1 H) 7.52 (d, *J*=1.22 Hz, 1 H) 6.42 (br d, *J*=7.09 Hz, 1 H) 4.56 - 4.67 (m, 1 H) 3.92 (s, 3 H) 3.03 (br s, 4 H) 2.59 - 2.73 (m, 5 H) 2.47 (dd, *J*=12.53, 6.42 Hz, 1 H) 2.35 (d, *J*=1.10 Hz, 3 H) 1.27 (d, *J*=6.48 Hz, 3 H)

The Examples in the following table were prepared from commercially available reagents by using methods analogous to Example 4.

| **Example No.** | **Structure & Name** | **Analytical data** |
|---|---|---|
| 5 | 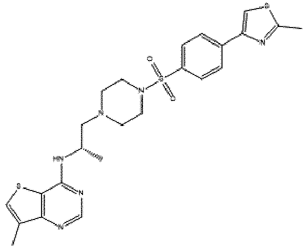 | LC-MS (ESI): m/z (M+1): 529.16 (Method 3) |
| | 7-methyl-N-[(2S)-1-{4-[4-(2-methyl-1,3-thiazol-4-yl)benzenesulfonyl]piperazin-1-yl}propan-2-yl]thieno[3,2-d]pyrimidin-4-amine | ¹H NMR (400 MHz, *Acetone-d₆*) δ ppm 8.46 (s, 1 H) 8.18 - 8.23 (m, 2 H) 7.99 (s, 1 H) 7.75 - 7.81 (m, 2 H) 7.45 (d, *J*=1.10 Hz, 1 H) 6.40 (br d, *J*=6.97 Hz, 1 H) 4.51 - 4.61 (m, 1 H) 2.97 (s, 4 H) 2.77 (s, 3 H) 2.55 - 2.67 (m, 5 H) 2.45 (dd, *J*=12.47, 6.36 Hz, 1 H) 2.31 (d, *J*=1.10 Hz, 3 H) 1.24 (d, *J*=6.48 Hz, 3 H) |
| 6 | 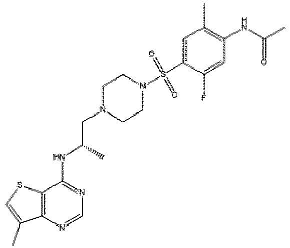 | LC-MS (ESI): m/z (M+1): 521.16 (Method 3) |
| | N-[5-fluoro-2-methyl-4-({4-[(2S)-2-({7-methylthieno[3,2-d]pyrimidin-4-yl}amino)propyl]piperazin-1-yl}sulfonyl)phenyl]acetamide | ¹H NMR (400 MHz, *DMSO-d₆*) δ ppm 9.45 (s, 1 H) 8.41 (s, 1 H) 7.88 (d, *J*=12.93 Hz, 1 H) 7.68 (d, *J*=1.10 Hz, 1 H) 7.52 (d, *J*=7.89 Hz, 1 H) 7.39 (d, *J*=7.89 Hz, 1 H) 4.46 - 4.56 (m, 1 H) 2.95 (br s, 4 H) 2.33 - 2.39 (m, 1 H) 2.30 (d, *J*=0.88 Hz, 3 H) 2.28 (s, 3 H) 2.15 (s, 3 H) 1.15 (d, *J*=6.36 Hz, 3 H) |
| 7 | 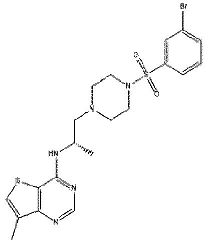 | LC-MS (ESI): m/z (M+1): 512.00 (Method 3) |
| | N-[(2S)-1-[4-(3-bromobenzenesulfonyl)piperazin-1-yl]propan-2-yl]-7-methylthieno[3,2-d]pyrimidin-4-amine | ¹H NMR (400 MHz, *DMSO-d₆*) δ ppm 8.74 (br s, 1 H) 8.03 (br s, 1 H) 7.94 - 8.00 (m, 1 H) 7.89 (br s, 1 H) 7.77 (d, *J*=8.33 Hz, 1 H) 7.63 (t, *J*=8.00 Hz, 1 H) 4.84 - 5.02 (m, 1 H) 3.71 -4.32 (m, 8 H) 2.40 (s, 2 H) 1.29 (br d, *J*=6.58 Hz, 3 H) |
| 8 | 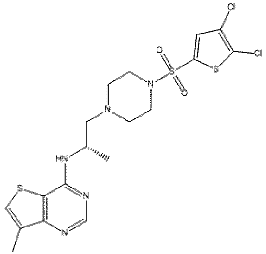 | LC-MS (ESI): m/z (M+1): 506.05 (Method 3 ) |
| | N-[(2S)-1-{4-[(4,5-dichlorothiophen-2-yl)sulfonyl]piperazin-1-yl}propan-2-yl]-7-methylthieno[3,2-d]pyrimidin-4-amine | ¹H NMR (400 MHz *Acetone-d₆*) δ ppm 8.46 (s, 1 H) 7.51 - 7.55 (m, 2 H) 6.42 (br d, *J*=7.23 Hz, 1 H) 4.61 (spt, *J*=6.87 Hz, 1 H) 3.05 (br s, 4 H) 2.59 - 2.70 (m, 5 H) 2.47 (dd, *J*=12.50, 6.36 Hz, 1 H) 2.35 (s, 3 H) 1.26 (d, *J*=6.58 Hz, 3 H). |
| 9 | 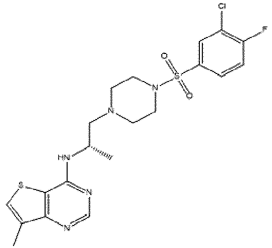 | LC-MS (ESI): m/z (M+1): 484.12 (Method 5 ) |
| | N-[(2S)-1-[4-(3-chloro-4-fluorobenzenesulfonyl)piperazin-1-yl]propan-2-yl]-7-methylthieno[3,2-d]pyrimidin-4-amine | ¹H NMR (400 MHz, *Acetone-d₆*) δ ppm 8.45 (s, 1 H) 7.86 (dd, *J*=6.85, 2.20 Hz, 1 H) 7.76 (ddd, *J*=8.59, 4.49, 2.32 Hz, 1 H) 7.55 (t, *J*=8.80 Hz, 1 H) 7.52 (d, *J*=0.98 Hz, 1 H) 6.41 (br s, 1 H) 4.53 - 4.66 (m, 1 H) 2.98 (br s, 4 H) 2.78 (br s, 1 H) 2.62 (br s, 4 H) 2.39 - 2.51 (m, 1 H) 2.34 (d, *J*=0.98 Hz, 3 H) 1.24 (d, *J*=6.60 Hz, 3 H) |
| 10 | 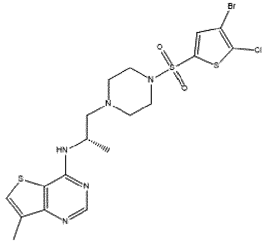 | LC-MS (ESI): m/z (M+1): 551.24 (Method 5 ) |
| | N-[(2S)-1-{4-[(4-bromo-5-chlorothiophen-2-yl)sulfonyl]piperazin-1-yl}propan-2-yl]-7-methylthieno[3,2-d]pyrimidin-4-amine | ¹H NMR (400 MHz, *Acetone-d₆*) δ ppm 8.46 (s, 1 H) 7.54 (s, 1 H) 7.53 (d, *J*=1.22 Hz, 1 H) 6.43 (br d, *J*=6.97 Hz, 1 H) 4.56 - 4.66 (m, 1 H) 3.01 - 3.10 (m, 4 H) 2.60 - 2.71 (m, 5 H) 2.47 (dd, *J*=12.53, 6.42 Hz, 1 H) 2.35 (d, *J*=1.10 Hz, 3 H) 1.26 (d, *J*=6.48 Hz, 3 H) |
| 11 | 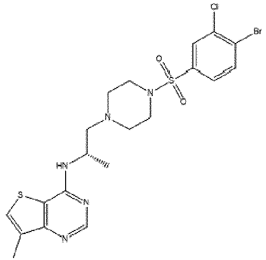 | LC-MS (ESI): m/z (M+1): 545.44 (Method 5 ) |
| | N-[(2S)-1-[4-(4-bromo-3-chlorobenzenesulfonyl)piperazin-1-yl]propan-2-yl]-7-methylthieno[3,2-d]pyrimidin-4-amine | ¹H NMR (400 MHz, *Acetone-d6)* δ ppm 8.45 (s, 1 H) 7.98 (d, *J*=8.31 Hz, 1 H) 7.85 (d, *J*=2.08 Hz, 1 H) 7.59 (dd, *J*=8.38, 2.14 Hz, 1 H) 7.52 (d, *J*=1.10 Hz, 1 H) 6.40 (br d, *J*=7.46 Hz, 1 H) 4.54 - 4.63 (m, 1 H) 2.99 (br s, 4 H) 2.55 - 2.66 (m, 5 H) 2.44 (dd, *J*=12.47, 6.36 Hz, 1 H) 2.34 (d, *J*=1.10 Hz, 3 H) 1.24 (d, *J*=6.60 Hz, 3 H) |
| 12 | 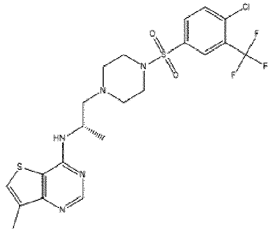 | LC-MS (ESI): m/z (M+1): 533.6 (Method 5 ) |
| | N-[(2S)-1-{4-[4-chloro-3-(trifluoromethyl)benzenesulfonyl]piperaz in-1-yl}propan-2-yl]-7-methylthieno[3,2-d]pyrimidin-4-amine | ¹H NMR (400 MHz, *Acetone-d₆*) δ ppm 8.44 (s, 1 H) 8.04 - 8.07 (m, 1 H) 7.99 - 8.04 (m, 1 H) 7.91 - 7.96 (m, 1 H) 7.51 (d, *J*=0.86 Hz, 1 H) 6.40 (br d, *J*=7.21 Hz, 1 H) 4.58 (spt, *J*=6.91 Hz, 1 H) 2.96 - 3.08 (m, 4 H) 2.56 - 2.67 (m, 5 H) 2.43 (dd, *J*=12.53, 6.42 Hz, 1 H) 2.34 (d, *J*=0.86 Hz, 3 H) 1.23 (d, *J*=6.48 Hz, 3 H) |
| 13 | 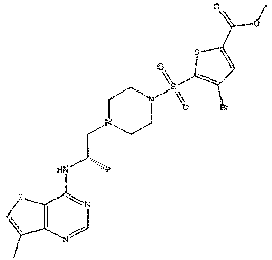 | LC-MS (ESI): m/z (M+1): 575.4 (Method 5 ) |
| | methyl 4-bromo-5-({4-[(2S)-2-({7-methylthieno[3,2-d]pyrimidin-4-yl}amino)propyl]piperazin-1-yl} sulfonyl)thiophene-2-carboxylate | ¹H NMR (400 MHz, *Acetone-d₆*) δ ppm 8.46 (s, 1 H) 7.77 (s, 1 H) 7.52 (d, *J*=0.88 Hz, 1 H) 6.42 (br d, *J*=6.80 Hz, 1 H) 4.56 - 4.67 (m, 1 H) 3.91 (s, 3 H) 3.24 (br s, 4 H) 2.57 - 2.69 (m, 5 H) 2.47 (br dd, *J*=12.39, 6.25 Hz, 1 H) 2.34 (d, *J*=1.10 Hz, 3 H) 1.27 (d, *J*=6.36 Hz, 3 H) |
| 14 | 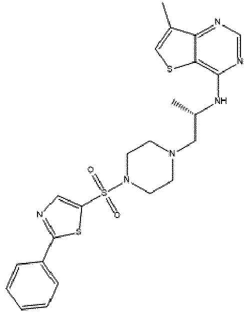 | LC-MS (ESI): m/z (M+1): 515.2 (Method 3 ) |
| | 7-methyl-N-[(2S)-1-{4-[(2-phenyl-1,3-thiazol-5-yl)sulfonyl]piperazin-1-yl}propan-2-yl]thieno[3,2-d]pyrimidin-4-amine | ¹H NMR (400 MHz, *Acetone-d₆*) δ ppm 8.45 (s, 1 H) 8.19 (s, 1 H) 8.04 (dd, *J*=7.89, 1.64 Hz, 2 H) 7.52 - 7.61 (m, 3 H) 7.40 (d, *J*=1.15 Hz, 1 H) 6.40 (br d, *J*=7.07 Hz, 1 H) 4.54 - 4.65 (m, 1 H) 3.08 (br s, 4 H) 2.60 - 2.72 (m, 4 H) 2.47 (dd, *J*=12.50, 6.41 Hz, 1 H) 2.28 (d, *J*=0.99 Hz, 3 H) 1.25 (d, *J*=6.58 Hz, 3 H) |
| 15 | 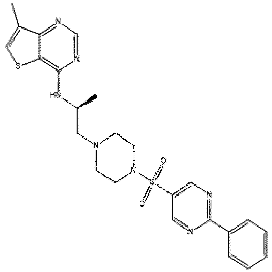 | LC-MS (ESI): m/z (M+1): 510.27 (Method 3) |
| | 7-methyl-N-[(2S)-1-{4-[(2-phenylpyrimidin-5-yl)sulfonyl]piperazin-1-yl}propan-2-yl]thieno[3,2-d]pyrimidin-4-amine | ¹H NMR (400 MHz, *Acetone-d₆*) δ ppm 9.10 (s, 2 H) 8.53 (d, *J*=7.20 Hz, 2 H) 8.44 (s, 1 H) 7.54 - 7.63 (m, 3 H) 7.41 (d, *J*=0.98 Hz, 1 H) 6.39 (br d, *J*=6.60 Hz, 1 H) 4.54 - 4.64 (m, 1 H) 3.13 (br s, 4 H) 2.59-2.71 (m, 5 H) 2.45 (dd, *J*=12.53, 6.30 Hz, 1 H) 2.28 (d, *J*=0.98 Hz, 3 H) 1.23 (d, *J*=6.48 Hz, 3 H) |
| 16 | 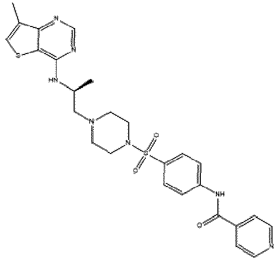 | LC-MS (ESI): m/z (M+1): 552.20 (Method 3 ) |
| | N-[4-({4-[(2S)-2-({7-methylthieno[3,2-d]pyrimidin-4-yl}amino)propyl]piperazin-1-yl} sulfonyl)phenyl]pyridine-4-carboxamide | ¹H NMR (400 MHz, *Acetone-d₆*) δ ppm 9.27 (br s, 1 H) 8.90 (d, *J*=5.11 Hz, 2 H) 8.15 (s, 1 H) 8.06 (d, *J*=8.70 Hz, 2 H) 7.91 (d, *J*=5.09 Hz, 2 H) 7.73 - 7.87 (m, 2 H) 7.53 (d, *J*=1.22 Hz, 1 H) 5.83 (br d, *J*=7.17 Hz, 1 H) 4.50 - 4.62 (m, 1 H) 2.93 - 3.03 (m, 4 H) 2.61 - 2.73 (m, 5 H) 2.52 (br dd, *J*=12.59, 5.87 Hz, 1 H) 2.43 (d, *J*=1.07 Hz, 3 H) 1.32 (d, *J*=6.56 Hz, 3 H) |
| 17 | 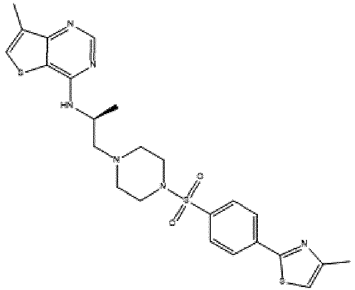 | LC-MS (ESI): m/z (M+1): 529.18 (Method 3 ) |
| | 7-methyl-N-[(2S)-1-{4-[4-(4-methyl-1,3-thiazol-2-yl)benzenesulfonyl]piperazin-1- yl}propan-2-yl]thieno[3,2-d]pyrimidin-4-amine | ¹H NMR (400 MHz, *Acetone-d₆*) δ ppm 8.54 (s, 1 H) 8.20 (d, *J*=8.33 Hz, 2 H) 7.85 (d, *J*=8.33 Hz, 2 H) 7.45 (d, *J*=0.88 Hz, 1 H) 7.31 (s, 1 H) 5.80 (br d, *J*=6.80 Hz, 1 H) 4.49 - 4.61 (m, 1 H) 2.92 - 3.06 (m, 4 H) 2.60 - 2.74 (m, 5 H) 2.60 (s, 3 H) 2.47 - 2.55 (m, 1 H) 2.39 (s, 3 H) 1.31 (d, *J*=6.36 Hz, 3 H) |
| 18 | 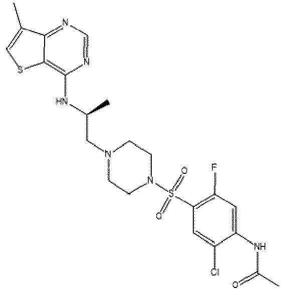 | LC-MS (ESI): m/z (M+1): 541.13 (Method 3 ) |
| | N-[2-chloro-5-fluoro-4-({4-[(2S)-2-({7-methylthieno[3,2-d]pyrimidin-4-yl}amino)propyl]piperazin-1-yl}sulfonyl)phenyl]acetamide | ¹H NMR (400 MHz, *Acetone-d₆*) δ ppm 8.98 (br s, 1 H) 8.47 (s, 1 H) 8.41 - 8.46 (m, 1 H) 7.76 (d, *J*=6.87 Hz, 1 H) 7.53 (d, *J*=1.07 Hz, 1 H) 6.42 (br d, *J*=7.17 Hz, 1 H) 4.56 - 4.66 (m, 1 H) 3.12 (br s, 4 H) 2.57-2.68 (m, 5 H) 2.47 (dd, *J*=12.44, 6.33 Hz, 1 H) 2.35 (d, *J*=1.07 Hz, 3 H) 2.29 (s, 3 H) 1.28 (d, *J*=6.56 Hz, 3 H) |
| 19 | 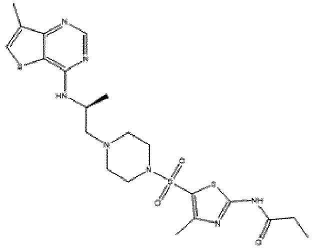 | LC-MS (ESI): m/z (M+1): 524.19 (Method 3 ) |
| | N-[4-methyl-5-({4-[(2S)-2-({7-methylthieno[3,2-d]pyrimidin-4-yl}amino)propyl]piperazin-1-yl}sulfonyl)-1,3-thiazol-2-yl]propanamide | 1H NMR (400 MHz, *Acetone-d₆*) δ ppm 11.20 (br s, 1 H) 8.47 (s, 1 H) 7.52 (s, 1 H) 6.43 (br d, *J*=7.23 Hz, 1 H) 4.62 (spt, *J*=6.87 Hz, 1 H) 3.06 (br s, 4 H) 2.58 - 2.70 (m, 7 H) 2.49 (br d, *J*=6.58 Hz, 2 H) 2.45 (s, 3 H) 2.35 (s, 3 H) 1.28 (d, *J*=6.58 Hz, 3 H) 1.19 (t, *J*=7.45 Hz, 3 H). |
| 20 | 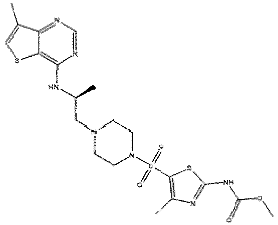 | LC-MS (ESI): m/z (M+1): 526.15 (Method 3 ) |
| | methyl N-[4-methyl-5-({4-[(2S)-2-({7-methylthieno[3,2-d]pyrimidin-4-yl}amino)propyl]piperazin-1-yl}sulfonyl)-1,3-thiazol-2-yl]carbamate | 1H NMR (400 MHz, *Acetone-d₆*) δ ppm 10.90 (br s, 1 H) 8.45 (s, 1 H) 7.49 (s, 1 H) 6.42 (br s, 1 H) 4.66 (dquin, *J*=13.54, 6.86, 6.86, 6.86, 6.86 Hz, 1 H) 3.84 (s, 3 H) 3.07 (br s, 4 H) 2.59 - 2.73 (m, 5 H) 2.44 - 2.53 (m, 1 H) 2.40 (s, 3 H) 2.33 (s, 3 H) 1.27 (d, *J*=6.36 Hz, 3 H) |
| 21 | 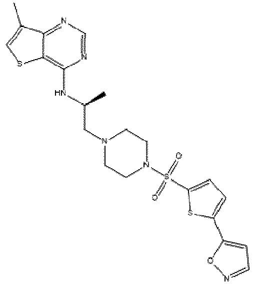 | LC-MS (ESI): m/z (M+1): 504.71 (Method 3 ) |
| | 7-methyl-N-[(2S)-1-(4-{[5-(1,2-oxazol-5-yl)thiophen-2-yl]sulfonyl}piperazin-1- yl)propan-2-yl]thieno[3,2-d]pyrimidin-4-amine | ¹H NMR (400 MHz, *Acetone-d₆*) δ ppm 8.54 (d, *J*=1.97 Hz, 1 H) 8.45 (s, 1 H) 7.73 (d, J=3.95 Hz, 1 H) 7.61 (d, *J*=4.17 Hz, 1 H) 7.46 (d, *J*=1.10 Hz, 1 H) 6.94 (d, *J*=1.97 Hz, 1 H) 6.42 (br d, *J*=7.02 Hz, 1 H) 4.55 - 4.65 (m, 1 H) 3.05 (br s, 4 H) 2.61 - 2.73 (m, 5 H) 2.48 (dd, *J*=12.61, 6.25 Hz, 1 H) 2.31 (d, *J*= 1.10 Hz, 3 H) 1.25 (d, *J*=6.58 Hz, 3 H). |
| 23 | 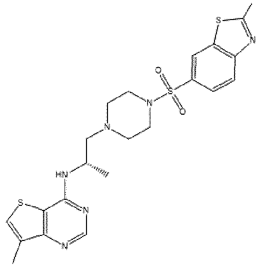 | LC-MS (ESI): m/z (M+1): 503.18 (Method 3 ) |
| | 7-methyl-N-[(2S)-1-{4-[(2-methyl-1,3-benzothiazol-6-yl)sulfonyl]piperazin-1- yl}propan-2-yl]thieno[3,2-d]pyrimidin-4-amine | ¹H NMR (400 MHz, *Acetone-d₆*) δ ppm 8.44 (s, 1 H) 8.43 (d, *J*=1.82 Hz, 1 H) 8.07 (d, *J*=8.56 Hz, 1 H) 7.81 (dd, *J*=8.56, 1.82 Hz, 1 H) 7.45 (d, *J*=1.12 Hz, 1 H) 6.36 (br d, *J*=7.15 Hz, 1 H) 4.51 - 4.61 (m, 1 H) 2.98 (br s, 4 H) 2.55 - 2.66 (m, 5 H) 2.43 (dd, *J*=12.48, 6.45 Hz, 1 H) 2.32 (d, *J*=0.98 Hz, 3 H) 1.23 (d, *J*=6.59 Hz, 3 H) |
| 24 | 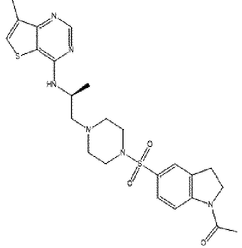 | LC-MS (ESI): m/z (M+1): 514.5 (Method 3 ) |
| | 1-[5-({4-[(2S)-2-({7-methylthieno[3,2-d]pyrimidin-4-yl}amino)propyl]piperazin-1-yl}sulfonyl)-2,3-dihydro-1H-indol-1-yl]ethan-1-one | ¹H NMR (400 MHz, *Acetone-d₆*) δ ppm 8.46 (s, 1 H) 7.52 (br d, *J*=8.11 Hz, 3 H) 6.40 (br d, *J*=7.23 Hz, 1 H) 4.54 - 4.65 (m, 1 H) 4.25 (br t, *J*=8.66 Hz, 2 H) 3.28 (br t, *J*=8.55 Hz, 2 H) 2.90 (br s, 4 H) 2.53 - 2.63 (m, 5 H) 2.43 (dd, *J*=12.50, 6.36 Hz, 1 H) 2.34 (d, *J*=0.88 Hz, 3 H) 2.22 (s, 3 H) 1.25 (d, *J*=6.58 Hz, 3 H). (Missing NH protone) |
| 25 | 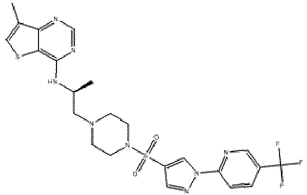 | LC-MS (ESI): m/z (M+1): 567.19 (Method 3 ) |
| | 7-methyl-N-[(2S)-1-[4-({1-[5-(trifluoromethyl)pyridin-2-yl]-1H-pyrazol-4-yl}sulfonyl)piperazin-1- yl]propan-2-yl]thieno[3,2-d]pyrimidin-4-amine | ¹H NMR (400 MHz, *Acetone-d₆*) δ ppm 8.97 (s, 1 H) 8.87 - 8.91 (m, 1 H) 8.45 (s, 1 H) 8.41 - 8.45 (m, 1 H) 8.25 (d, *J*=8.68 Hz, 1 H) 8.08 (s, 1 H) 7.45 (d, *J*=1.10 Hz, 1 H) 6.40 (br d, *J*=7.34 Hz, 1 H) 4.56 - 4.65 (m, 1 H) 2.97 - 3.08 (m, 4 H) 2.60 - 2.72 (m, 5 H) 2.47 (dd, *J*=12.47, 6.36 Hz, 1 H) 2.31 (d, *J*=0.98 Hz, 3 H) 1.26 (d, *J*=6.48 Hz, 3 H) |
| 26 | 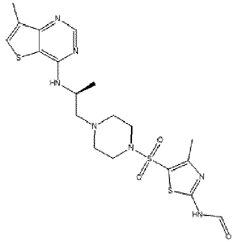 | LC-MS (ESI): m/z (M+1): 496.18 (Method 3 ) |
| | N-[4-methyl-5-({4-[(2S)-2-({7-methylthieno[3,2-d]pyrimidin-4-yl}amino)propyl]piperazin-1- yl}sulfonyl)-1,3-thiazol-2-yl]formamide | ¹H NMR (400 MHz, *Acetone-d₆*) δ ppm 8.70 (s, 1 H) 8.47 (s, 1 H) 7.51 (s, 1 H) 6.42 (br d, *J*=7.45 Hz, 1 H) 4.59 - 4.70 (m, 1 H) 3.08 (br t, *J*=4.71 Hz, 4 H) 2.59 - 2.73 (m, 5 H) 2.49 (d, *J*=6.37 Hz, 1 H) 2.47 (s, 3 H) 2.35 (s, 3 H) 1.28 (d, *J*=6.58 Hz, 3 H) |
| 27 | 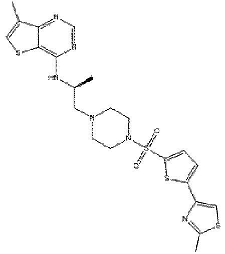 | LC-MS (ESI): m/z (M+1): 535.13 (Method 3 ) |
| | 7-methyl-N-[(2S)-1-(4-{[5-(2-methyl-1,3-thiazol-4-yl)thiophen-2-yl]sulfonyl}piperazin-1-yl)propan-2-yl]thieno[3,2-d]pyrimidin-4-amine | ¹H NMR (400 MHz, *Acetone-d₆*) δ ppm 8.47 (s, 1 H) 7.87 (s, 1 H) 7.61 (d, *J*=3.95 Hz, 1 H) 7.50 (d, *J*=3.95 Hz, 1 H) 7.45 (s, 1 H) 6.42 (br d, *J*=5.70 Hz, 1 H) 4.60 (dt, *J*=13.59, 6.80 Hz, 1 H) 3.04 (br s, 4 H) 2.73 (s, 3 H) 2.61 - 2.70 (m, 5 H) 2.48 (br dd, *J*=12.28, 6.58 Hz, 1 H) 2.32 (s, 3 H) 1.27 (d, *J*=6.58 Hz, 3 H) |
| 28 | 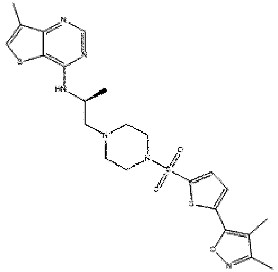 | LC-MS (ESI): m/z (M+1): 533.20 (Method 3 ) |
| | N-[(2S)-1-(4-{[5-(3,4-dimethyl-1,2-oxazol-5-yl)thiophen-2-yl]sulfonyl}piperazin-1-yl)propan-2-yl]-7-methylthieno[3,2-d]pyrimidin-4-amine | ¹H NMR (400 MHz, *Acetone-d₆*) δ ppm 8.36 - 8.56 (m, 1 H), 7.54 - 7.69 (m, 2 H), 7.48 (br d, *J*=1.10 Hz, 1 H), 6.22 - 6.59 (m, 1 H), 4.60 (br d, *J*=6.36 Hz, 1 H), 3.05 (br s, 4 H), 2.48 - 2.74 (m, 6 H), 2.17 - 2.37 (m, 9 H), 1.16 - 1.34 (m, 3 H) |
| 29 | 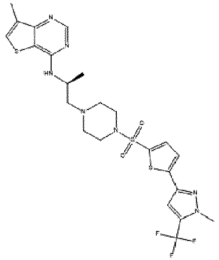 | LC-MS (ESI): m/z (M+1): 586.18 (Method 3) |
| | 7-methyl-N-[(2S)-1-[4-({5-[1-methyl-5-(trifluoromethyl)-1H-pyrazol-3-yl]thiophen-2-yl}sulfonyl)piperazin-1- yl]propan-2-yl]thieno[3,2-d]pyrimidin-4-amine | ¹H NMR (400 MHz, *Acetone-d₆*) δ ppm 8.33-8.63 (m, 1H), 7.46-7.62 (m, 3H), 7.30 (s, 1 H), 4.54 - 4.91 (m, 1 H), 4.06 (s, 3 H), 2.40 - 3.56 (m, 10 H),2.34 (s, 3 H), 1.23 - 1.37 (m, 3 H) (Missing NH proton) |
| 30 | 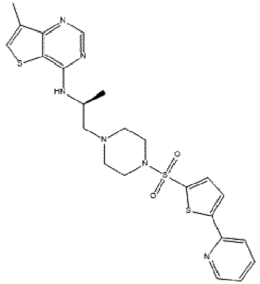 | LC-MS (ESI): m/z (M+1): 515.18 (Method 3 ) |
| | 7-methyl-N-[(2S)-1-(4-{[5-(pyridin-2-yl)thiophen-2-yl]sulfonyl}piperazin-1-yl)propan-2-yl]thieno[3,2-d]pyrimidin-4-amine | ¹H NMR (400 MHz, *Acetone-d₆*) δ ppm 8.58 (br d, *J*=4.82 Hz, 1 H), 8.46 (s, 1 H), 7.95 - 8.22 (m, 1 H), 7.90 (td, *J*=7.73, 1.64 Hz, 1 H), 7.82 (d, *J*=3.95 Hz, 1 H), 7.55 (d, *J*=3.95 Hz, 1 H), 7.32 - 7.49 (m, 2 H), 6.44 (br d, *J*=7.02 Hz, 1 H), 4.54 - 4.72 (m, 1 H), 3.05 (br s, 4 H), 2.41 - 2.76 (m, 6 H), 2.30 (s, 3 H), 1.20 - 1.43 (m, 3 H) |

### Example 22

### N-[5-({4-[(2S)-2-({2-cyclopropyl-7-methylthieno[3,2-d]pyrimidin-4-yl}amino)propyl]piperazin-1-yl}sulfonyl)-1,3-thiazol-2-yl]acetamide

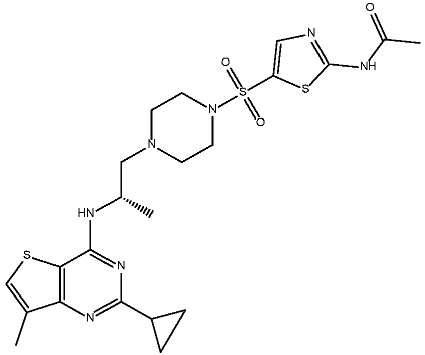

### Step 1: Preparation of tert-butyl 4-[(2S)-2-({2-cyclopropyl-7-methylthieno[3,2-d]pyrimidin-4-yl}amino)propyl]piperazine-1-carboxylate (Intermediate 17)

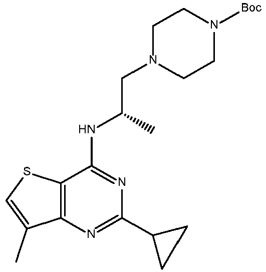

To a mixture of tert-butyl 4-[(2S)-2-aminopropyl]piperazine-1-carboxylate hydrochloride (0.763 g, 2.73 mmol) and 4-chloro-2-cyclopropyl-7-methylthieno[3,2-d]pyrimidine (0.613 g, 2.73 mmol) in EtOH 96% (6 mL), DIPEA (1.43 mL, 8.18 mmol) was added and the mixture was heated at 85°C for 35 h. The volatiles were removed under reduced pressure and the residue was partitioned between DCM and water. The aqueous phase was extracted with DCM and the combined organic layers were washed with sat. NaHCO₃, dried over sodium sulfate, filtered and concentrated. The residue was purified by flash chromatography eluting with (DCM : MeOH = 99.5 : 0.5 to 95 : 5) to afford title compound as pale yellow solid (0.870 g, 2.011 mmol, 74% yield).
LC-MS (ESI): *m*/*z* (M+1): 432.2 (Method 4)

### Step 2: Preparation of 2-cyclopropyl-7-methyl-N-[(2S)-1-(piperazin-1-yl)propan-2-yl]thieno[3,2-d]pyrimidin-4-amine (Intermediate 18)

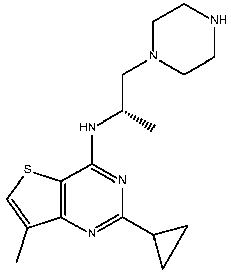

To a solution of tert-butyl 4-[(2S)-2-({2-cyclopropyl-7-methylthieno[3,2-d]pyrimidin-4-yl}amino)propyl]piperazine-1-carboxylate (Intermediate 17, 0.868 g, 2.011 mmol) in DCM (15 mL) cooled to 5°C, 4M HCl in dioxane (5.52 mL, 14.08 mmol) was added and the mixture was left to warm to r.t. and stirred overnight. Additional 4M HCl in dioxane (0.503 mL) was added and the mixture was stirred at r.t. for further 24 h. The volatiles were removed under reduced pressure to afford 2-cyclopropyl-7-methyl-N-[(2S)-1-(piperazin-1-yl)propan-2-yl]thieno[3,2-d]pyrimidin-4-amine as multiple HCl salt (0.970 g). 0.300 g of this salt were dissolved in MeOH, charged on SCX cartridge (5 g) and washed with MeOH. The product was eluted with 2M NH₃ in MeOH and evaporated to afford title compound as a free base (0.200 g, 0.604 mmol, 96% yield).
LC-MS (ESI): *m*/*z* (M+1): 332.0 (Method 4 )

### Step 3: Preparation of N-[5-({4-[(2S)-2-({2-cyclopropyl-7-methylthieno[3,2-d]pyrimidin-4-yl } amino)propyl]piperazin-1-yl } sulfonyl)-1,3-thiazol-2-yl]acetamide (Example 22)

To a solution of 2-cyclopropyl-7-methyl-N-[(2S)-1-(piperazin-1-yl)propan-2-yl]thieno[3,2-d]pyrimidin-4-amine (Intermediate 18, 0.070 g, 0.211 mmol) and pyridine (0.051 mL, 0.634 mmol) in DCM (3 mL) cooled to 0°C, 2-acetamido-1,3-thiazole-5-sulfonyl chloride (0.051 g, 0.211 mmol) was added and the mixture was left to warm to r.t. and stirred for 3 h. The mixture was partitioned between DCM and water. The organic phase was washed with sat. NaHCO₃, dried over sodium sulfate, filtered and concentrated. The crude was purified by flash chromatography eluting with (DCM : MeOH = 99.5 : 0.5 to 95 : 5) to afford title compound as a pale yellow foam (0.087 g, 0.162 mmol, 77% yield).
LC-MS (ESI): m/z (M+1): 536.1 (Method 4)
¹H NMR (300 MHz, *DMSO-d₆*) δ ppm 12.74 (s, 1 H), 7.95 (s, 1 H), 7.60 (s, 1 H), 7.21 (d, 1 H), 4.32 - 4.50 (m, 1 H), 2.92 (br s, 4 H), 2.38 - 2.65 (m, 5 H), 2.22 - 2.34 (m, 4 H), 2.20 (s, 3 H), 1.95 - 2.08 (m, 1 H), 1.16 (d, 3 H), 0.81 - 1.02 (m, 4 H)

The Examples in the following table were prepared from commercially available reagents by using methods analogous to Example 22.

| **Example No.** | **Structure & Name** | **Analytical data** |
|---|---|---|
| 31 | 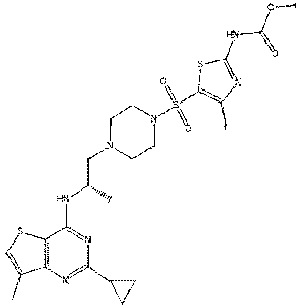 methyl N-[5-({4-[(2S)-2-({2-cyclopropyl-7-methylthieno[3,2-d]pyrimidin-4-yl}amino)propyl]piperazin-1-yl}sulfonyl)-4-methyl-1,3-thiazol-2-yl]carbamate | LC-MS (ESI): m/z (M+1): 566.2 (Method 4 ) |
| | | ¹H NMR (300 MHz, *DMSO-d₆*) δ ppm 12.35 (br s, 1 H), 7.62 (s, 1 H), 7.23 (d, 1 H), 4.33 - 4.55 (m, 1 H), 3.79 (s, 3 H), 2.99 (br s, 4 H), 2.47 - 2.70 (m, 5 H), 2.46 (s, 3 H), 2.18 - 2.38 (m, 4 H), 1.95 - 2.15 (m, 1 H), 1.18 (d, *J* = 6.5 Hz, 3 H), 0.85 - 1.05 (m, 4 H) |
| 35 | 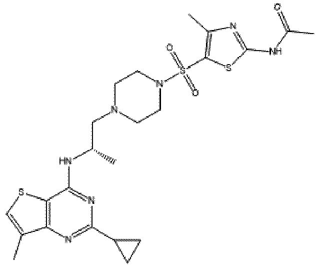 N-[5-({4-[(2S)-2-({2-cyclopropyl-7-methylthieno[3,2-d]pyrimidin-4-yl} amino)propyl]piperazin-1-yl } sulfonyl)-4-m ethyl-1, 3 -thi azol-2-yl]acetamide | LC-MS (ESI): m/z (M+1): 550.3 (Method X) |
| | | ¹H NMR (300 MHz, *DMSO-d₆*) δ ppm 12.62 (s, 1 H), 7.60 (s, 1 H), 7.22 (d, *J* = 7.69, 1 H), 4.31 - 4.51 (m, 1 H), 2.97 (br s, 4 H), 2.51 - 2.67 (m, 5 H), 2.47 (s, 3 H), 2.23 - 2.38 (m, 4 H), 2.18 (s, 3 H), 1.95 - 2.10 (m, 1 H), 1.16 (d, *J* = 6.67 Hz, 3 H), 0.80 - 1.03 (m, 4 H). |

### Example 32

### methyl N-[5-({4-[(2S)-2-{[2-chloro-7-(trifluoromethyl)thieno[3,2-d]pyrimidin-4-yl]amino}propyl]piperazin-1-yl}sulfonyl)-4-methyl-1,3-thiazol-2-yl]carbamate

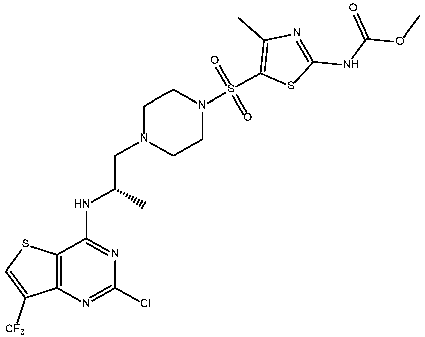

### Step 1: Preparation of tert-butyl 4-[(2S)-2-{[2-chloro-7-(trifluoromethyl)thieno[3,2-d]pyrimidin-4-yl]amino}propyl]piperazine-1-carboxylate (Intermediate 19)

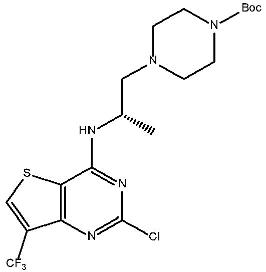

Title compound was prepared following the procedure used for the synthesis of Intermediate 3, starting from 2,4-dichloro-7-(trifluoromethyl)thieno[3,2-d]pyrimidine, [prepared using procedure described in WO 2013078765 compound 7-5] (50 mg, 0.183 mmol) to provide title compound (83 mg, 0.172 mmol, 94% yield) as a whitenish solid.
LC-MS (ESI): m/z (M+1): 480.11 (Method 5 )
¹H NMR (400 MHz, *Ac-d₆*) δ ppm 8.64 (s, 1 H), 7.42 (br d, *J*=7.45 Hz, 1 H), 4.55 - 4.73 (m, 1 H), 3.30 (br t, *J*=4.60 Hz, 4 H), 2.37 - 2.65 (m, 6 H), 1.41 (s, 9 H), 1.33 (d, *J*=6.58 Hz, 3 H)

### Step 2: Preparation of 2-chloro-N-[(2S)-1-(piperazin-1-yl)propan-2-yl]-7-(trifluoromethyl)thieno[3,2-d]pyrimidin-4-amine (intermediate 20)

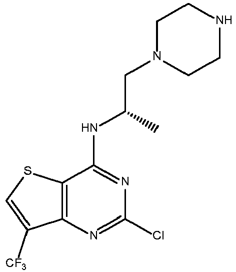

Title compound was prepared following the procedure used for the synthesis of Intermediate 4, starting from tert-butyl 4-[(2S)-2-{[2-chloro-7-(trifluoromethyl)thieno[3,2-d]pyrimidin-4-yl]amino}propyl]piperazine-1-carboxylate (Intermediate 19, 83 mg, 0.17 mmol) to afford title compound (64 mg, 0.168 mmol, 98.% yield) as a yellowish solid.
LC-MS (ESI): m/z (M+1): 380.06 (Method 5 )

### Step 3: Preparation of methyl N-[5-({4-[(2S)-2-{[2-chloro-7-(trifluoromethyl)thieno[3,2-d]pyrimidin-4-yl]amino}propyl]piperazin-1-yl}sulfonyl)-4-methyl-1,3-thiazol-2-yl]carbamate (Example 32)

To a solution of 2-chloro-N-[(2S)-1-(piperazin-1-yl)propan-2-yl]-7-(trifluoromethyl)thieno[3,2-d]pyrimidin-4-amine (intermediate 20, 42 mg, 0.086 mmol) in dry pyridine (0.7 ml) methyl N-[5-(chlorosulfonyl)-4-methyl-1,3-thiazol-2-yl]carbamate (30 mg, 0.112 mmol) was added and the solution was sonicated for 5 min. After evaporation of pyridine, the residue was treated with heptane (35 ml) and the solvent was evaporated under vacuum. The crude was dissolved in DMF (1ml) and purified by flash chromatography in reverse phase (gradient A:B from 100:0 to 0:100 with 12 CV, eluent A: H2O:ACN:HCOOH 95:5:0.1 eluent B:H2O:ACN:HCOOH 5:95:0.1). Appropriate fractions were combined and evaporated to give title compound (20.1 mg, 0.033 mmol, 37.9 % yield) as white solid
LC-MS (ESI): m/z (M+1): 614.10 (Method 5 )
1H NMR (600 MHz, *Acetone-d₆*) δ ppm 8.55 (s, 1 H) 7.40 (br d, *J*=7.87 Hz, 1 H) 4.64 - 4.73 (m, 1 H) 3.84 (s, 3 H) 3.09 (br s, 4 H) 2.80 (br s, 2 H) 2.73 (dd, *J*=12.76, 8.82 Hz, 1 H) 2.59 - 2.68 (m, 2 H) 2.48 (dd, *J*=12.70, 5.30 Hz, 1 H) 2.41 (s, 3 H) 1.29 (d, *J*=6.68 Hz, 3 H)2.41 (s, 3 H) 1.29 (d, *J*=6.68 Hz, 3 H)

### Example 33

### tert-butyl 3-{[4-methyl-5-({4-[(2S)-2-({7-methylthieno[3,2-d]pyrimidin-4-yl}amino)propyl]piperazin-1-yl}sulfonyl)-1,3-thiazol-2-yl]carbamoyl}azetidine-1-carboxylate

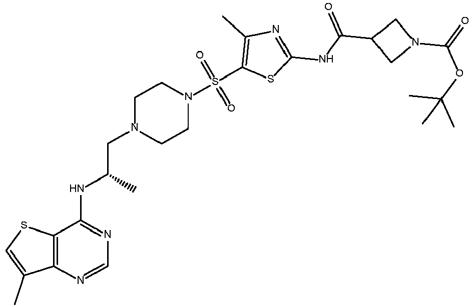

### Step 1: Preparation of 4-methyl-5-({4-[(2S)-2-({7-methylthieno[3,2-d]pyrimidin-4-yl}amino)propyl]piperazin-1-yl} sulfonyl)-1,3 -thiazol-2-amine (Intermediate 21)

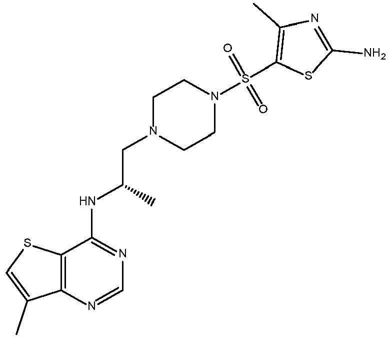

N-[4-methyl-5-({4-[(2S)-2-({7-methylthieno[3,2-d]pyrimidin-4-yl}amino)propyl]piperazin-1-yl}sulfonyl)-1,3-thiazol-2-yl]acetamide (1.48 g 2.90, mmol) was dissolved in 6 mL of acq. H₂SO₄ (50% w/w), the solution was stirred 24 h at 60°C. 30 mL of acq. NaOH 2N was added and a large amount of solids were precipitated. The solids were filtered by suction, washed with water and dried. Thereby the solid was recrystallized in IPA to provide title compound (0.90 g, 1.92 mmol, 66% yield).
LC-MS (ESI): m/z (M+1): 467.98 (Method 3 )

### Step 2: Preparation of tert-butyl 3-{[4-methyl-5-({4-[(2S)-2-({7-methylthieno[3,2-d]pyrimidin-4-yl}amino)propyl]piperazin-1-yl}sulfonyl)-1,3-thiazol-2-yl]carbamoyl}azetidine-1-carboxylate (Example 34)

4-methyl-5-({4-[(2S)-2-({7-methylthieno[3,2-d]pyrimidin-4-yl}amino)propyl]piperazin-1-yl}sulfonyl)-1,3-thiazol-2-amine (Intermediate 21, 87 mg, 0.186 mmol), 1-[(tert-butoxy)carbonyl]azetidine-3-carboxylic acid (90 mg, 0.446 mmol) and HATU (116 mg, 0.305 mmol) were dissolved in and DMF (1.5 ml) , then N-ethyl-N-isopropylpropan-2-amine (0.060 ml, 0.346 mmol) was added in one portion. The solution was stirred on at 50°C. The mixture was partitioned between DCM and water. The organic phase was washed with sat. NaHCO₃, dried over sodium sulfate, filtered and concentrated. The crude was purified by flash chromatography on direct phase (gradient A:B from 100:0 to 0:100 with 10 CV, eluent A: DCM eluent B:DCM/MeOH = 90:10) to afford title compound as an off-white foam (71 mg, 0.109 mmol, 65% yield).
LC-MS (ESI): m/z (M+1): 651.33 (Method 3 )
¹H NMR (400 MHz, *Acetone-d₆*) δ ppm 11.37 (br s, 1 H) 8.46 (s, 1 H) 7.50 (s, 1 H) 6.40 (br d, *J*=7.45 Hz, 1 H) 4.61 (spt, *J*=6.91 Hz, 1 H) 4.02 - 4.21 (m, 4 H) 3.78 (quin, *J*=7.29 Hz, 1 H) 2.99 - 3.11 (m, 4 H) 2.59 - 2.70 (m, 5 H) 2.47 (br d, *J*=6.36 Hz, 1 H) 2.44 (s, 3 H) 2.33 (s, 3 H) 1.41 (s, 9 H) 1.26 (d, *J*=6.58 Hz, 3 H)

### Example 34

### methyl N- [5-({4- [(2S)-2-{[2-cyclopropyl-7-(trifluoromethyl)thieno [3,2-d]pyrimidin-4-yl]amino}propyl]piperazin-1-yl}sulfonyl)-4-methyl-1,3-thiazol-2-yl]carbamate

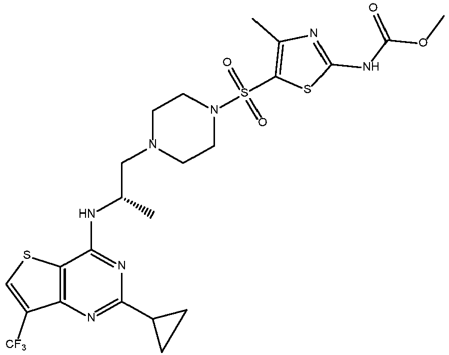

### Step 1: Preparation of tert-butyl 4-[(2S)-2-{[2-cyclopropyl-7-(trifluoromethyl)thieno[3,2-d]pyrimidin-4-yl]amino}propyl]piperazine-1-carboxylate (Intermediate 22)

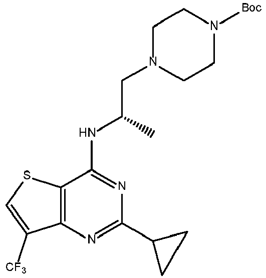

In a 20 mL microvawes vial tert-butyl 4-[(2S)-2-{[2-chloro-7-(trifluoromethyl)thieno[3,2-d]pyrimidin-4-yl]amino}propyl]piperazine-1-carboxylate (100 mg, 0.208 mmol), 2-cyclopropyl-4,4,5,5-tetramethyl-1,3,2-dioxaborolane (0.076 ml, 0.417 mmol) and Na₂CO₃ (66.2 mg, 0.625 mmol) were loaded and suspended in 10 ml of dioxane/water 2:1. The mixture was heated at 100 ° C then Pd(dppf)Cl₂ (15.25 mg, 0.021 mmol) was added.

The vial was sealed and the reaction was stirred at 110 °C overnight. The mixture was partitioned between AcOEt and water. The organic phase was washed with sat. NaHCO₃, dried over sodium sulfate, filtered and concentrated, and the crude was purified by flash chromatography (gradient A:B from 100:0 to 40:60 in 10 CV, Eluent A: n-Heptane, eluent B: aceone).

Appropriate fractions were combined and evaporated to title compound (34.8 mg, 0.072 mmol, 34.4 % yield) as off-white solid.
LC-MS (ESI): rt = 0.80 min; m/z (M+1): 486.18 (Method 3 )

### Step 2: 2-cyclopropyl-N-[(2S)-1-(piperazin-1-yl)propan-2-yl]-7-(trifluoromethyl)thieno[3,2-d]pyrimidin-4-amine (Intermediate 23)

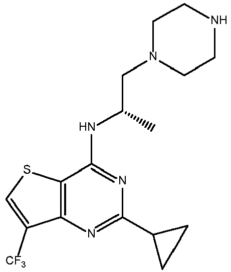

Title compound was prepared following the procedure used for the synthesis of Intermediate 4, starting from tert-butyl 4-[(2S)-2-{[2-cyclopropyl-7-(trifluoromethyl)thieno[3,2-d]pyrimidin-4-yl]amino}propyl]piperazine-1-carboxylate (Intermediate 22, 35 mg, 0.17 mmol) to afford title compound (27 mg, 0.070 mmol, 98.% yield) as a yellowish solid.
LC-MS (ESI): rt = 0.48 min; m/z (M+1): 386.13 (Method 3 )

### Step 3: Preparation of methyl N-[5-({4-[(2S)-2-{[2-cyclopropyl-7-(trifluoromethyl)thieno[3,2-d]pyrimidin-4-yl]amino}propyl]piperazin-1-yl}sulfonyl)-4-methyl-1,3-thiazol-2-yl]carbamate (Example 34)

To a solution of 2-cyclopropyl-N-[(2S)-1-(piperazin-1-yl)propan-2-yl]-7-(trifluoromethyl)thieno[3,2-d]pyrimidin-4-amine (Intermediate 23, 27 mg, 0.070 mmol) in dry pyridine (0.7 ml) methyl N-[5-(chlorosulfonyl)-4-methyl-1,3-thiazol-2-yl]carbamate (23 mg, 0.084 mmol) was added and the solution was sonicated for 5 min. After evaporation of pyridine, the residue was treated with heptane (35 ml) and the solvent was evaporated under vacuum. The crude was purified by flash chromatography in direct phase (gradient A:B from 100:0 to 0:100 with 10 CV, eluent A:DCM eluent B:DCM:EtOH 80:20). Appropriate fractions were combined and evaporated to give title compound (38.9 mg, 0.063 mmol, 90 % yield) as off-white solid
LC-MS (ESI):rt= 1.47min; m/z (M+1): 620.11 (Method 5)
¹H NMR (400 MHz, *Acetone-d₆*) δ ppm 10.75 - 11.02 (m, 1 H) 8.43 (s, 1 H) 6.72 (br d, *J*=7.23 Hz, 1 H) 4.55 - 4.68 (m, 1 H) 3.85 (s, 3 H) 3.11 (br t, *J*=4.60 Hz, 4 H) 2.62 -2.88 (m, 6 H) 2.43 (s, 3 H) 2.08 - 2.16 (m, 1 H) 1.29 (d, J=6.58 Hz, 3 H) 0.82 - 1.09 (m, 4 H)

### Example 36

### N-[5-({4-[(2S)-2-({2-ethyl-7-methylthieno[3,2-d]pyrimidin-4-yl}amino)propyl]piperazin-1-yl}sulfonyl)-4-methyl-1,3-thiazol-2-yl[acetamide

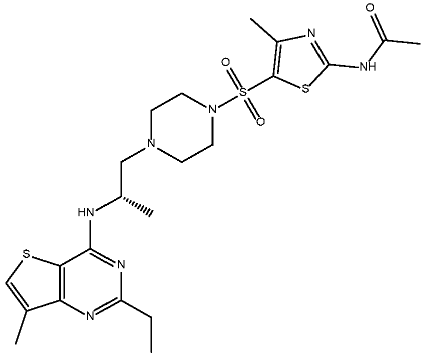

### Step 1: Preparation of tert-butyl 4-[(2S)-2-({2-ethyl-7-methylthieno[3,2-d]pyrimidin-4-yl}amino)propyl]piperazine-1-carboxylate (Intermediate 24)

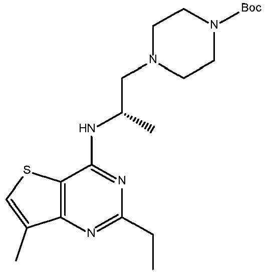

To a mixture of tert-butyl 4-[(2S)-2-aminopropyl]piperazine-1-carboxylate hydrochloride (0.714 g, 2.55 mmol) and 4-chloro-2-ethyl-7-methylthieno[3,2-d]pyrimidine (0.512 g, 2.41 mmol) in EtOH 96% (7.2mL), DIPEA (1.26 mL) was added and the mixture was heated at 85°C for 36 h. The volatiles were removed under reduced pressure and the residue was partitioned between DCM and water. The aqueous phase was extracted with DCM and the combined organic layers were washed with sat. NaHCO₃, dried over sodium sulfate, filtered and concentrated. The residue was purified by flash chromatography on silica gel column (DCM to DCM : MeOH = 95 : 5) to afford title compound (yield considered to be quantitative).
LC-MS (ESI): *m*/*z* (M+1): 420.3 (Method)

### Step 2: 2-ethyl-7-methyl-N-[(2S)-1-(piperazin-1-yl)propan-2-yl]thieno[3,2-d]pyrimidin-4-amine (Intermediate 25)

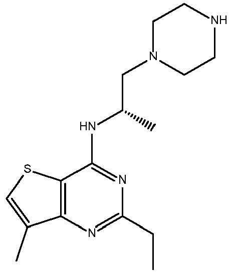

To a solution of crude tert-butyl 4-[(2S)-2-({2-ethyl-7-methylthieno[3,2-d]pyrimidin-4-yl}amino)propyl]piperazine-1-carboxylate (Intermediate 24, theoretical 2.4 mmol) in DCM (16 mL) cooled to 5°C, 4M HCl in dioxane (4 mL, 16.0 mmol) was added and the mixture was left to warm to r.t. and stirred overnight. The volatiles were removed under reduced pressure and the residue was dissolved in MeOH, charged on SCX cartridge (2 × 10 g) and washed with MeOH. The product was eluted with 2M NH₃ in MeOH and the volatiles were evaporated to afford compound title compound as a free base (0.600 g, 1.88 mmol, 78% yield over two steps).
LC-MS (ESI): *m*/*z* (M+1): 320.1 (Method 4 )

### Step 3: Preparation ofN-[5-({4-[(2S)-2-({2-ethyl-7-methylthieno[3,2-d]pyrimidin-4-yl}amino)propyl]piperazin-1-yl}sulfonyl)-4-methyl-1,3-thiazol-2-yl]acetamide (Example 36)

To a solution of 2-ethyl-7-methyl-N-[(2S)-1-(piperazin-1-yl)propan-2-yl]thieno[3,2-d]pyrimidin-4-amine (Intermediate 25, 0.104 g, 0.33 mmol) and pyridine (0.082 mL, 0.99 mmol) in DCM (3 mL) cooled to 0°C, 2-acetamido-4-methyl-1,3-thiazole-5-sulfonyl chloride (0.083 g, 0.33 mmol) was added and the mixture was left to warm to r.t. and stirred for 3 h. The mixture was diluted with DCM and washed with water, and the aqueous phase was extracted with DCM. The combined organic layers were washed with sat. NaHCO₃, dried over sodium sulfate, filtered and concentrated. The residue was purified by flash chromatography eluting with DCM : MeOH = 99.5 : 0.5 to 96 : 4 to afford title compound as white foam (0.101 g, 0.188 mmol, 58% yield).
LC-MS (ESI): m/z (M+1): 538.2 (Method 4)
¹H NMR (300 MHz, *DMSO-d6)* δ ppm 12.62 (s, 1 H), 7.62 (d, *J* = 0.98 Hz, 1 H), 7.25 (d, *J* = 8.03 Hz, 1 H), 4.42 - 4.65 (m, 1 H), 2.94 (br s, 4 H), 2.70 (q, *J* = 7.57 Hz, 2 H), 2.46 - 2.65 (m, 5 H), 2.45 (s, 3 H), 2.22 - 2.38 (m, 4 H), 2.18 (s, 3 H), 1.24 (t, *J* = 7.57 Hz, 3 H), 1.17 (d, *J* = 6.53 Hz, 3 H)

The Example in the following table was prepared from commercially available reagents by using methods analogous to Example 36.

| **Example No.** | **Structure & Name** | **Analytical data** |
|---|---|---|
| 38 | 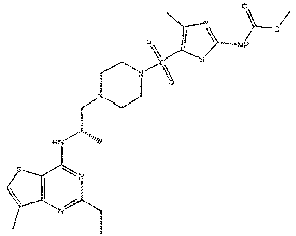 | LC-MS (ESI): m/z (M+1): 554.1 (Method 4) |
| | methyl N-[5-({4-[(2S)-2-({2-ethyl-7-methylthieno[3,2-d]pyrimidin-4-yl}amino)propyl]piperazin-1-yl}sulfonyl)-4-methyl-1,3-thiazol-2-yl]carbamate | ¹H NMR (300 MHz, *DMSO-d₆*) δ ppm 12.57 (s, 1 H), 7.63 (d, *J* = 0.97 Hz, 1 H), 7.24 (d, 1 H), 4.42 - 4.62 (m, 1 H), 3.78 (s, 3 H), 2.95 (br s, 4 H), 2.70 (q, *J* = 7.56 Hz, 2 H), 2.45 - 2.63 (m, 5 H), 2.43 (s, 3 H), 2.19 - 2.38 (m, 4 H), 1.25 (t, *J* = 7.56 Hz, 3 H), 1.18 (d, *J* = 6.48 Hz, 3 H) |

### Example 37

### Methyl N-[5-({4-[(2S)-2-({2,7-dimethylthieno[3,2-d]pyrimidin-4-yl}amino)propyl]piperazin-1-yl}sulfonyl)-4-methyl-1,3-thiazol-2-yl]carbamate

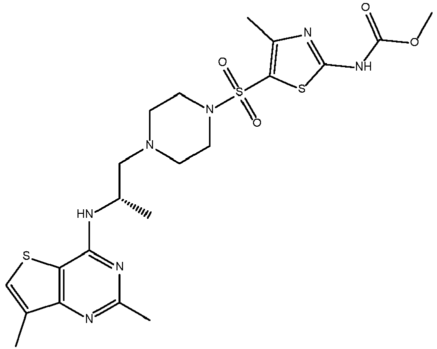

### Step 1: Preparation of tert-butyl 4-[(2S)-2-({2,7-dimethylthieno[3,2-d]pyrimidin-4-yl}amino)propyl]piperazine-1-carboxylate (Intermediate 26)

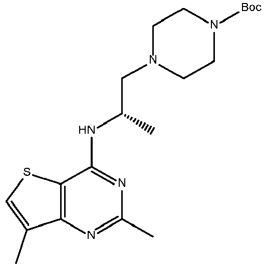

To a mixture of tert-butyl 4-[(2S)-2-aminopropyl]piperazine-1-carboxylate hydrochloride (1.197 g, 4.278 mmol) and 4-chloro-2,7-dimethylthieno[3,2-d]pyrimidine (0.850 g, 4.278 mmol) in EtOH (9 mL), DIPEA (2.236 mL, 12.835 mmol) was added and the mixture was heated at 85°C for 32 h. The mixture was cooled to r.t., the volatiles were removed under reduced pressure and the residue was partitioned between DCM and water. The aqueous phase was extracted with DCM and the combined organic layers were washed with sat. NaHCO₃ and brine, dried over sodium sulfate, filtered and concentrated. The residue was purified by flash chromatography on Biotage silica Ultra 25g cartridge (DCM: MeOH = 99.5 : 0.5 to 95 : 5) to afford title compound as pale yellow foam (74% yield).
LC-MS (ESI): *m*/*z* (M+1): 406.0 (Method 4 )

### Step 2: 2,7-dimethyl-N-[(2S)-1-(piperazin-1-yl)propan-2-yl]thieno[3,2-d]pyrimidin-4-amine (Intermediate 27)

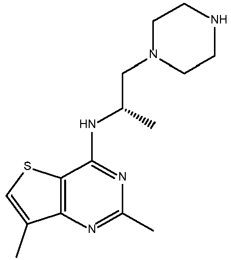

To a solution of tert-butyl 4-[(2S)-2-({2,7-dimethylthieno[3,2-d]pyrimidin-4-yl}amino)propyl]piperazine-1-carboxylate (Intermediate 26, 1.5 g, 3.7 mmol) in DCM (30 mL) cooled to 5°C, 4M HCl in dioxane (6.5 mL, 25.9 mmol) was added and the mixture was left to warm to r.t. and stirred overnight. The volatiles were removed under reduced pressure to afford (S)-2,7-dimethyl-N-(1-(piperazin-1-yl)propan-2-yl)thieno[3,2-d]pyrimidin-4-amine as multiple HCl salt (1.6 g). 0.300 g of this salt were dissolved in MeOH, charged on SCX cartridge (5 g) and washed with MeOH. The product was eluted with 2M NH₃ in MeOH /DCM and evaporated to afford title compound as a free base, as pale yellow foam (0.220 g).
LC-MS (ESI): *m*/*z* (M+1): 306.0 (Method 4 )

### Step 3: Preparation of methyl N-[5-({4-[(2S)-2-({2,7-dimethylthieno[3,2-d]pyrimidin-4-yl} amino)propyl]piperazin-1 -yl} sulfonyl)-4-m ethyl-1,3 -thiazol-2-yl]carbamate (Example 37)

To a solution of 2,7-dimethyl-N-[(2S)-1-(piperazin-1-yl)propan-2-yl]thieno[3,2-d]pyrimidin-4-amine (Intermediate 27, 0.080 g, 0.262 mmol) and pyridine (0.064 mL, 0.786 mmol) in DCM (3.5 mL) cooled to 0°C, methyl N-[5-(chlorosulfonyl)-4-methyl-1,3-thiazol-2-yl]carbamate (0.071 g, 0.262 mmol) was added and the mixture was left to warm to r.t. and stirred for 3 h. The mixture was diluted with DCM and washed with water. The organic phase was washed with sat. NaHCO₃, dried over sodium sulfate, filtered and concentrated. The crude was purified by flash chromatography on silica Sepachrom 4 g cartridge (DCM : MeOH = 99.5 : 0.5 to 95 : 5) to afford title compound as a pink foam (0.082 g, 0.152 mmol, 58% yield).
LC-MS (ESI): m/z (M+1): 540.1 (Method 4)
¹H NMR (300 MHz, *DMSO-d6)* δ ppm 12.60 (s, 1 H), 7.62 (s, 1 H), 7.25 (d, 1 H), 4.41 - 4.57 (m, 1 H), 3.78 (s, 3 H), 2.95 (br s, 4 H), 2.46 - 2.67 (m, 5 H), 2.43 (s, 3 H), 2.21 - 2.36 (m, 4 H), 1.16 (d, *J* = 6.50 Hz, 3 H)

### Example 41

### methyl N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]-N-methylcarbamate

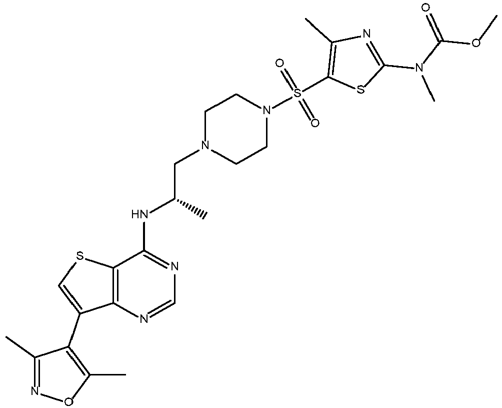

### Step 1: Preparation of 4-methyl-2-(methylamino)-1,3-thiazole-5-sulfonyl chloride (Intermediate 28)

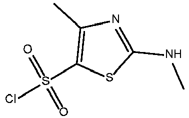

To a solution of N,4-dimethyl-1,3-thiazol-2-amine (250 mg, 1.95 mmol) in DCM (2.5 mL), sulfurochloridic acid (1.95 mL, 29.25 mmol) was slowly added at 25 °C. The reaction was then stirred at 25 °C for 2h and then quenched with cold water and extracted with DCM (twice). The solvent was removed under vacuum and the crude product (53 mg, 0.23 mmol, 12 % yield) was used in the next step without any further purification.
LC-MS (ESI): *m*/*z* (M+1): 223 (Method 1)

### Step 2: Preparation of 7-(3,5-dimethyl-1,2-oxazol-4-yl)-N-[(2S)-1-[4-[[4-methyl-2-(methylamino)-1,3-thiazol-5-yl]sulfonyl]piperazin-1-yl]propan-2-yl]thieno[3,2-d]pyrimidin-4-amine (Intermediate 29)

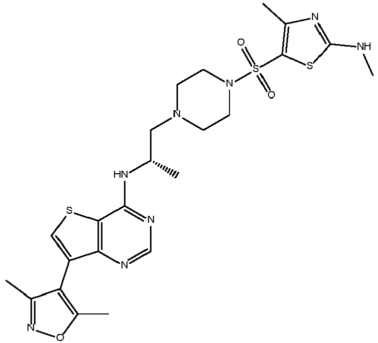

Title compound was prepared following the procedure used for the synthesis of Example 1, starting from 7-(3,5-dimethyl-1,2-oxazol-4-yl)-N-[(2S)-1-piperazin-1-ylpropan-2-yl]thieno[3,2-d]pyrimidin-4-amine hydrochloride (Intermediate 9, 70 mg, 0.17 mmol) and 4-methyl-2-(methylamino)-1,3-thiazole-5-sulfonyl chloride (Intermediate 28, 53 mg, 0.23 mmol) to afford the title compound (36.4 mg, 0.06 mmol, 38% yield).
LC-MS (ESI): m/z (M+1): 563.2 (Method 1)

### Step 3: Preparation of methyl N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]-N-methylcarbamate (Example 41)

To a solution of 7-(3,5-dimethyl-1,2-oxazol-4-yl)-N-[(2S)-1-[4-[[4-methyl-2-(methylamino)-1,3-thiazol-5-yl]sulfonyl]piperazin-1-yl]propan-2-yl]thieno[3,2-d]pyrimidin-4-amine (Intermediate 29, 36.4 mg, 0.06 mmol) in DCM (1 mL) at 0 °C was added N,N-dimethyl-4-pyridinamine (19.8 mg, 0.16 mmol), followed by Chloroformic acid methyl ester (0.01 mL, 0.130 mmol) and the mixture was stirred at room temperature for 2h. Solvent was removed under reduced pressure and the crude purified by flash chromatography eluting with MeOH in DCM from 0% to 2% affording title compound (Example 41, 24 mg, 0.039 mmol, 59.7% yield)
LC-MS (ESI): m/z (M+1): 621.06 (Method 2)
¹H NMR (400 MHz, *DMSO-d6)* δ ppm 8.41 (s, 1 H), 8.13 (s, 1 H), 7.66 (d, *J*=8.3 Hz, 1 H), 4.55 (dt, *J*=14.3, 6.9 Hz, 1 H), 3.85 (s, 3 H), 3.46 (s, 3 H), 2.98 (br s, 4 H), 2.54 - 2.62 (m, 5 H), 2.47 (s, 3 H), 2.17 - 2.42 (m, 7 H), 1.17 (d, *J*=6.6 Hz, 3 H)

### Example 50

### 2-methoxy-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]acetamide

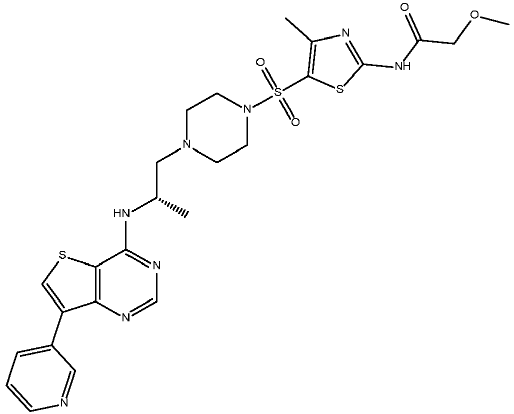

### Step 1: Preparation of tert-butyl 4-[(2S)-2-[(7-bromothieno[3,2-d]pyrimidin-4-yl)amino]propyl]piperazine-1-carboxylate (Intermediate 30)

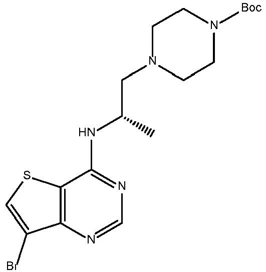

Title compound was prepared following the procedure used for the synthesis of Intermediate 3, starting from 7-bromo-4-chlorothieno[3,2-d]pyrimidine (1 g, 4.01 mmol) to provide title compound (1.64 g, 3.6 mmol, 90 % yield).
LC-MS (ESI): *m*/*z* (M+1): 458.1 (Method 1)

### Step 2: Preparation of tert-butyl 4-[(2S)-2-[(7-pyridin-3-ylthieno[3,2-d]pyrimidin-4-yl)amino]propyl]piperazine-1-carboxylate (Intermediate 31)

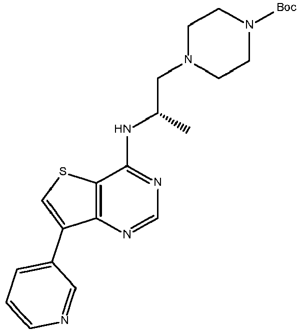

To a suspension of tert-butyl 4-[(2S)-2-[(7-bromothieno[3,2-d]pyrimidin-4-yl)amino]propyl]piperazine-1-carboxylate (Intermediate 30, 250 mg, 0.55 mmol), K₃PO₄ (232 mg, 1.1 mmol) in Water (0.5 mL) and THF (1.5 mL), 3-pyridinylboronic acid (101 mg, 0.82 mmol) was added. After degassing with nitrogen Pd(dppf)Cl₂ (12 mg, 0.02 mmol) was added and the tube was sealed. The reaction was heated at 80 °C for 24 h. Water was added and the mixture was extracted with AcOEt. The organic layer was dried over sodium sulphate and concentrated under reduced pressure. The crude was purified by flash chromatography on a 28g NH column eluting with AcOEt in Cyclohexane from 0% to 65% affording title compound (158 mg, 0.35 mmol, 64 % yield).
LC-MS (ESI): m/z (M+1): 455.2 (Method 2)

### Step 3: Preparation of N-[(2S)-1-piperazin-1-ylpropan-2-yl]-7-pyridin-3-ylthieno[3,2-d]pyrimidin-4-amine hydrochloride (Intermediate 32)

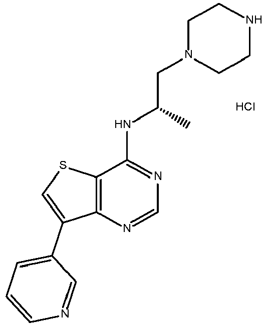

Title compound was prepared following the procedure used for the synthesis of Intermediate 9, starting from tert-butyl 4-[(2S)-2-[(7-pyridin-3-ylthieno[3,2-d]pyrimidin-4-yl)amino]propyl]piperazine-1-carboxylate (Intermediate 31, 158.6 mg, 0.350 mmol) to afford title compound (165 mg, crude) that was used in the next step without further purification.
LC-MS (ESI): *m*/*z* (M+1): 355 (Method 2)

### Step 4: Preparation of N-[4-methyl-5-[4-[(2S)-2-[(7-pyridin-3-ylthieno[3,2-d]pyrimidin-4-yl)amino]propyl]piperazin-1-yl]sulfonyl-1,3-thiazol-2-yl]acetamide (Intermediate 33)

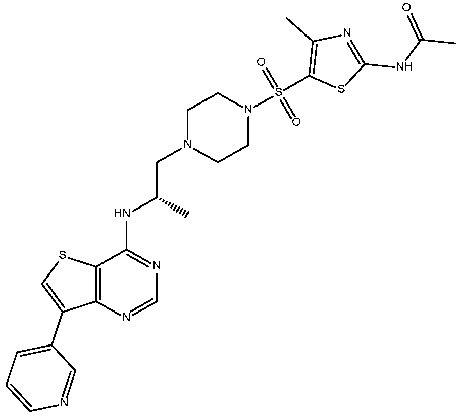

Title compound was prepared following the procedure used for the synthesis of Example 1, starting from N-[(2S)-1-piperazin-1-ylpropan-2-yl]-7-pyridin-3-ylthieno[3,2-d]pyrimidin-4-amine hydrochloride (Intermediate 32, 136 mg, 0.349 mmol) to afford title compound (191.4 mg, 0.33 mmol, 96% yield) as a whitenish solid.
LC-MS (ESI): m/z (M+1): 573.1 (Method 2)

### Step 5: Preparation of N-[(2S)-1-[4-[(2-amino-4-methyl-1,3-thiazol-5-yl)sulfonyl]piperazin-1-yl]propan-2-yl]-7-pyridin-3-ylthieno[3,2-d]pyrimidin-4-amine (Intermediate 34)

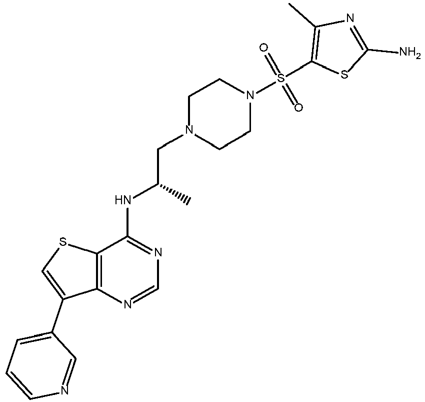

Compound N-[4-methyl-5-[4-[(2S)-2-[(7-pyridin-3-ylthieno[3,2-d]pyrimidin-4-yl)amino]propyl]piperazin-1-yl]sulfonyl-1,3-thiazol-2-yl]acetamide (Intermediate 33, 191.4 mg, 0.33 mmol) was dissolved in a mixture of sulfuric acid (1.89 mL, 34 mmol) and Water (1.9 mL) and the mixture was stirred at 80 °C for 16 h. The mixture was cooled down at 0 ° C and water was added followed by a solution of NaOH 5M until pH 6. Then NaHCO₃ sat. solution was added dropwise and a white precipitate was formed. The precipitate was filtered off on a buchner funnel and washed with water to title compound (140 mg, 0.26 mmol, 79 % yield) that was used in the next step without further purification.
LC-MS (ESI): m/z (M+1): 531.1 (Method 2)

### Step 6: Preparation of 2-methoxy-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]acetamide (Example 50)

Title compound was prepared following the procedure used for the synthesis of Example 41, starting from N-[(2S)-1-[4-[(2-amino-4-methyl-1,3-thiazol-5-yl)sulfonyl]piperazin-1-yl]propan-2-yl]-7-pyridin-3-ylthieno[3,2-d]pyrimidin-4-amine (Intermediate 34, 70 mg, 0.132 mmol) and 2-methoxyacetyl chloride (0.01 mL, 0.160 mmol) to afford the title compound (40 mg, 0.066 mmol, 50% yield).
LC-MS (ESI): m/z (M+1): 603.1 (Method 2)
¹H NMR (400 MHz, *DMSO-d6)* δ ppm 12.60 (br s, 1 H), 9.19 (d, *J*=1.5 Hz, 1 H), 8.56 (dd, *J*=4.7, 1.6 Hz, 1 H), 8.51 (s, 1 H), 8.47 (s, 1 H), 8.42 - 8.46 (m, 1 H), 7.67 (d, *J*=7.9 Hz, 1 H), 7.46 - 7.56 (m, 1 H), 4.47 - 4.63 (m, 1 H), 4.15 (s, 2 H), 3.33 (s, 3 H), 2.99 (br s, 4 H), 2.54 - 2.63 (m, 5 H), 2.46 (s, 3 H), 2.35 - 2.42 (m, 1 H), 1.19 (d, *J*=6.6 Hz, 3 H)

The Example in the following table was prepared from commercially available reagents by using methods analogous to Example 50.

| **Example No.** | **Structure & Name** | **Analytical data** |
|---|---|---|
| 51 | 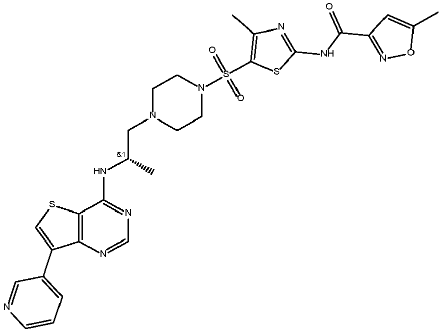 | LC-MS (ESI): m/z (M+1): 640 (Method 2) |
| | | ¹H NMR (400 MHz, *DMSO-d₆*) δ ppm 13.12 - 13.84 (m, 1 H), 9.18 (d, *J*=1.5 Hz, 1 H), 8.55 (dd, *J*=4.7, 1.6 Hz, 1 H), 8.51 (s, 1 H), 8.47 (s, 1 H), 8.43 (dt, *J*=8.1, 1.9 Hz, 1 H), 7.67 (d, *J*=7.9 Hz, 1 H), 7.45 - 7.52 (m, 1 H), 6.79 (s, 1 H), 4.57 (dt, *J*=13.6, 6.8 Hz, 1 H), 3.03 (br |
| | 5-methyl-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]-1,2-oxazole-3-carboxamide | s, 4 H), 2.59 (br s, 5 H), 2.46 - 2.53 (m, 6 H), 2.35 - 2.44 (m, 1 H), 1.19 (d, *J*=6.6 Hz, 3 H) |

### Example 52

### methyl N-[5-({4-[(2S)-2-1[7-(trifluoromethyl)thieno[3,2-d]pyrimidin-4-yl]amino}propyl]piperazin-1-yl}sulfonyl)-1,3-thiazol-2-yl]carbamate

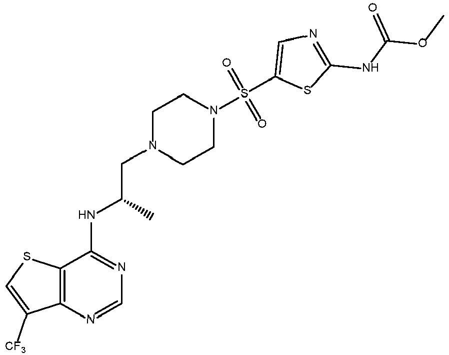

### Step 1: Preparation of N-[5-[4-[(2S)-2-[[7-(trifluoromethyl)thieno[3,2-d]pyrimidin-4-yl]amino]propyl]piperazin-1-yl]sulfonyl-1,3-thiazol-2-yl]acetamide (Intermediate 35)

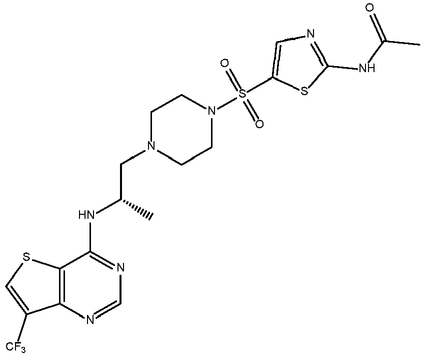

Title compound was prepared following the procedure used for the synthesis of Example 1, starting from N-[(2S)-1-piperazin-1-ylpropan-2-yl]-7-(trifluoromethyl)thieno[3,2-d]pyrimidin-4-amine (Intermediate 4, 40 mg, 0.116 mmol) to afford title compound (45 mg, 0.079 mmol, 71 % yield) as white solid.
LC-MS (ESI): m/z (M+1): 550.2 (Method 1)

### Step 2: Preparation of N-[(2S)-1-[4-[(2-amino-1,3-thiazol-5-yl)sulfonyl]piperazin-1-yl]propan-2-yl]-7-(trifluoromethyl)thieno[3,2-d]pyrimidin-4-amine (Intermediate 36)

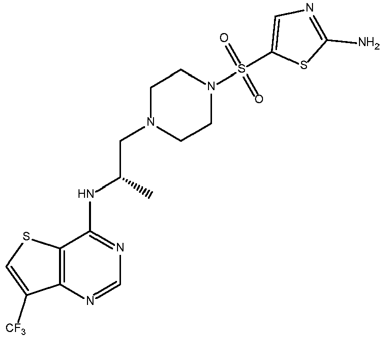

Title compound was prepared following the procedure used for the synthesis of Intermediate 31 starting from N-[5-[4-[(2S)-2-[[7-(trifluoromethyl)thieno[3,2-d]pyrimidin-4-yl]amino]propyl]piperazin-1-yl]sulfonyl-1,3-thiazol-2-yl]acetamide (Intermediate 35, 45 mg, 0.079 mmol) to afford title compound (34 mg, 0.067 mmol, 84 % yield) as a white solid.

### Step 3: Preparation of methyl N-[5-({4-[(2S)-2-{[7-(trifluoromethyl)thieno[3,2-d]pyrimidin-4-yl]amino}propyl]piperazin-1-yl}sulfonyl)-1,3-thiazol-2-yl] carbamate (Example 52)

Title compound was prepared following the procedure used for the synthesis of Example 41 starting from N-[(2S)-1-[4-[(2-amino-1,3-thiazol-5-yl)sulfonyl]piperazin-1-yl]propan-2-yl]-7-(trifluoromethyl)thieno[3,2-d]pyrimidin-4-amine (Intermediate 36, 34 mg, 0.067 mmol) and Chloroformic acid methyl ester (0.01 mL, 0.130 mmol) to afford the title compound (14 mg, 0.024 mmol, 36 % yield).
LC-MS (ESI): m/z (M+1): 566 (Method 2)
¹H NMR (400 MHz, *DMSO-d₆*) δ ppm 12.47 (s, 1 H), 8.75 (q, *J*=1.1 Hz, 1 H), 8.48 (s, 1 H), 7.90 (d, *J*=8.1 Hz, 1 H), 7.87 (s, 1 H), 4.54 (quin, *J*=6.8 Hz, 1 H), 3.77 (s, 3 H), 2.93 (br s, 4 H), 2.52 - 2.61 (m, 5 H), 2.38 (dd, *J*=12.4, 6.9 Hz, 1 H), 1.17 (d, *J*=6.4 Hz, 3 H)

The Example in the following table was prepared from commercially available reagents by using methods analogous to Example 52.

| **Example No.** | **Structure & Name** | **Analytical data** |
|---|---|---|
| 55 | 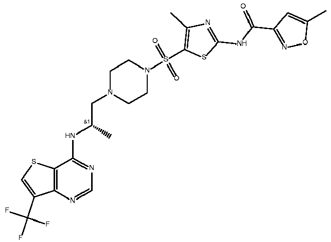 5-methyl-N-[4-methyl-5-({4-[(2S)-2-{[7-(trifluoromethyl)thieno[3,2-d]pyrimidin-4-yl]amino}propyl]piperazin-1-yl} sulfonyl)-1,3 -thiazol-2-yl]-1,2-oxazole-3-carboxamide | LC-MS (ESI): m/z (M+1): 631 (Method 2) |
| | | ¹H NMR (400 MHz, *DMSO-d₆*) δ ppm 13.23 - 13.92 (m, 1 H), 8.75 (s, 1 H), 8.48 (s, 1 H), 7.91 (d, *J*=7.9 Hz, 1 H), 6.79 (br s, 1 H), 4.44 - 4.75 (m, 1 H), 3.01 (br s, 4 H), 2.53 - 2.64 (m, 5 H), 2.45 - 2.53 (m, 6 H), 2.35 - 2.44 (m, 1 H), 1.17 (d, *J*=6.4 Hz, 3 H) |

### PHARMACOLOGICAL ACTIVITY OF THE COMPOUNDS OF THE INVENTION

### In vitro Assays

The effectiveness of compounds of the present invention as LPA2 antagonist can be determined at the human recombinant LPA2 expressed in CHO cells, using a FLIPR assay in 384 well format.

CHO-hLPA2 cell lines are cultured in a humidified incubator at 5% CO2 in DMEM/F-12 (1:1) MIXTURE with 2mM Glutamax, supplemented with 10% of Foetal Bovine Serum, 1 mM Sodium Pyruvate, 11 mM Hepes and 1X Penicillin/Streptomycin. CHO hLPA2 cells are seeded into black walled clear-bottom 384-well plates (#781091, Greiner Bio-One GmbH) at a density of 7,500 cells per well in 50 µl culture media and grown overnight in a 37°C humidified CO2-incubator. Serial dilutions (1:3 or 1:4, 11 points CRC) of compounds are performed in 100% DMSO at 200X the final concentration. The compounds are diluted 1:50 prior to the experiment with Assay Buffer (20 mM HEPES, 145 mM NaCl, 5 mM KCl, 5.5 mM glucose, 1 mM MgCl2 and 2 mM CaCl2, pH 7.4 containing 0.01% Pluronic F-127) to obtain a solution corresponding to 5-fold the final concentration in the assay (4X, 2% DMSO). The final concentration of DMSO in the assay will be 0.5% in each well. Medium is removed by aspiration and cells are then incubated with 30 µl of a loading solution containing 5 µM of the cytoplasmic Ca2+ indicator Cal-520 AM in Assay Buffer containing 2.5 mM probenecid for 30 min at 37°C incubator (cell loading). The loaded cell plates are transferred into the FLIPR instrument and calcium responses are monitored during the on-line addition protocols. For testing of compounds, after the cell loading, 10 µl/well of 4X antagonists' solution was added onto the cells. After 30 min pre-incubation (at 37°C), 10 µl/well of 5X concentrated LPA EC80 was added and Ca2+ mobilization responses was followed during the on-line addition protocol. Intracellular peak fluorescence values subtracted by baseline fluorescence are exported and analysed to determine IC₅₀ values, respectively. The calcium response is expressed as percentage of the maximal inhibition of the EC80 agonist response.

The raw data obtained in unstimulated controls (DMSO, no LPA) are set as "100% inhibition", while the raw data obtained in negative controls, i.e. in the absence of compounds and stimulating with LPA EC80, are set as "0% inhibition".

The raw data (peak height expressed as relative fluorescence units) are normalized and transformed into "percent of inhibition". Curve fitting and pIC₅₀ (-LogIC₅₀) estimations are carried out using a four-parameter logistic model using XLfit Software.

The results for individual compounds are provided below in Table 2 wherein the compounds are classified in term of potency with respect to their inhibitory activity on LPA2 isoform:

**Table 2**

| **Example N** | **LPA2 IC₅₀** |
|---|---|
| 4 | + |
| 25 | + |
| 18 | + |
| 19 | + |
| 24 | + |
| 16 | + |
| 23 | + |
| 15 | + |
| 26 | + |
| 10 | + |
| 5 | + |
| 12 | + |
| 29 | + |
| 13 | + |
| 51 | + |
| 9 | + |
| 6 | + |
| 17 | + |
| 7 | + |
| 33 | + |
| 37 | + |
| 22 | + |
| 20 | + |
| 36 | + |
| 30 | + |
| 11 | + |
| 49 | + |
| 8 | + |
| 50 | + |
| 21 | + |
| 55 | + |
| 14 | + |
| 27 | + |
| 32 | + |
| 54 | ++ |
| 52 | ++ |
| 38 | ++ |
| 35 | ++ |
| 28 | ++ |
| 31 | ++ |
| 41 | ++ |
| 1 | ++ |
| 43 | ++ |
| 53 | ++ |
| 40 | +++ |
| 48 | +++ |
| 2 | +++ |
| 44 | +++ |
| 42 | +++ |
| 34 | +++ |
| 47 | +++ |
| 45 | +++ |
| 39 | +++ |
| 46 | +++ |
| 3 | +++ |

wherein the compounds are classified in term of potency with respect to their inhibitory activity on and LPA2 isoforms according to the following classification criterion:
LPA receptor 2 (LPA2)
+: LPA2 IC₅₀ less than 1000 nM
++: LPA2 IC₅₀ comprised between about 100 nM and 10 nm
+++: LPA2 IC₅₀ less than about 10 nM.

As it can be appreciated in Table 2, the compounds of the present invention show a good activity as antagonists of LPA2.

### Comparative Example A

### Methyl (S)-(4-methyl-5-((4-(2-((5-methyl-7-(pyridin-3-yl)-5H-pyrrolo[3,2-d]pyrimidin-4-yl)amino)propyl)piperazin-1-yl)sulfonyl)thiazol-2-yl)carbamate

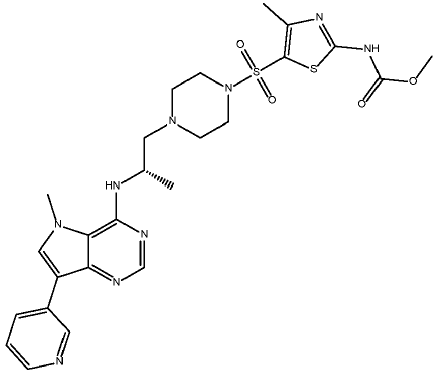

The activity of comparative Example A as has been tested in the in vitro assay for the determination of activity on LPA2 receptor as described above.

Differently from the compounds of formula (I) of the present invention, the comparative Example A shows an IC₅₀ greater than 1 µm, even greater than 2 µm, and thus the compound is inactive on receptor LPA2.

The above results demonstrate that the scaffold of the compounds of formula (I) of the invention comprising a thienopyrimidine moiety linked to the piperazine through an amino-alkyl linker leads unexpectedly to a series of compounds that is active on receptor LPA2.

The scope of the invention and thus of protection is limited by the appended claims. Any subject-matter in the preceding description not encompassed by the claims is not to be understood as forming part of the invention. In particular, the invention does not include any method of therapeutic treatment of the human or animal body, even if such subject-matter is herein described.

## Claims

1. A compound of formula (I) 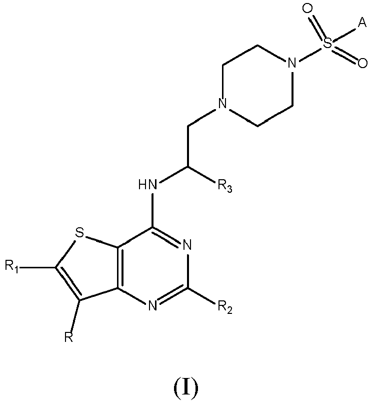 wherein
**R** is H or selected from the group consisting of (C₁-C₄)alkyl, (C₁-C₄)haloalkyl and 5-6 membered heteroaryl, wherein each of said heteroaryl may be optionally substituted by one or more group selected from (C₁-C₄)alkyl, halo, (C₁-C₄)haloalkyl;
**R₁** is H or (C₁-C₄)alkyl;
**R₂** is H or selected from the group consisting of (C₁-C₄)alkyl, halo, (C₁-C₄)haloalkyl and (C₃-C₈)cycloalkyl;
**R₃** is H or (C₁-C₄)alkyl;
**A** is selected from the group consisting of 5-6 membered heteroaryl wherein each of said heteroaryl may be optionally substituted by one or more group selected from (C₁-C₄)alkyl, -C(O)R₁, -C(O)OR₁, -C(O)R₁, (C₁-C₄)haloalkyl, halo,-NR_{A}C(O)R₁, -NR_{A}C(O)OR₁, -NR_{A}C(O)-(C₁-C₄)alkylene-OR₁, -NR_{A}C(O)R_{C},-NR_{A}C(O)NR_{A}R_{B}, -N(C₁-C₄)alkylene-NR_{A}R_{B}, heteroaryl optionally substituted by one or more (C₁-C₄)alkyl and (C₁-C₄)haloalkyl,
**R_{C}** is selected from the group consisting of heteroaryl, aryl, (C₃-C₈) cycloalkyl and (C₄-C₈) heterocycloalkyl wherein said heteroaryl, aryl, heterocycloalkyl and cycloalkyl may be optionally substituted by one or more (C₁-C₄)alkyl and-C(O)OR₁;
**R_{A}** and **R_{B}** are at each occurrence independently H or selected from the group consisting of (C₁-C₄)alkyl, (C₃-C₈)cycloalkyl, (C₁-C₆)haloalkyl and halo, or
R_{A} and R_{B} may form together with the nitrogen atom to which they are attached a 4-6 membered saturated heterocyclic ring system optionally containing a further heteroatom selected from N, S and O, said heterocyclic ring system may be optionally substituted by one or more groups selected from (C₁-C₄)alkyl, (C₁-C₄) haloalkyl and halo.

2. The compound of formula (I) according to claim 1, wherein
**R** is H or selected from the group consisting of (C₁-C₄)alkyl, (C₁-C₄)haloalkyl and 5-6 membered heteroaryl wherein each of said heteroaryl may be optionally substituted by one or more group selected from (C₁-C₄)alkyl, halo, and (C₁-C₄)haloalkyl;
**R₁** is H or (C₁-C₄)alkyl;
**R₂** is H or selected from the group consisting of (C₁-C₄)alkyl, halo, (C₁-C₄)haloalkyl and (C₃-C₈)cycloalkyl;
**R₃** is (C₁-C₄)alkyl;
**A** is selected from the group consisting of 5-6 membered heteroaryl wherein each of said heteroaryl may be optionally substituted by one or more group selected from (C₁-C₄)alkyl, -C(O)R₁, -C(O)OR₁, (C₁-C₄)haloalkyl, halo, -NR_{A}C(O)R₁,-NR_{A}C(O)OR₁, -NR_{A}C(O)-(C₁-C₄)alkylene-OR₁, -NR_{A}C(O)R_{C}, aryl and heteroaryl optionally substituted by one or more (C₁-C₄)alkyl and (C₁-C₄)haloalkyl,;
**R_{C}** is selected from the group consisting of heteroaryl and (C₄-C₈) heterocycloalkyl wherein said heteroaryl and heterocycloalkyl may be optionally substituted by one or more (C₁-C₄)alkyl and -C(O)OR₁;
**R_{A}** is H or (C₁-C₄)alkyl,
with the proviso that when R is methyl and A is aryl, said aryl is not substituted by one or more methyl and chlorine.

3. The compound of formula (I) according to claims 1 and 2, wherein A is selected from the group consisting of 5-6 membered heteroaryl wherein each of said heteroaryl may be optionally substituted by one or more group selected from (C₁-C₄)alkyl, -C(O)R₁, -C(O)OR₁, -C(O)R₁, (C₁-C₄)haloalkyl, halo, -NR_{A}C(O)R₁,-NR_{A}C(O)OR₁, -NR_{A}C(O)-(C₁-C₄)alkylene-OR₁, -NR_{A}C(O)R_{C},-NR_{A}C(O)NR_{A}R_{B}, -N(C₁-C₄)alkylene-NR_{A}R_{B}, aryl and heteroaryl optionally substituted by one or more (C₁-C₄)alkyl and (C₁-C₄)haloalkyl selected from the group consisting of isoxazole, pyridine, thiazole, oxazole, 1,2,4-oxadiazole, 1,3,4-oxadiazole and pyrazole;

4. The compound of formula (I) according to any claims 1 to 3, wherein when A is 5-6 membered heteroaryl said 5-6 membered heteroaryl is selected from the group consisting of thiazole, thiophene and furan.

5. The compound of formula (I) according to any claims 1 to 4, wherein when **R_{C}** is heteroaryl said heteroaryl is isoxazole optionally substituted by one or more (C₁-C₄)alkyl and -C(O)OR₁.

6. The compound of formula (I) according to claims 1 to 5 selected from at least one of:
methyl N-[4-methyl-5-({4-[(2S)-2-{[7-(trifluoromethyl)thieno[3,2-d]pyrimidin-4-yl]amino}propyl]piperazin-1-yl}sulfonyl)-1,3-thiazol-2-yl]carbamate,
methyl N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]carbamate,
2-cyclopropyl-7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine,
methyl 5-({4-[(2S)-2-({7-methylthieno[3,2-d]pyrimidin-4-yl}amino)propyl]piperazin-1-yl}sulfonyl)thiophene-2-carboxylate,
N-[(2S)-1-{4-[(4,5-dichlorothiophen-2-yl)sulfonyl]piperazin-1-yl}propan-2-yl]-7-methylthieno[3,2-d]pyrimidin-4-amine,
N-[(2S)-1-{4-[(4-bromo-5-chlorothiophen-2-yl)sulfonyl]piperazin-1-yl}propan-2-yl]-7-methylthieno[3,2-d]pyrimidin-4-amine,
methyl 4-bromo-5-({4-[(2S)-2-({7-methylthieno[3,2-d]pyrimidin-4-yl}amino)propyl]piperazin-1-yl}sulfonyl)thiophene-2-carboxylate,
7-methyl-N-[(2S)-1-{4-[(2-phenyl-1,3-thiazol-5-yl)sulfonyl]piperazin-1-yl}propan-2-yl]thieno[3,2-d]pyrimidin-4-amine,
7-methyl-N-[(2S)-1-{4-[(2-phenylpyrimidin-5-yl)sulfonyl]piperazin-1-yl}propan-2-yl]thieno[3,2-d]pyrimidin-4-amine,
N-[4-methyl-5-({4-[(2S)-2-({7-methylthieno[3,2-d]pyrimidin-4-yl}amino)propyl]piperazin-1-yl}sulfonyl)-1,3-thiazol-2-yl]propenamide,
methyl N-[4-methyl-5-({4-[(2S)-2-({7-methylthieno[3,2-d]pyrimidin-4-yl}amino)propyl]piperazin-1-yl}sulfonyl)-1,3-thiazol-2-yl]carbamate,
7-methyl-N-[(2S)-1-(4-{[5-(1,2-oxazol-5-yl)thiophen-2-yl]sulfonyl}piperazin-1-yl)propan-2-yl]thieno[3,2-d]pyrimidin-4-amine,
N-[5-({4-[(2S)-2-({2-cyclopropyl-7-methylthieno[3,2-d]pyrimidin-4-yl}amino)propyl]piperazin-1-yl}sulfonyl)-1,3-thiazol-2-yl]acetamide,
7-methyl-N-[(2S)-1-[4-({1-[5-(trifluoromethyl)pyridin-2-yl]-1H-pyrazol-4-yl}sulfonyl)piperazin-1-yl]propan-2-yl]thieno[3,2-d]pyrimidin-4-amine,
N-[4-methyl-5-({4-[(2S)-2-({7-methylthieno[3,2-d]pyrimidin-4-yl}amino)propyl]piperazin-1-yl}sulfonyl)-1,3-thiazol-2-yl]formamide,
7-methyl-N-[(2S)-1-(4-{[5-(2-methyl-1,3-thiazol-4-yl)thiophen-2-yl]sulfonyl}piperazin-1-yl)propan-2-yl]thieno[3,2-d]pyrimidin-4-amine,
N-[(2S)-1-(4-{[5-(3,4-dimethyl-1,2-oxazol-5-yl)thiophen-2-yl]sulfonyl}piperazin-1-yl)propan-2-yl]-7-methylthieno[3,2-d]pyrimidin-4-amine,
7-methyl-N-[(2S)-1-[4-({5-[1-methyl-5-(trifluoromethyl)-1H-pyrazol-3-yl]thiophen-2-yl}sulfonyl)piperazin-1-yl]propan-2-yl]thieno[3,2-d]pyrimidin-4-amine,
7-methyl-N-[(2S)-1-(4-{[5-(pyridin-2-yl)thiophen-2-yl]sulfonyl}piperazin-1-yl)propan-2-yl]thieno[3,2-d]pyrimidin-4-amine,
methyl N-[5-({4-[(2S)-2-({2-cyclopropyl-7-methylthieno[3,2-d]pyrimidin-4- yl} amino)propyl]piperazin-1 -yl} sulfonyl)-4-m ethyl-1,3 -thiazol-2-yl]carbamate,
methyl N-[5-({4-[(2S)-2-{[2-chloro-7-(trifluoromethyl)thieno[3,2-d]pyrimidin-4-yl]amino}propyl]piperazin-1-yl}sulfonyl)-4-methyl-1,3-thiazol-2-yl]carbamate,
tert-butyl 3-{[4-methyl-5-({4-[(2S)-2-({7-methylthieno[3,2-d]pyrimidin-4-yl}amino)propyl]piperazin-1-yl}sulfonyl)-1,3-thiazol-2-yl]carbamoyl}azetidine-1-carboxylate,
methyl N-[5-({4-[(2S)-2-{[2-cyclopropyl-7-(trifluoromethyl)thieno[3,2-d]pyrimidin-4-yl]amino}propyl]piperazin-1-yl}sulfonyl)-4-methyl-1,3-thiazol-2-yl]carbamate,
N-[5-({4-[(2S)-2-({2-cyclopropyl-7-methylthieno[3,2-d]pyrimidin-4-yl} amino)propyl]piperazin-1 -yl} sulfonyl)-4-m ethyl-1,3 -thiazol-2-yl]acetamide,
N-[5-({4-[(2S)-2-({2-ethyl-7-methylthieno[3,2-d]pyrimidin-4-yl}amino)propyl]piperazin-1-yl}sulfonyl)-4-methyl-1,3-thiazol-2-yl]acetamide,
methyl N-[5-({4-[(2S)-2-({2,7-dimethylthieno[3,2-d]pyrimidin-4-yl } amino)propyl]piperazin-1-yl} sulfonyl)-4-methyl-1,3-thiazol-2-yl] carbamate,
methyl N-[5-({4-[(2S)-2-({2-ethyl-7-methylthieno[3,2-d]pyrimidin-4-yl}amino)propyl]piperazin-1-yl}sulfonyl)-4-methyl-1,3-thiazol-2-yl]carbamate,
7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine,
7-(3,5-dimethyl-1,2-oxazol-4-yl)-N-[(2S)-1-(4-{[5-(3,4-dimethyl-1,2-oxazol-5-yl)thiophen-2-yl]sulfonyl}piperazin-1-yl)propan-2-yl]thieno[3,2-d]pyrimidin-4-amine,
methyl N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]-N-methylcarbamate,
7-(2,4-dimethyl-1,3-thiazol-5-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine,
N-[(2S)-1-(4-{[5-(3 -methyl-1,2-oxazol-5-yl)thiophen-2-yl]sulfonyl}piperazin-1-yl)propan-2-yl]-7-(pyridin-4-yl)thieno[3,2-d]pyrimidin-4-amine,
N-[(2S)-1-(4-{[5-(3-methyl-1,2-oxazol-5-yl)thiophen-2-yl]sulfonyl}piperazin-1-yl)propan-2-yl]-7-(pyridin-3-yl)thieno[3,2-d]pyrimidin-4-amine,
N-[(2S)-1-(4-{[5-(3-methyl-1,2-oxazol-5-yl)thiophen-2-yl]sulfonyl}piperazin-1-yl)propan-2-yl]-7-(1,3,5-trimethyl-1H-pyrazol-4-yl)thieno[3,2-d]pyrimidin-4-amine,
7-(3,5-dimethyl-1,2-oxazol-4-yl)-2-ethyl-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amine,
N-[(2S)-1-(4-{[5-(3-methyl-1,2-oxazol-5-yl)thiophen-2-yl]sulfonyl}piperazin-1-yl)propan-2-yl]-7-[1-methyl-3-(trifluoromethyl)-1H-pyrazol-4-yl]thieno[3,2-d]pyrimidin-4-amine,
7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]-2-(propan-2-yl)thieno[3,2-d]pyrimidin-4-amine,
N-[5-({4-[(2S)-2-{[7-(trifluoromethyl)thieno[3,2-d]pyrimidin-4-yl]amino}propyl]piperazin-1-yl}sulfonyl)-1,3-thiazol-2-yl]acetamide,
2-methoxy-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]acetamide,
5-methyl-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]-1,2-oxazole-3-carboxamide,
methyl N-[5-({4-[(2S)-2-{[7-(trifluoromethyl)thieno[3,2-d]pyrimidin-4-yl]amino}propyl]piperazin-1-yl}sulfonyl)-1,3-thiazol-2-yl]carbamate,
methyl N-[4-methyl-5-({4-[(2S)-2-{[7-(pyridin-3-yl)thieno[3,2-d]pyrimidin-4-yl]amino}propyl]piperazin-l-yl}sulfonyl)-1,3-thiazol-2-yl]carbamate,
5-methyl-N-[4-methyl-5-({4-[(2S)-2-{[7-(trifluoromethyl)thieno[3,2-d]pyrimidin-4-yl]amino}propyl]piperazin-l-yl}sulfonyl)-1,3-thiazol-2-yl]-1,2-oxazole-3-carboxamide.

7. A pharmaceutical composition comprising a compound of formula (I) according to any one of claims 1 to 6, in admixture with one or more pharmaceutically acceptable carrier or excipient.

8. The pharmaceutical composition according to claim 7 for oral administration.

9. A compound of formula (I) according to any one of claims 1 to 6 or a pharmaceutical composition according to claims 7 and 8 for use as a medicament.

10. A compound of formula (I) or a pharmaceutical composition for use according to claims 9 in the prevention and/or treatment of fibrosis and/or diseases, disorders, or conditions that involve fibrosis.

11. A compound of formula (I) or a pharmaceutical composition for use according to claim 10 in the prevention and/or treatment of fibrosis including pulmonary fibrosis, idiopathic pulmonary fibrosis (IPF), hepatic fibrosis, renal fibrosis, ocular fibrosis, cardiac fibrosis, arterial fibrosis and systemic sclerosis.

12. A compound of formula (I) or a pharmaceutical composition for use according to claim 11 in the prevention and/or treatment idiopathic pulmonary fibrosis (IPF).

## Patentansprüche

1. Eine Verbindung der Formel (I) 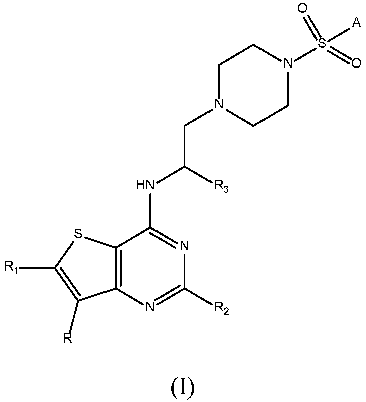 wobei
**R ist** H oder ausgewählt aus der Gruppe, bestehend aus (C₁-C₄)-Alkyl, (C₁-C₄)-Halogenalkyl und 5-6-gliedrigem Heteroaryl, wobei jedes der Heteroaryle gegebenenfalls durch eine oder mehrere Gruppen, ausgewählt aus (C₁-C₄)-Alkyl, Halogen, (C₁-C₄)-Halogenalkyl, substituiert sein kann;
**R₁** ist H oder (C₁-C₄)Alkyl;
**R₂** ist H oder ausgewählt aus der Gruppe, bestehend aus (C₁-C₄)Alkyl, Halogen, (C₁-C₄)Halogenalkyl und (C₃-C₈)Cycloalkyl;
**R₃** ist H oder (C₁-C₄)-Alkyl;
**A** ist ausgewählt aus der Gruppe, bestehend aus 5-6-gliedrigem Heteroaryl, wobei jedes Heteroaryl gegebenenfalls durch eine oder mehrere Gruppen substituiert sein kann, die ausgewählt sind aus (C₁-C₄)-Alkyl, -C(O)R₁, -C(O)OR₁, -C(O)R₁, (C₁-C₄)-Halogenalkyl, Halogen, -NR_{A}C(O)R₁, -NR_{A}C(O)OR₁, -NR_{A}C(O)-(C₁-C₄)-Alkylen-OR₁, -NR_{A}C(O)R_{C}, -NR_{A}C(O)NR_{A}R_{B}, -N(C₁-C₄)-Alkylen-NR_{A}R_{B}, Heteroaryl, gegebenenfalls substituiert durch ein oder mehrere (C₁-C₄)-Alkyle und (C₁-C₄)-Halogenalkyle,
**R_{C}** ist ausgewählt aus der Gruppe, bestehend aus Heteroaryl, Aryl, (C₃-C₈)-Cycloalkyl und (C₄-C₈)-Heterocycloalkyl, wobei das Heteroaryl, Aryl, Heterocycloalkyl und Cycloalkyl gegebenenfalls mit einem oder mehreren (C₁-C₄)-Alkyle und -C(O)OR₁ substituiert sein kann;
**R_{A}** und **R_{B}** sind bei jedem Auftreten unabhängig voneinander H oder ausgewählt aus der Gruppe, bestehend aus (C₁-C₄)-Alkyl, (C₃-C₈)-Cycloalkyl, (C₁-C₆)-Halogenalkyl und Halogen oder
R_{A} und R_{B} können zusammen mit dem Stickstoffatom, an das sie gebunden sind, ein 4-6-gliedriges gesättigtes heterocyclisches Ringsystem bilden, das gegebenenfalls ein weiteres Heteroatom, ausgewählt aus N, S und O, enthält, wobei das heterocyclische Ringsystem gegebenenfalls durch eine oder mehrere Gruppen, ausgewählt aus (C₁-C₄)-Alkyl, (C₁-C₄)-Halogenalkyl und Halogen, substituiert sein kann.

2. Verbindung der Formel (I) nach Anspruch 1, wobei
**R ist** H oder ausgewählt aus der Gruppe, bestehend aus (C₁-C₄)-Alkyl, (C₁-C₄)-Halogenalkyl und 5-6-gliedrigem Heteroaryl, wobei jedes Heteroaryl gegebenenfalls durch eine oder mehrere Gruppen, ausgewählt aus (C₁-C₄)-Alkyl, Halogen und (C₁-C₄)-Halogenalkyl, substituiert sein kann;
**R₁** ist H oder (C₁-C₄)Alkyl;
**R₂** ist H oder ausgewählt aus der Gruppe, bestehend aus (C₁-C₄)Alkyl, Halogen, (C₁-C₄)Halogenalkyl und (C₃-C₈)Cycloalkyl;
**R₃** ist (C₁-C₄)Alkyl;
**A** ist ausgewählt aus der Gruppe bestehend aus 5-6-gliedrigem Heteroaryl, wobei jedes Heteroaryl gegebenenfalls substituiert sein kann durch eine oder mehrere Gruppen, ausgewählt aus (C₁-C₄)-Alkyl, -C(O)R₁, -C(O)OR₁, (C₁-C₄)-Halogenalkyl, Halogen, -NR_{A}C(O)R₁, -NR_{A}C(O)OR₁, -NR_{A}C(O)-(C₁-C₄)-Alkylen-OR₁, -NR_{A}C(O)R_{C}, Aryl und Heteroaryl, gegebenenfalls substituiert durch ein oder mehrere (C₁-C₄)-Alkyle und (C₁-C₄)-Halogenalkyle;
**R_{C}** ist ausgewählt aus der Gruppe, bestehend aus Heteroaryl und (C₄-C₈)-Heterocycloalkyl, wobei das Heteroaryl und das Heterocycloalkyl gegebenenfalls durch ein oder mehrere (C₁-C₄)-Alkyle und -C(O)OR₁ substituiert sein können;
**R_{A}** ist H oder (C₁-C₄)-Alkyl,
mit der Maßgabe, dass, wenn R für Methyl und A für Aryl steht, das Aryl nicht durch ein oder mehrere Methyl- und Chloratome substituiert ist.

3. Verbindung der Formel (I) nach Anspruch 1 und 2, wobei A ausgewählt ist aus der Gruppe, bestehend aus 5-6-gliedrigem Heteroaryl, wobei jedes Heteroaryl gegebenenfalls substituiert sein kann durch eine oder mehrere Gruppen, ausgewählt aus (C₁-C₄)-Alkyl, -C(O)R₁, -C(O)OR₁, -C(O)R₁, (C₁-C₄)-Halogenalkyl, Halogen, -NR_{A}C(O)R₁, -NR_{A}C(O)OR₁, -NR_{A}C(O)-(C₁-C₄)-Alkylen-OR₁, -NR_{A}C(O)R_{C}, -NR_{A}C(O)NR_{A}R_{B}, -N(C₁-C₄)-Alkylen-NR_{A}R_{B}, Aryl und Heteroaryl, gegebenenfalls substituiert durch ein oder mehrere (C₁-C₄)-Alkyle und (C₁-C₄)-Halogenalkyle, ausgewählt aus der Gruppe bestehend aus Isoxazol, Pyridin, Thiazol, Oxazol, 1,2,4-Oxadiazol, 1,3,4-Oxadiazol und Pyrazol;

4. Verbindung der Formel (I) nach einem der Ansprüche 1 bis 3, wobei, wenn A ein 5-6-gliedriges Heteroaryl ist, das 5-6-gliedrige Heteroaryl ausgewählt ist aus der Gruppe, bestehend aus Thiazol, Thiophen und Furan.

5. Verbindung der Formel (I) nach einem der Ansprüche 1 bis 4, wobei, wenn Rc Heteroaryl ist, das Heteroaryl Isoxazol ist, das gegebenenfalls durch ein oder mehrere (C₁-C₄)-Alkyle und -C(O)OR₁ substituiert ist.

6. Verbindung der Formel (I) nach den Ansprüchen 1 bis 5, ausgewählt aus mindestens einer der folgenden Verbindungen:
Methyl-N-[4-methyl-5-({4-[(2S)-2-{[7-(trifluormethyl)thieno[3,2-d]pyrimidin-4-yl]amino}propyl]piperazin-1-yl}sulfonyl)-1,3-thiazol-2-yl]carbamat,
Methyl-N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]carbamat,
2-cyclopropyl-7-(3,5-dimethyl-1,2-oxazol-4-yl)-N-[(2S)-1-(4-{ [5-(3-methyl-1,2-oxazol-5-yl)thiophen-2-yl]sulfonyl}piperazin-1-yl)propan-2-yl]thieno[3,2-d]pyrimidin-4-amin,
Methyl-5-({4-[(2S)-2-({7-methylthieno[3,2-d]pyrimidin-4-yl}amino)propyl]piperazin-1-yl}sulfonyl)thiophen-2-carboxylat,
N-[(2S)-1-{4-[(4,5-dichlorthiophen-2-yl)sulfonyl]piperazin-1-yl}propan-2-yl]-7-methylthieno[3,2-d]pyrimidin-4-amin,
N-[(2S)-1-{4-[(4-brom-5-chlorthiophen-2-yl)sulfonyl]piperazin-1-yl}propan-2-yl]-7-methylthieno[3,2-d]pyrimidin-4-amin,
Methyl-4-brom-5-({4-[(2S)-2-({7-Methylthieno[3,2-d]pyrimidin-4-yl}amino)propyl]piperazin-1-yl}sulfonyl)thiophen-2-carboxylat,
7-methyl-N-[(2S)-1-{4-[(2-phenyl-1,3-thiazol-5-yl)sulfonyl]piperazin-1-yl}propan-2-yl]thieno[3,2-d]pyrimidin-4-amin,
7-methyl-N-[(2S)-1-{4-[(2-phenylpyrimidin-5-yl)sulfonyl]piperazin-1-yl}propan-2-yl]thieno[3,2-d]pyrimidin-4-amin,
N-[4-methyl-5-({4-[(2S)-2-({7-methylthieno[3,2-d]pyrimidin-4-yl}amino)propyl]piperazin-1-yl}sulfonyl)-1,3-thiazol-2-yl]propenamid,
Methyl-N-[4-methyl-5-({4-[(2S)-2-({7-methylthieno[3,2-d]pyrimidin-4-yl}amino)propyl]piperazin-1-yl}sulfonyl)-1,3-thiazol-2-yl]carbamat,
7-methyl-N-[(2S)-1-(4-{[5-(1,2-oxazol-5-yl)thiophen-2-yl]sulfonyl}piperazin-1-yl)propan-2-yl]thieno[3,2-d]pyrimidin-4-amin,
N-[5-({4-[(2S)-2-({2-cyclopropyl-7-methylthieno[3,2-d]pyrimidin-4-yl}amino)propyl]piperazin-1-yl}sulfonyl)-1,3-thiazol-2-yl]acetamid,
7-methyl-N-[(2S)-1-[4-({1-[5-(trifluormethyl)pyridin-2-yl]-1H-pyrazol-4-yl}sulfonyl)piperazin-1-yl]propan-2-yl]thieno[3,2-d]pyrimidin-4-amin,
N-[4-methyl-5-({4-[(2S)-2-({7-methylthieno[3,2-d]pyrimidin-4-yl}amino)propyl]piperazin-1-yl}sulfonyl)-1,3-thiazol-2-yl]formamid,
7-methyl-N-[(2S)-1-(4-{[5-(2-methyl-1,3-thiazol-4-yl)thiophen-2-yl]sulfonyl}piperazin-1-yl)propan-2-yl]thieno[3,2-d]pyrimidin-4-amin,
N-[(2S)-1-(4-{[5-(3,4-dimethyl-1,2-oxazol-5-yl)thiophen-2-yl]sulfonyl}piperazin-1-yl)propan-2-yl]-7-methylthieno[3,2-d]pyrimidin-4-amin,
7-methyl-N-[(2S)-1-[4-({5-[1-methyl-5-(trifluormethyl)-1H-pyrazol-3-yl]thiophen-2-yl}sulfonyl)piperazin-1-yl]propan-2-yl]thieno[3,2-d]pyrimidin-4-amin,
7-methyl-N-[(2S)-1-(4-{[5-(pyridin-2-yl)thiophen-2-yl]sulfonyl}piperazin-1-yl)propan-2-yl]thieno[3,2-d]pyrimidin-4-amin,
Methyl-N-[5-({4-[(2S)-2-({2-cyclopropyl-7-methylthieno[3,2-d]pyrimidin-4-yl}amino)propyl]piperazin-1-yl}sulfonyl)-4-methyl-1,3 -thiazol-2-yl]carbamat,
Methyl-N-[5-({4-[(2S)-2-{[2-chlor-7-(trifluormethyl)thieno[3,2-d]pyrimidin-4-yl]amino}propyl]piperazin-1-yl}sulfonyl)-4-methyl-1,3-thiazol-2-yl]carbamat,
Tert-butyl 3-{[4-methyl-5-({4-[(2S)-2-({7-methylthieno[3,2-d]pyrimidin-4-yl}amino)propyl]piperazin-1-yl}sulfonyl)-1,3-thiazol-2-yl]carbamoyl}azetidin-1-carboxylat,
Methyl-N-[5-({4-[(2S)-2-{[2-cyclopropyl-7-(trifluormethyl)thieno[3,2-d]pyrimidin-4-yl]amino}propyl]piperazin-1-yl}sulfonyl)-4-methyl-1,3-thiazol-2-yl]carbamat,
N-[5-({4-[(2S)-2-({2-cyclopropyl-7-methylthieno[3,2-d]pyrimidin-4-yl} amino)propyl]piperazin-1 -yl} sulfonyl)-4-methyl-1,3 -thiazol-2-yl]acetamid,
N-[5-({4-[(2S)-2-({2-ethyl-7-methylthieno[3,2-d]pyrimidin-4-yl} amino)propyl]piperazin-1 -yl} sulfonyl)-4-methyl-1,3 -thiazol-2-yl]acetamid,
Methyl-N-[5-({4-[(2S)-2-({2,7-dimethylthieno[3,2-d]pyrimidin-4-yl}amino)propyl]piperazin-1-yl}sulfonyl)-4-methyl-1,3-thiazol-2-yl]carbamat,
Methyl-N-[5-({4-[(2S)-2-({2-ethyl-7-methylthieno[3,2-d]pyrimidin-4-yl} amino)propyl]piperazin-1 -yl} sulfonyl)-4-methyl-1,3 -thiazol-2-yl]carbamat,
7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amin,
7-(3,5-dimethyl-1,2-oxazol-4-yl)-N-[(2S)-1-(4-{[5-(3,4-dimethyl-1,2-oxazol-5-yl)thiophen-2-yl]sulfonyl}piperazin-1-yl)propan-2-yl]thieno[3,2-d]pyrimidin-4-amin,
Methyl-N-[5-({4-[(2S)-2-{[7-(3,5-dimethyl-1,2-oxazol-4-yl)thieno[3,2-d]pyrimidin-4-yl]amino}propyl]piperazin-1-yl}sulfonyl)-4-methyl-1,3-thiazol-2-yl]-N-methylcarbamat,
7-(2,4-dimethyl-1,3-thiazol-5-yl)-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amin,
N-[(2S)-1-(4-{[5-(3-methyl-1,2-oxazol-5-yl)thiophen-2-yl]sulfonyl}piperazin-1-yl)propan-2-yl]-7-(pyridin-4-yl)thieno[3,2-d]pyrimidin-4-amin,
N-[(2S)-1-(4-{ [5-(3-methyl-1,2-oxazol-5-yl)thiophen-2-yl]sulfonyl}piperazin-1-yl)propan-2-yl]-7-(pyridin-3-yl)thieno[3,2-d]pyrimidin-4-amin,
N-[(2S)-1-(4-{[5-(3-methyl-1,2-oxazol-5-yl)thiophen-2-yl]sulfonyl}piperazin-1-yl)propan-2-yl]-7-(1,3,5-trimethyl-1H-pyrazol-4-yl)thieno[3,2-d]pyrimidin-4-amin,
7-(3,5-dimethyl-1,2-oxazol-4-yl)-2-ethyl-N-[(2S)-1-(4-{[5-(3-methyl-1,2-oxazol-5-yl)thiophen-2-yl]sulfonyl}piperazin-1-yl)propan-2-yl]thieno[3,2-d]pyrimidin-4-amin,
N-[(2S)-1-(4-{[5-(3-methyl-1,2-oxazol-5-yl)thiophen-2-yl]sulfonyl}piperazin-1-yl)propan-2-yl]-7-[1-methyl-3-(trifluormethyl)-1H-pyrazol-4-yl]thieno[3,2-d]pyrimidin-4-amin,
7-(3,5-dimethyl-1,2-oxazol-4-yl)-N-[(2S)-1-(4-{[5-(3-methyl-1,2-oxazol-5-yl)thiophen-2-yl]sulfonyl}piperazin-1-yl)propan-2-yl]-2-(propan-2-yl)thieno[3,2-d]pyrimidin-4-amin,
N-[5-({4-[(2S)-2-{[7-(trifluormethyl)thieno[3,2-d]pyrimidin-4-yl]amino}propyl]piperazin-1-yl}sulfonyl)-1,3-thiazol-2-yl]acetamid,
2-methoxy-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]acetamid,
5-methyl-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]-1,2-oxazol-3-carboxamid,
Methyl-N-[5-({4-[(2S)-2-{[7-(Trifluormethyl)thieno[3,2-d]pyrimidin-4-yl]amino}propyl]piperazin-1-yl}sulfonyl)-1,3-thiazol-2-yl]carbamat,
Methyl-N-[4-methyl-5-({4-[(2S)-2-{[7-(pyridin-3-yl)thieno[3,2-d]pyrimidin-4-yl]amino}propyl]piperazin-1-yl}sulfonyl)-1,3-thiazol-2-yl]carbamat,
5-methyl-N-[4-methyl-5-({4-[(2S)-2-{[7-(trifluormethyl)thieno[3,2-d]pyrimidin-4-yl]amino}propyl]piperazin-1-yl}sulfonyl)-1,3-thiazol-2-yl]-1,2-oxazol-3-carboxamid.

7. Pharmazeutische Zusammensetzung, die eine Verbindung der Formel (I) umfasst, nach einem der Ansprüche 1 bis 6, unter Beigabe eines oder mehrerer pharmazeutisch annehmbarer Träger oder Hilfsstoffe.

8. Pharmazeutische Zusammensetzung nach Anspruch 7 zur oralen Verabreichung.

9. Verbindung der Formel (I) gemäß einem der Ansprüche 1 bis 6 oder pharmazeutische Zusammensetzung gemäß den Ansprüchen 7 und 8 zur Verwendung als Arzneimittel.

10. Verbindung der Formel (I) oder pharmazeutische Zusammensetzung zur Verwendung gemäß Anspruch 9 bei der Vorbeugung und/oder Behandlung von Fibrose und/oder Krankheiten, Störungen oder Zuständen, die Fibrose beinhalten.

11. Verbindung der Formel (I) oder pharmazeutische Zusammensetzung zur Verwendung gemäß Anspruch 10 bei der Vorbeugung und/oder Behandlung von Fibrose einschließlich Lungenfibrose, idiopathischer Lungenfibrose (idiopathic pulmonary fibrosis, IPF), Leberfibrose, Nierenfibrose, Augenfibrose, Herzfibrose, arterieller Fibrose und systemischer Sklerose.

12. Verbindung der Formel (I) oder pharmazeutische Zusammensetzung zur Verwendung gemäß Anspruch 11 bei der Vorbeugung und/oder Behandlung von idiopathischer Lungenfibrose (IPF).

## Revendications

1. Composé de formule (I) 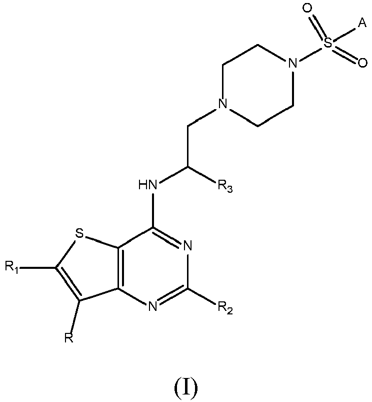 dans lequel
**R** est H ou choisi dans le groupe constitué d'alkyle (C₁-C₄), d'haloalkyle (C₁-C₄) et d'hétéroaryle à 5 ou 6 chaînons, dans lequel chacun dudit hétéroaryle peut facultativement être substitué par un ou plusieurs groupes choisis parmi alkyle (C₁-C₄), halo, haloalkyle (C₁-C₄) ;
**R₁** est H ou alkyle (C₁-C₄) ;
**R₂** est H ou choisi dans le groupe constitué d'alkyle (C₁-C₄), halo, haloalkyle (C₁-C₄) et de cycloalkyle (C₃-C₈) ;
**R₃** est H ou alkyle (C₁-C₄) ;
**A** est choisi dans le groupe constitué d'hétéroaryles à 5 ou 6 chaînons dans lequel chacun desdits hétéroaryles peut être facultativement substitué par un ou plusieurs groupes choisis parmi alkyle (C₁-C₄), -C(O)R₁, -C(O)OR₁, -C(O)R₁, haloalkyle (C₁-C₄), halo, -NR_{A}C(O)R₁, -NR_{A}C(O)OR₁, -NR_{A}C(O)-(C₁-C₄)alkylène-OR₁,-NR_{A}C(O)R_{C}, -NR_{A}C(O)NR_{A}R_{B}, -N(C₁-C₄)alkylène-NR_{A}R_{B}, facultativement substitué par un ou plusieurs alkyles (C₁-C₄) et haloalkyles (C₁-C₄),
**R_{C}** est choisi dans le groupe constitué de l'hétéroaryle, de l'aryle, du cycloalkyle (C₃-C₈) et de l'hétérocycloalkyle (C₄-C₈) dans lequel ledit hétéroaryle, aryle, hétérocycloalkyle et cycloalkyle peut facultativement être substitué par un ou plusieurs alkyles (C₁-C₄) et -C(O)OR₁ ;
**R_{A}** et **R_{B}** sont à chaque occurrence indépendamment H ou sélectionnés dans le groupe constitué d'alkyle (C₁-C₄), cycloalkyle (C₃-C₈), haloalkyle (C₁-C₆) et halo, ou
R_{A} et R_{B} peuvent former ensemble avec l'atome d'azote auquel ils sont attachés un système d'anneau hétérocyclique saturé de 4 à 6 chaînons contenant facultativement un autre hétéroatome choisi parmi N, S et O, ledit système d'anneau hétérocyclique peut facultativement être substitué par un ou plusieurs groupes choisis parmi alkyle (C₁-C₄), haloalkyle (C₁-C₄) et halo.

2. Composé de formule (I) selon la revendication 1, dans lequel
**R** est H ou choisi dans le groupe constitué d'alkyle (C₁-C₄), d'haloalkyle (C₁-C₄) et d'hétéroaryle à 5 ou 6 chaînons dans lequel chacun dudit hétéroaryle peut être facultativement substitué par un ou plusieurs groupes choisis parmi alkyle (C₁-C₄), halo et haloalkyle (C₁-C₄) ;
**R₁** est H ou alkyle (C₁-C₄) ;
**R₂** est H ou choisi dans le groupe constitué d'alkyle (C₁-C₄), halo, haloalkyle (C₁-C₄) et de cycloalkyle (C₃-C₈) ;
**R₃** est alkyle (C₁-C₄) ;
**A** est choisi dans le groupe constitué d'hétéroaryles à 5 ou 6 chaînons dans lequel chacun desdits hétéroaryles peut être facultativement substitué par un ou plusieurs groupes choisis parmi alkyle (C₁-C₄), -C(O)R₁, -C(O)OR₁, haloalkyle (C₁-C₄), halo, -NR_{A}C(O)R₁, -NR_{A}C(O)OR₁, -NR_{A}C(O)-(C₁-C₄)alkylène-OR₁,-NR_{A}C(O)R_{C}, aryle et hétéroaryle substitués facultativement par un ou plusieurs alkyles (C₁-C₄) et haloalkyles (C₁-C₄) ;
**R_{C}** est choisi dans le groupe constitué d'hétéroaryle et d'hétérocycloalkyle (C₄-C₈), dans lequel ledit hétéroaryle et hétérocycloalkyle peuvent facultativement être substitués par un ou plusieurs alkyles (C₁-C₄) et -C(O)OR₁ ;
**R_{A}** est H ou alkyle (C₁-C₄),
à condition que lorsque R est méthyle et A est aryle, ledit aryle ne soit pas substitué par un ou plusieurs méthyles et chlores.

3. Composé de formule (I) selon les revendications 1 et 2, dans lequel A est choisi dans le groupe constitué d'hétéroaryles à 5 ou 6 chaînons dans lequel chacun dudit hétéroaryle peut facultativement être substitué par un ou plusieurs groupes choisis parmi alkyle (C₁-C₄), -C(O)R₁, -C(O)OR₁, -C(O)R₁, haloalkyle (C₁-C₄), halo,-NR_{A}C(O)R₁, -NR_{A}C(O)OR₁, -NR_{A}C(O)-(C₁-C₄)alkylène-OR₁, -NR_{A}C(O)R_{C},-NR_{A}C(O)NR_{A}R_{B}, -N(C₁₋C₄)alkylène-NR_{A}R_{B}, aryle et hétéroaryle facultativement substitués par un ou plusieurs alkyles (C₁-C₄) et haloalkyles (C₁-C₄) choisis dans le groupe constitué par l'isoxazole, pyridine, thiazole, oxazole, 1,2,4-oxadiazole, 1,3,4-oxadiazole et pyrazole ;

4. Composé de formule (I) selon l'une des revendications 1 à 3, dans lequel, lorsque A est hétéroaryle à 5 ou 6 chaînons, ledit hétéroaryle à 5 ou 6 chaînons est choisi dans le groupe constitué de thiazole, de thiophène et de furane.

5. Composé de formule (I) selon l'une des revendications 1 à 4, dans lequel, lorsque **R_{C}** est hétéroaryle, ledit hétéroaryle est facultativement substitué par un ou plusieurs alkyles (C₁-C₄) et -C(O)OR₁.

6. Composé de formule (I) selon les revendications 1 à 5 choisi parmi au moins l'un des :
N-[4-méthyl-5-({4-[(2S)-2-{[7-(trifluorométhyl)thiéno[3,2-d]pyrimidin-4-yl]amino}propyl]pipérazin-1-yl}sulfonyl)-1,3-thiazol-2-yl]carbamate de méthyle,
N-[5-({4-[(2S)-2-{[7-(3,5-diméthyl-1,2-oxazol-4-yl)thiéno[3,2-d]pyrimidin-4-yl]amino}propyl]pipérazin-1-yl}sulfonyl)-4-méthyl-1,3-thiazol-2-yl]carbamate de méthyle,
2-cyclopropyl-7-(3,5-diméthyl-1,2-oxazol-4-yl)-N-[(2S)-1-(4-{[5-(3-méthyl-1,2-oxazol-5-yl)thiophen-2-yl]sulfonyl}pipérazin-1-yl)propan-2-yl]thiéno[3,2-d]pyrimidin-4-amine,
5-({4-[(2S)-2-({7-méthylthieno[3,2-d]pyrimidin-4-yl}amino)propyl]pipérazin-1-yl}sulfonyl)thiophène-2-carboxylate de méthyle,
N-[(2S)-1-{4-[(4,5-dichlorothiophen-2-yl)sulfonyl]pipérazin-1-yl}propan-2-yl]-7-méthylthieno[3,2-d]pyrimidin-4-amine,
N-[(2S)-1-{4-[(4-bromo-5-chlorothiophen-2-yl)sulfonyl]pipérazin-1-yl}propan-2-yl]-7-méthylthieno[3,2-d]pyrimidin-4-amine,
4-bromo-5-({4-[(2S)-2-({7-méthylthieno[3,2-d]pyrimidin-4-yl}amino)propyl]pipérazin-1-yl}sulfonyl)thiophène-2-carboxylate de méthyle,
7-méthyl-N -[(2S)-1-{4-[(2-phényl-1,3-thiazol-5-yl)sulfonyl]pipérazin-1-yl}propan-2-yl]thiéno[3,2-d]pyrimidin-4-amine,
7-méthyl-N-[(2S)-1-{4-[(2-phénylpyrimidin-5-yl)sulfonyl]pipérazin-1-yl}propan-2-yl]thiéno[3,2-d]pyrimidin-4-amine,
N-[4-méthyl-5-({4-[(2S)-2-({7-méthylthieno[3,2-d]pyrimidin-4-yl}amino)propyl]pipérazin-1-yl}sulfonyl)-1,3-thiazol-2-yl]propénamide,
N-[4-méthyl-5-({4-[(2S)-2-({7-méthylthieno[3,2-d]pyrimidin-4-yl}amino)propyl]pipérazin-1 -yl}sulfonyl)-1,3-thiazol-2-yl]carbamate de méthyle,
7-methyl-N -[(2S)-1-(4-{ [5-(1,2-oxazol-5-yl)thiophen-2-yl]sulfonyl}pipérazin-1-yl)propan-2-yl]thiéno[3,2-d]pyrimidin-4-amine,
N-[5-({4-[(2S)-2-({2-cyclopropyl-7-méthylthieno[3,2-d]pyrimidin-4-yl}amino)propyl]piperazin-1-yl}sulfonyl)-1,3-thiazol-2-yl]acétamide,
7-méthyl-N-[(2S)-1-[4-({1-[5-(trifluorométhyl)pyridine-2-yl]-1H-pyrazol-4-yl}sulfonyl)pipérazin-1-yl]propan-2-yl]thiéno[3,2-d]pyrimidin-4-amine,
N-[4-méthyl-5-({4-[(2S)-2-({7-méthylthieno[3,2-d]pyrimidin-4-yl}amino)propyl]pipérazin-1-yl}sulfonyl)-1,3-thiazol-2-yl]formamide,
7-méthyl-N-[(2S)-1-(4-{[5-(2-méthyl-1,3-thiazol-4-yl)thiophen-2-yl]sulfonyl}pipérazin-1-yl)propan-2-yl]thiéno[3,2-d]pyrimidin-4-amine,
N-[(2S)-1-(4-{[5-(3,4-diméthyl-1,2-oxazol-5-yl)thiophen-2-yl]sulfonyl}pipérazin-1-yl)propan-2-yl]-7-méthylthieno[3,2-d]pyrimidin-4-amine,
7-méthyl-N-[(2S)-1-[4-({5-[1-méthyl-5-(trifluorométhyl)-1H-pyrazol-3-yl]thiophen-2-yl}sulfonyl)pipérazin-1-yl]propan-2-yl]thiéno[3,2-d]pyrimidin-4-amine,
7-méthyl-N-[(2S)-1-(4-{[5-(pyridine-2-yl)thiophen-2-yl]sulfonyl}pipérazin-1-yl)propan-2-yl]thiéno[3,2-d]pyrimidin-4-amine,
N-[5-({4-[(2S)-2-({2-cyclopropyl-7-méthylthieno[3,2-d]pyrimidin-4-yl}amino)propyl]pipérazin-1-yl}sulfonyl)-4-méthyl-1,3-thiazol-2-yl]carbamate de méthyle,
N-[5-({4-[(2S)-2-{[2-chloro-7-(trifluorométhyl)thiéno[3,2-d]pyrimidin-4-yl]amino}propyl]pipérazin-1-yl}sulfonyl)-4-méthyl-1,3-thiazol-2-yl]carbamate de méthyle,
tert-butyle 3-{[4-méthyl-5-({4-[(2S)-2-({7-méthylthieno[3,2-d]pyrimidin-4-yl}amino)propyl]pipérazin-1-yl}sulfonyl)-1,3-thiazol-2-yl]carbamoyl}azétidine-1-carboxylate,
N-[5-({4-[(2S)-2-{[2-cyclopropyl-7-(trifluorométhyl)thiéno[3,2-d]pyrimidin-4-yl]amino}propyl]pipérazin-1-yl } sulfonyl)-4-méthyl-1,3-thiazol-2-yl]carbamate de méthyle,
N-[5-({4-[(2S)-2-({2-cyclopropyl-7-méthylthieno[3,2-d]pyrimidin-4-yl}amino)propyl]pipérazin-1-yl}sulfonyl)-4-méthyl-1,3-thiazol-2-yl]acétamide,
N-[5-({4-[(2S)-2-({2-éthyl-7-méthylthieno[3,2-d]pyrimidin-4-yl}amino)propyl]pipérazin-1-yl}sulfonyl)-4-méthyl-1,3-thiazol-2-yl]acétamide,
N-[5-({4-[(2S)-2-({2,7-diméthylthieno[3,2-d]pyrimidin-4-yl}amino)propyl]pipérazin-1-yl}sulfonyl)-4-méthyl-1,3-thiazol-2-yl]carbamate de méthyle,
N-[5-({4-[(2S)-2-({2-éthyl-7-méthylthieno[3,2-d]pyrimidin-4-yl}amino)propyl]pipérazin-1-yl}sulfonyl)-4-méthyl-1,3-thiazol-2-yl]carbamate de méthyle,
7-(3,5-diméthyl-1,2-oxazol-4-yl)-N-[(2S)-1-(4-{[5-(3-méthyl-1,2-oxazol-5-yl)thiophen-2-yl]sulfonyl}pipérazin-1-yl)propan-2-yl]thiéno[3,2-d]pyrimidin-4-amine,
7-(3,5-diméthyl-1,2-oxazol-4-yl)-N-[(2S)-1-(4-{[5-(3,4-diméthyl-1,2-oxazol-5-yl)thiophen-2-yl]sulfonyl}pipérazin-1-yl)propan-2-yl]thieno[3,2-d]pyrimidin-4-amine,
N-[5-({4-[(2S)-2-{[7-(3,5-diméthyl-1,2-oxazol-4-yl)thiéno[3,2-d]pyrimidin-4-yl]amino}propyl]pipérazin-1-yl}sulfonyl)-4-méthyl-1,3-thiazol-2-yl]-N-méthylcarbamate de méthyle,
7-(2,4-diméthyl-1,3-thiazol-5-yl)-N-[(2S)-1-(4-{[5-(3-méthyl-1,2-oxazol-5-yl)thiophen-2-yl]sulfonyl}pipérazin-1-yl)propan-2-yl]thiéno[3,2-d]pyrimidin-4-amine,
N-[(2S)-1-(4-{ [5-(3-méthyl-1,2-oxazol-5-yl)thiophen-2-yl]sulfonyl}pipérazin-1-yl)propan-2-yl]-7-(pyridine-4-yl)thiéno[3,2-d]pyrimidin-4-amine,
N-[(2S)-1-(4-{ [5-(3-méthyl-1,2-oxazol-5-yl)thiophen-2-yl]sulfonyl}pipérazin-1-yl)propan-2-yl]-7-(pyridine-3-yl)thiéno[3,2-d]pyrimidin-4-amine,
N-[(2S)-1-(4-{[5-(3-méthyl-1,2-oxazol-5-yl)thiophen-2-yl]sulfonyl}pipérazin-1-yl)propan-2-yl]-7-(1,3,5-triméthyl-1H-pyrazol-4-yl)thiéno[3,2-d]pyrimidin-4-amine,
7-(3,5-diméthyl-1,2-oxazol-4-yl)-2-éthyl-N-[(2S)-1-(4-{[5-(3-méthyl-1,2-oxazol-5-yl)thiophen-2-yl]sulfonyl}pipérazin-1-yl)propan-2-yl]thieno[3,2-d]pyrimi din-4-amine,
N-[(2S)-1-(4-{ [5-(3-méthyl-1,2-oxazol-5-yl)thiophen-2-yl]sulfonyl}pipérazin-1-yl)propan-2-yl]-7-[1-méthyl-3-(trifluorométhyl)-1H-pyrazol-4-yl]thiéno[3,2-d]pyrimidin-4-amine,
7-(3,5-diméthyl-1,2-oxazol-4-yl)-N-[(2S)-1-(4-{[5-(3-méthyl-1,2-oxazol-5-yl)thiophen-2-yl]sulfonyl}pipérazin-1-yl)propan-2-yl]-2-(propan-2-yl)thiéno[3,2-d]pyrimidin-4-amine,
N-[5-({4-[(2S)-2-{[7-(trifluorométhyl)thiéno[3,2-d]pyrimidin-4-yl]amino}propyl]pipérazin-1-yl } sulfonyl)-1,3-thiazol-2-yl]acétamide,
2-methoxy-N-[4-méthyl-5-({4-[(2S)-2-{[7-(pyridin-3-yl)thiéno[3,2-d]pyrimidin-4-yl]amino}propyl]pipérazin-1-yl } sulfonyl)-1, 3-thiazol-2-yl]acétamide,
5-methyl-N-[4-méthyl-5-({4-[(2S)-2-{[7-(pyridin-3-yl)thieno[3,2-d]pyrimidin-4-yl]amino}propyl]pipérazin-1-yl}sulfonyl)-1,3-thiazol-2-yl]-1,2-oxazole-3-carboxamide,
N-[5-({4-[(2S)-2-{[7-(trifluorométhyl)thiéno[3,2-d]pyrimidin-4-yl]amino}propyl]pipérazin-1-yl}sulfonyl)-1,3-thiazol-2-yl]carbamate de méthyle,
N-[4-méthyl-5-({4-[(2S)-2-{[7-(pyridine-3-yl)thiéno[3,2-d]pyrimidin-4-yl]amino}propyl]pipérazin-1-yl}sulfonyl)-1,3-thiazol-2-yl]carbamate de méthyle,
5-méthyl-N-[4-méthyl-5-({4-[(2S)-2-{[7-(trifluorométhyl)thiéno[3,2-d]pyrimidin-4-yl]amino}propyl]pipérazin-1-yl}sulfonyl)-1,3-thiazol-2-yl]-1,2-oxazole-3-carboxamide.

7. Composition pharmaceutique comprenant un composé de formule (I) selon l'une des revendications 1 à 6, en mélange avec un ou plusieurs transporteurs ou excipients acceptables du point de vue pharmaceutique.

8. Composition pharmaceutique selon la revendication 7 pour administration par voie orale.

9. Composé de formule (I) selon l'une des revendications 1 à 6 ou composition pharmaceutique selon les revendications 7 et 8 pour une utilisation en tant que médicament.

10. Composé de formule (I) ou composition pharmaceutique pour une utilisation selon la revendication 9 dans la prévention et/ou le traitement de la fibrose et/ou de maladies, troubles ou affections impliquant la fibrose.

11. Composé de formule (I) ou composition pharmaceutique pour une utilisation selon la revendication 10 dans la prévention et/ou le traitement de la fibrose incluant la fibrose pulmonaire, la fibrose pulmonaire idiopathique (FPI), la fibrose hépatique, la fibrose rénale, la fibrose oculaire, la fibrose cardiaque, la fibrose artérielle et la sclérose systémique.

12. Composé de formule (I) ou composition pharmaceutique pour une utilisation selon la revendication 11 dans la prévention et/ou le traitement de la fibrose pulmonaire idiopathique (FPI).
